# EUROPEAN PATENT APPLICATION

(11) **EP 4 613 291 A1**
(43) Date of publication of application: **10.09.2025**
(21) Application number: 23886260.1
(22) Date of filing: 01.11.2023
(51) Int. Cl.: A61K 47/68, C07K 16/28

(54) **ANTIBODY-DRUG CONJUGATE COMPRISING ANTI-CLAUDIN-18.2 ANTIBODY, AND THERAPEUTIC USE THEREOF FOR CANCER**

(30) Priority: 01.11.2022 KR 20220143600; 07.09.2023 KR 20230119252
(71) Applicant: ABTIS Co., Ltd., Yongin-si, Gyeonggi-do 17073 (KR); Research & Business Foundation Sungkyunkwan University, Gyeonggi-do 16419 (KR)
(72) Inventor: CHUNG, Sang Jeon, Suwon-si, Gyeonggi-do 16420 (KR); OH, Yeong Soo, Gwacheon-si, Gyeonggi-do 13833 (KR); KIM, Juhwan, Suwon-si, Gyeonggi-do 16489 (KR); LEE, Younggeun, Icheon-si, Gyeonggi-do 17309 (KR); SEO, Jinwoo, Suwon-si, Gyeonggi-do 16408 (KR); LEE, Geonmin, Hwaseong-si, Gyeonggi-do 18264 (KR); KIM, Gahyeon, Suwon-si, Gyeonggi-do 16630 (KR); KWON, Sejeong, Suwon-si, Gyeonggi-do 16357 (KR); PARK, Sunhee, Suwon-si, Gyeonggi-do 16421 (KR); YEOM, Hojin, Incheon 22760 (KR); OH, Hui Jo, Suwon-si, Gyeonggi-do 16605 (KR); SON, Jinyoung, Suwon-si, Gyeonggi-do 16329 (KR)
(74) Representative: Dr. Schön, Neymeyr & Partner Patentanwälte mbB
(86) International application number: PCT/KR2023/017242
(87) International publication number: WO 2024/096564

(57) **Abstract**

The present application relates to an antibody-drug conjugate and a use thereof.

## Description

### [Technical Field]

The present application relates to an antibody-drug conjugate. The antibody-drug conjugates provided according to some embodiments of the present application have therapeutic uses for cancer or tumors.

Furthermore, the present application provides a pharmaceutical composition including the antibody-drug conjugate, a treatment method using the antibody-drug conjugate, a therapeutic use of the antibody-drug conjugate, and use of the antibody-drug conjugate for the manufacture of a medication for treating cancer.

### [Background Art]

Antibodies are biomolecules having a function of recognizing specific molecules, and are used in various industrial applications. As an example, a specific material may be detected or searched for (screened) using antibodies, a path through which a specific material moves within the body or within cells may be identified, and the antibody may be used for therapeutic use by inducing an immune response to a specific material.

Attempts have been made to improve such antibodies to expand their functionality. Typically, attempts have been made to label or conjugate various materials (for example, drugs or radioactive moieties, and the like) to complement or expand the functionality of antibodies. Typically, an antibody may be labeled with a fluorescent material and used in a fluorescence assay, or an antibody may be labeled with or conjugated to an agent for treating a specific disease to maximize the therapeutic efficacy of the antibody. These attempts and techniques may be referred to as antibody labeling or antibody-target moiety conjugation, and the present application relates to antibody labeling or antibody-target moiety conjugation.

In past studies, antibody-target moiety conjugates were prepared using highly reactive amino acid residues (for example, amine groups or thiol groups) among the amino acid residues that constitute antibodies. Specifically, after reactive groups capable of reacting with the residues were introduced into a target moiety, a target moiety capable of reacting with the reactive residues of the antibody (more specifically, a modified target moiety into which the reactive groups have been introduced) was prepared, and an antibody-target moiety conjugate was prepared by reacting the modified target moiety with an antibody.

Such past studies were conducted by randomly attaching a target moiety material to an antibody, and such past methods had many problems.

Essentially, the past methods were unable to accurately control the 'site where the target moiety was bound to the antibody, and furthermore, it was not possible to accurately control the 'number' of target moieties which were bound to the antibody. That is, an antibody-target moiety conjugate prepared by methods in the prior method has an inhomogeneity problem of drug structure.

Such a problem of drug structure inhomogeneity inevitably causes a problem of inhomogeneity in drug effects caused by 'differences' in drug structure. These problems have become a major obstacle to the development of antibody-drug conjugate (ADC) technology, which requires high safety and reproducibility.

Further, the problem of drug structure inhomogeneity causes a problem in that the function of the antibody is inhibited. The antibody comprises a Fab region comprising an antigen-binding domain which recognizes an antigen and an Fc region which is involved in the crystallization of the antibody. Non-site specific conjugation/labeling makes it impossible to precisely control the binding position of a target moiety to an antibody, making it impossible to prevent the target moiety from binding to the antigen-binding domain of the antibody or a position adjacent to the antigen-binding domain, thereby disturbing the recognition function of the antibody.

As a result, there is a need in the art for techniques for labeling an antibody in a site-specific manner in order to ensure the structural homogeneity of an antibody-target moiety conjugate. Although some techniques have been developed, most of them lack technical and economic effectiveness, such as genetic manipulation or modification of antibodies.

Accordingly, the present inventors developed a novel antibody-drug conjugate using a technology capable of transferring a click-chemistry functional group, which is a target substance, to an antibody using a compound including an Fc binding unit.

### [Disclosure]

### [Technical Problem]

In the production of an antibody-target moiety conjugate, when the 'position' where the target moiety binds to the antibody and the 'number' of target moieties bound to the antibody cannot be precisely controlled, there will be an inhomogeneity problem of drug structure. In particular, in drug manufacturing, this inhomogeneity problem of drug structure inevitably causes a problem of inhomogeneity in drug effects. In addition, the problem of drug structure inhomogeneity causes the problem of inhibiting the function of the antibody. Accordingly, the present invention provides an antibody-drug conjugate produced through a method for site-specifically delivering a target substance (e.g., a drug) to an antibody, and a use thereof.

### [Technical Solution]

The present invention provides an antibody-drug conjugate including an anti-claudin18.2 antibody. The antibody-drug conjugate of the present invention is characterized in that a drug is site-specifically linked to an antibody.

### [Advantageous Effects]

The present invention provides a pharmaceutical composition for treating cancer, including an antibody-drug conjugate including an anti-claudin18.2 antibody.

Furthermore, the present invention provides a method for treating cancer, using an antibody-drug conjugate including an anti-claudin18.2 antibody.

Furthermore, the present invention provides a use of an antibody-drug conjugate including an anti-claudin18.2 antibody for treating cancer.

Furthermore, the present invention provides a use of an antibody-drug conjugate including an anti-claudin18.2 antibody for the manufacture of a medication for treating cancer.

### [Description of Drawings]

FIG. 1 shows a graph illustrating the absorbance measured after a CHO-K1 cell line (MOCK CHO-K1) to which only an NOCK vector (empty vector) was transiently transfected was treated with each of antibody-A and ADC-A.
FIG. 2 shows a graph illustrating the absorbance measured after a CHO-K1 cell line (Claudin18.1 CHO-K1) to which a gene (SEQ ID NO: 18) encoding a claudin18.1 protein (CLDN18.1) was transiently transfected was treated with each of antibody-A and ADC-A.
FIG. 3 shows a graph illustrating the absorbance measured after a CHO-K1 cell line (Claudin18.2 CHO-K1) to which a gene (SEQ ID NO: 19) encoding a claudin18.2 protein (CLDN18.2) was transiently transfected was treated with each of antibody-A and ADC-A.
FIG. 4 shows a graph illustrating the absorbance measured after CLDN18.2~virus-like particle (VLP) expressing the claudin18.2 protein (CLDN18.2) was treated with each of antibody-A and ADC-A.
FIG. 5 shows a graph illustrating the absorbance measured after a MIA PaCa-2~CLDN18.2 cell line was treated with each of antibody-A, ADC-A, antibody-B, and ADC-B.
FIG. 6 shows a graph illustrating the absorbance measured after an SNU601 cell line was treated with each of antibody-A, ADC-A, antibody-B, and ADC-B.
FIG. 7 shows a graph illustrating the absorbance measured after a PATU8988S cell line were treated with each of antibody-A, ADC-A, antibody-B, and ADC-B.
FIG. 8 shows a graph illustrating the absorbance measured after a MIA PaCa-2 (CLDN18.2-) cell line was treated with each of antibody-A, ADC-A, antibody-B, and ADC-B.
FIG. 9 shows a graph illustrating the level of internalization measured every hour after the MIA PaCa-2~CLDN18.2 cell line was treated with each of antibody-A and ADC-A. At this time, the proportion of the red area of the vertical axis means the proportion of the area of antibody-A or ADC-A that has permeated into the cells (area measured as the red dots) in the total area occupied by the cells.
FIG. 10 shows a graph illustrating the level of internalization measured every hour after the MIA PaCa-2 (CLDN18.2-) cell line was treated with each of antibody-A and ADC-A. At this time, the proportion of the red area of the vertical axis means the proportion of the area of antibody-A or ADC-A that has permeated into the cells (area measured as the red dots) in the total area occupied by the cells.
FIG. 11 shows a graph illustrating the level of internalization measured every hour after the SNU601 cell line was treated with each of antibody-A and ADC-A. At this time, the proportion of the red area of the vertical axis means the proportion of the area of antibody-A or ADC-A that has permeated into the cells (area measured as the red dots) in the total area occupied by the cells.
FIG. 12 shows a graph illustrating the changes in cell viability measured after the MIA PaCa-2~CLDN18.2 cell line was treated with each of antibody-A, ADC-A, antibody-B, ADC-B, and a combination of antibody-A and MMAE at various concentrations.
FIG. 13 shows a graph illustrating the changes in cell viability measured after the PATU8988S cell line was treated with each of antibody-A, ADC-A, antibody-B, ADC-B, and a combination of antibody-A and MMAE at various concentrations.
FIG. 14 shows a graph illustrating the changes in cell viability measured after the SNU601 cell line was treated with each of antibody-A, ADC-A, antibody-B, ADC-B, and a combination of antibody-A and MMAE at various concentrations.
FIG. 15 shows a graph illustrating the changes in cell viability measured after the NUGC4 cell line was treated with each of antibody-A, ADC-A, antibody-B, ADC-B, and a combination of antibody-A and MMAE at various concentrations.
FIG. 16 shows a graph illustrating the changes in cell viability measured after the MIA PaCa-2 (CLDN18.2-) cell line was treated with each of antibody-A, ADC-A, antibody-B, ADC-B, and a combination of antibody-A and MMAE at various concentrations.
FIG. 17 shows a graph illustrating the changes in cell viability measured after the AGS cell line was treated with each of antibody-A, ADC-A, antibody-B, ADC-B, and a combination of antibody-A and MMAE at various concentrations.
FIG. 18 shows a graph illustrating the relative percentage of total antibodies or total ADC measured after human plasma was treated with each of antibody-A, ADC-A and ADC-B, and the supernatant was extracted from samples incubated for various periods of time.
FIG. 19 shows a graph illustrating the relative percentage of total antibodies or total ADC measured after monkey plasma was treated with each of antibody-A, ADC-A and ADC-B, and the supernatant was extracted from samples incubated for various periods of time.
FIG. 20 shows a graph illustrating the relative percentage of total antibodies or total ADC measured after rat plasma was treated with each of antibody-A, ADC-A and ADC-B, and the supernatant was extracted from samples incubated for various periods of time.
FIG. 21 shows a graph illustrating the relative percentage of total antibodies or total ADC measured after mouse plasma was treated with each of antibody-A, ADC-A and ADC-B, and the supernatant was extracted from samples incubated for various periods of time.
FIG. 22 shows a graph illustrating the tumor volume measured after each of antibody-A, ADC-A, and ADC-C was intravenously injected into tumor model mice under various conditions (G1 to G8 groups).
FIG. 23 shows a graph illustrating the body weight measured after each of antibody-A, ADC-A, and ADC-C was intravenously injected into tumor model mice under various conditions (G1 to G8 groups).
FIG. 24 shows comparative photographs of tumors autopsied 28 days after intravenous injection of each of antibody-A, ADC-A, and ADC-C into tumor model mice under various conditions (G1 to G8 groups).
FIG. 25 shows a graph illustrating the weight of tumors autopsied 28 days after intravenous injection of each of antibody-A, ADC-A, and ADC-C into tumor model mice under various conditions (G1 to G8 groups).
FIG. 26 shows a graph illustrating the concentrations of total antibodies and total ADC measured after a certain period of time after ADC-A was intravenously administered to rats at various concentrations.
FIG. 27 shows the structure of ADC-A.
FIG. 28 shows the detailed structure of the linker and drug moiety included in ADC-A.
FIG. 29 shows some reaction processes during the manufacturing process of ADC-A.
FIG. 30 shows the HIC-HPLC analysis results of antibody-A.
FIG. 31 shows the HIC-HPLC analysis results of compound 3.
FIG. 32 shows the HIC-HPLC analysis results of crude ADC-A.

### [Best modes of the Invention]

Some embodiments of the present application provide an antibody-drug conjugate.

Some embodiments of the present application provide an antibody-drug conjugate having a structure of Formula 1: 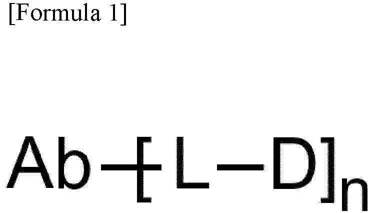 wherein
wherein, Ab is an antibody unit,
L is a linker unit,
D is a drug unit,
n is an integer of 1 to 4,
the antibody unit is a conjugated anti-claudin18.2 antibody,
the drug unit is linked to one or more of lysine residue 246 (K246) and lysine residue 248 (K248) of an Fc region of the antibody unit,
the linker unit has a structure of Formula 2: 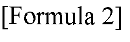 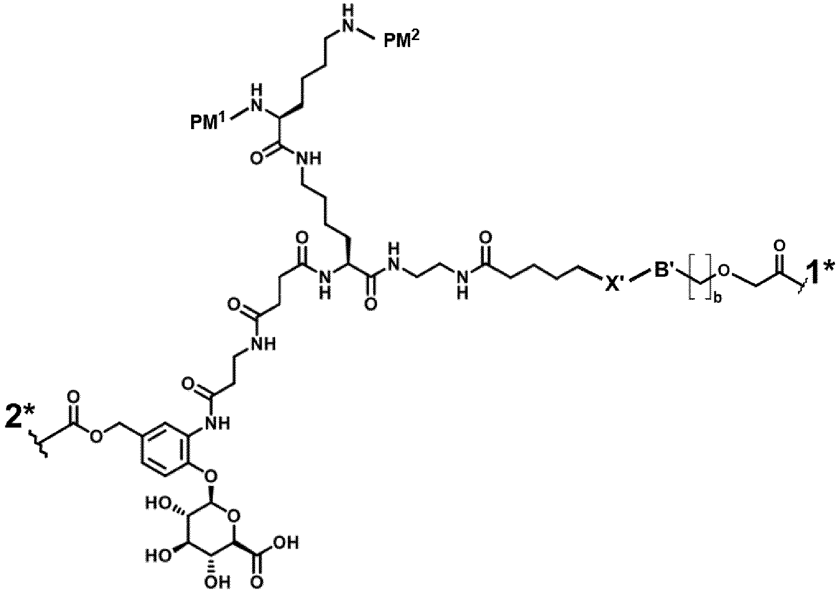
   wherein, b is an integer of 0 to 6,
   X' is NH-, -C(O)-, or -NHC(O)-,
   B' is a group formed by a click-chemistry reaction between click-chemistry functional groups,
   PM¹ and PM² are each independently a polyethylene (PEG) moiety, the PEG moiety includes 1 to 10 ethylene glycol units, and the ethylene glycol unit is CH₂OCH₂-, -OCH₂CH₂- or -CH₂CH₂O-,
   1* represents an attachment site with Ab,
   2* represents an attachment site with D, and
   the drug unit is a monomethyl auristatin E (MMAE).

In a specific embodiment, B' may include one structure selected from 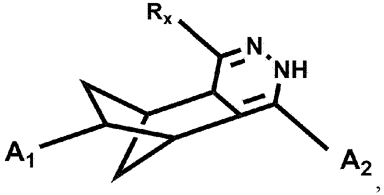 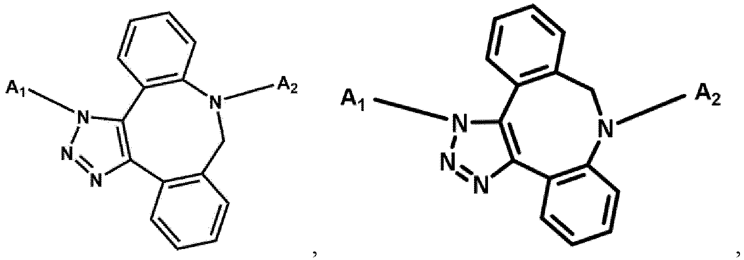 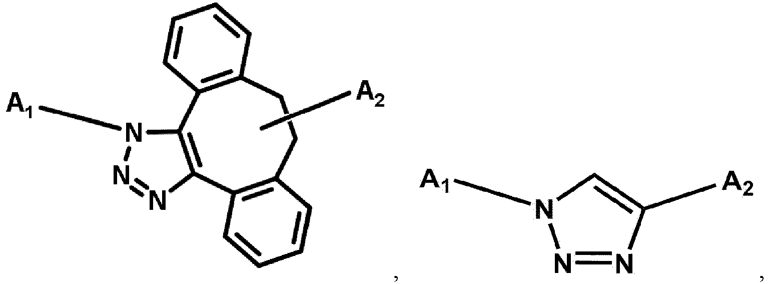 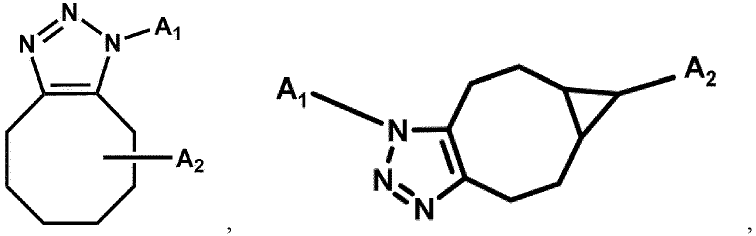 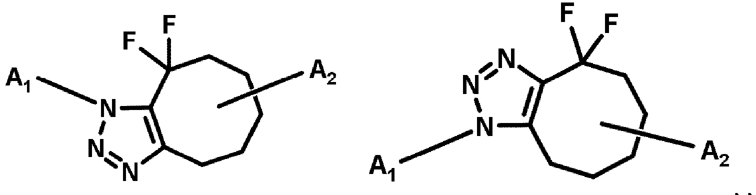 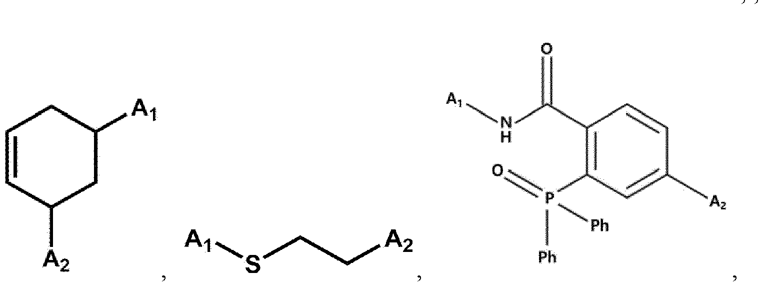 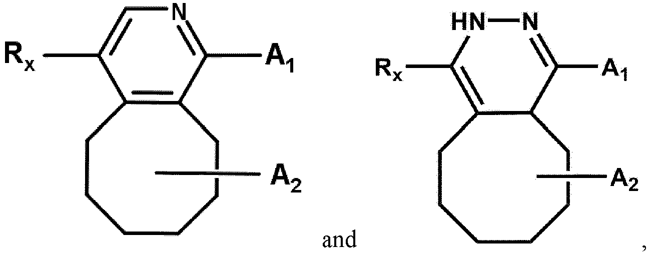
wherein, Rₓ may be selected from H, halogen, and C₁₋₃ alkyl, and
A₁ and A₂ may each represent an attachment site with the remaining structure of the linker unit.

In a specific embodiment, b may be 2.

In a specific embodiment, X' may be -C(O)-.

In a specific embodiment, PM¹ and PM² each independently represent a PEG moiety,
the PEG moiety may include 1 to 10 ethylene glycol units, and
the ethylene glycol unit may be -[CH₂OCH₂]-, -[OCH₂CH₂]- or - [CH₂CH₂O]-.

In a specific embodiment, PM¹ and PM² may each have the following structure:

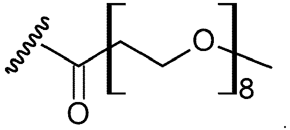

In a specific embodiment, the drug unit may have a structure of the following Formula 5: 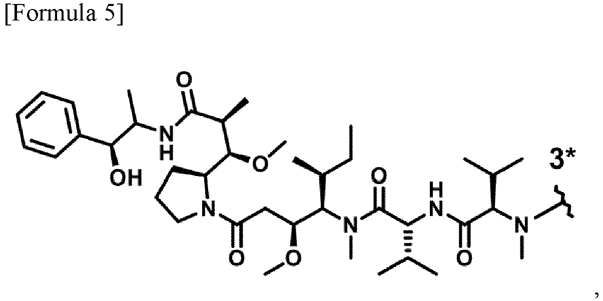 wherein, 3* may represent an attachment site with L.

In a specific embodiment, the structure of the conjugated anti-claudin18.2 antibody may be the same as that of the anti-claudin18.2 antibody, except for the conjugated portion with the linker unit. At this time, the conjugated portion is K246 or K248 of the heavy chain of the anti-claudin18.2 antibody.

In a specific embodiment, the anti-claudin18.2 antibody may be an IgG antibody, and the IgG antibody may be selected from the subclasses of IgG1, IgG2, IgG3, and IgG4

In a specific embodiment, the heavy chain of the anti-claudin18.2 antibody may include CDRH1 represented by an amino acid sequence of SEQ ID NO: 10, CDRH2 represented by an amino acid sequence of SEQ ID NO: 11, and CDRH3 represented by an amino acid sequence of SEQ ID NO: 12, and the light chain of the anti-claudin18.2 antibody may include CDRL1 represented by an amino acid sequence of SEQ ID NO: 13, CDRL2 represented by an amino acid sequence of SEQ ID NO: 14, and CDRL3 represented by an amino acid sequence of SEQ ID NO: 15.

In a specific embodiment, the anti-claudin18.2 antibody may include a heavy chain represented by an amino acid sequence of SEQ ID NO: 16, and a light chain represented by an amino acid sequence of SEQ ID NO: 17.

In a specific embodiment, n may be 2,
the antibody unit may include two heavy chains (a first heavy chain and a second heavy chain),
the antibody-drug conjugate may include two drug units (a first drug unit and a second drug unit),
the first drug unit may be linked to one of K246 and K248 of the first heavy chain, and
the second drug unit may be linked to one of K246 and K248 of the second heavy chain.

In a specific embodiment, the antibody-drug conjugate may have a structure of the following Formula 7:

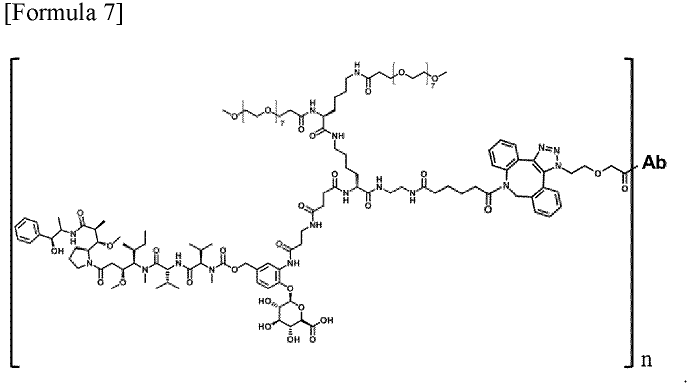

Some embodiments of the present application provide a pharmaceutical composition for treating cancer, including a therapeutically effective amount of an antibody-drug conjugate.

At this time, the pharmaceutical composition for treating cancer may further include a pharmaceutically acceptable carrier and/or a pharmaceutically acceptable adjuvant.

Some embodiments of the present invention provide a method for treating cancer, including:

administering a pharmaceutical composition including a therapeutically effective amount of an antibody-drug conjugate

Some embodiments of the present invention provide a use of an antibody-drug conjugate for treating cancer.

Some embodiments of the present invention provide a use of an antibody-drug conjugate for a manufacture of a medication for treating cancer.

### [Mode of the Inventions]

Hereinafter, the content of the invention will be described in more detail through embodiments and examples. The invention disclosed by the present application can be implemented in various forms, and is not limited to specific embodiments described herein.

A person with ordinary skill in the art to which the invention disclosed in the present application pertains will be able to conceive of various modifications and other aspects of the content of the invention disclosed in the present application. Therefore, it should be understood that the content of the invention disclosed in the present specification is not limited to the specific embodiments or examples described herein, and modifications thereof and other embodiments are also included within the invention disclosed in the present application.

### Explanation of terms

Unless otherwise described, all technical and scientific terms used in the present application have the same meaning as commonly understood by those skilled in the art to which the present application pertains. All publications, patents, and other references mentioned herein are incorporated by reference in their entireties.

"Halogen" or "halo" refers to a group including fluorine, chlorine, bromine, and iodine, which are included in the halogen group of elements in the periodic table.

As used herein, the term "hetero" refers to a compound or group including one or more heteroatoms. That is, the term hetero can be used with a term used to refer to a molecule itself or a term used to refer to a portion of a molecule. For example, heteroalkylene refers to an alkylene group including one or more heteroatoms in the main chain. As another example, heteroaryl refers to an aryl group including one or more heteroatoms on a ring (for example, a C₆ aryl group in which one or more carbons on a ring are each substituted with an independently selected heteroatom). The term "heteroatom" refers to an atom other than carbon or hydrogen, and includes for example, B, Si, N, P, O, S, F, Cl, Br, I and Se, and the like. Preferably, the term includes a polyvalent element such as N, O, and S. For example, when a structure includes one or more heteroatoms, each heteroatom may be independently selected from N, O, and S.

The term "alkyl" or "alkane" used to refer to a molecule by itself or to refer to a part of a molecule is used to mean a fully saturated straight-chained or branched hydrocarbon group. The straight chain and branched alkyl groups are, for example, methyl, ethyl, n-propyl, iso-propyl, n-butyl, sec-butyl, tert-butyl, iso-butyl, pentyl, hexyl, heptyl, octyl, nonyl, decyl, and the like. The alkyl group may include a cyclic structure. The term "C_{x-y}" is intended to include residues including x to y carbon atoms in the chain or ring, for example, when used with the term alkyl. For example, the term "C_{x-y} alkyl" may mean including x to y carbon atoms as a substituted or unsubstituted, chained alkyl group, branched alkyl group, or alkyl group including a cyclic structure. C₀ alkyl means hydrogen. Examples of the C₁₋₄ alkyl include methyl, ethyl, n-propyl, iso-propyl, n-butyl, sec-butyl, tert-butyl, and iso-butyl, but are not limited thereto. For example, a straight-chained or branched alkyl group may have 1 to about 60, 1 to 20, or 1 to 10 carbon atoms.

As used herein, the term "heteroalkyl" refers to an alkyl including one or more heteroatoms. In this case, the heteroatom is each independently selected.

The term "alkylene" used to refer to a molecule by itself or to refer to a part of a molecule means a divalent radical derived from alkyl. The term "alkylene" may be used with the term "substituted" or "unsubstituted", as needed. When the term "alkylene" is not used with the term "substituted" or "unsubstituted," the term "alkylene" is intended to encompass the aspects of substituted and unsubstituted alkylene. For example, alkylene may refer to a group having 1 to 100 carbon atoms in the main chain. Examples of the alkylene may include -CH₂-, -CH₂CH₂-, -CH₂CH₂CH₂-, and - CH₂CH₂CH₂CH₂-, but are not limited thereto. For example, alkylene may be used as C₂ alkylene, which refers to an alkylene group having two carbon atoms in the main chain. Illustratively, "C_{x-y} alkylene" is used herein to mean substituted or unsubstituted alkylene having X to Y carbon atoms in the main chain.

The term "heteroalkylene" used to refer to a molecule by itself or to refer to a part of a molecule means a divalent radical derived from heteroalkyl. The term "heteroalkylene" may be used with the term "substituted" or "unsubstituted", if necessary. When the term "heteroalkylene" is not used with the term "substituted" or "unsubstituted," the term "heteroalkylene" is intended to encompass the aspects of substituted and unsubstituted heteroalkylene. For example, heteroalkylene may refer to a group having 1 to 100 carbon atoms and heteroatoms in the main chain (for example, the sum of the number of carbon atoms and the number of heteroatoms in the main chain is 1 to 100). Examples of the heteroalkylene group include -CH₂-CH₂-O-CH₂-CH₂-, and -CH₂-O-CH₂-CH₂-NH-CH₂-, but are not limited thereto. The heteroalkylene group may comprise one or more heteroatoms, and each heteroatom may be the same or different. For example, the heteroalkylene group may comprise one or more heteroatoms at a position that is not the end of a chain or branch, and each heteroatom may be the same or different. For example, the heteroalkylene group may comprise one or more heteroatoms at each end of a chain or branch or all the ends of the chain or branch, and each heteroatom may be the same or different. Illustratively, "C_{x-y} heteroalkylene" in the present specification is used to refer to substituted or unsubstituted heteroalkylene having a total of x to y atoms in the main chain (for example, the sum of the number of carbon atoms and the number of heteroatoms located in the main chain is x to y). For example, C₃ heteroalkylene may be used to mean a heteroalkylene having two carbon atoms and one heteroatom in the main chain. As another example, C₅ heteroalkylene may be used to mean a heteroalkylene having 3 carbon atoms and 2 heteroatoms in the main chain. C₅ heteroalkylene encompasses a structure such as, for example, -CH₂-CH₂-O-CH₂-CH₂- and -CH₂-O-CH₂-NH-CH₂- and the like.

The term "cycloalkyl" is used to refer to a fully saturated cyclic hydrocarbon group. The "cycloalkyl" comprises monocyclic and polycyclic groups. Unless otherwise defined, a monocyclic cycloalkyl group generally has 3 to about 20, preferably 3 to 10 carbon atoms on the ring. A ring other than the first ring of the polycyclic cycloalkyl may be selected from saturated, unsaturated and aromatic rings. The cycloalkyl comprises bicyclic molecules in which one, two or three or more atoms are shared between two rings. The term "fused cycloalkyl" refers to a polycyclic cycloalkyl group in which each ring shares two adjacent atoms with other rings. A ring other than the first ring of the fused polycyclic cycloalkyl may be selected from saturated, unsaturated and aromatic rings. The cycloalkyl may be used with the term substituted or unsubstituted, and the substituted cycloalkyl refers to a group provided when one or more hydrogen atoms linked to carbon atom on the ring are substituted with one or more independent substituents. Furthermore, the cycloalkyl may be used with the term hetero, wherein heterocycloalkyl refers to a cycloalkyl group comprising one or more heteroatoms on the ring.

The term "cycloalkylene" is used to mean a divalent radical derived from cycloalkyl. For example, the term cycloalkylene may be used with the term substituted or unsubstituted. For example, the term cycloalkylene may be used with the term hetero.

The term "alkene" or "alkenyl" used to refer to a molecule by itself or to refer to a part of a molecule comprises one or more double bonds as a straight-chained or branched non-aromatic hydrocarbon. For example, a straight-chained or branched alkenyl group may have 2 to about 60, 2 to 20, or 2 to 10 carbon atoms.

The term "heteroalkene" or "heteroalkenyl" means an alkenyl comprising one or more heteroatoms. In this case, the heteroatom is each independently selected.

The term "alkenylene" used to refer to a molecule by itself or to refer to a part of a molecule means a divalent radical derived from alkenyl. The term "alkenylene" may be used with the term "substituted" or "unsubstituted", as needed. When the term alkenylene is not used with the term substituted or unsubstituted, the term alkenylene is intended to encompass the aspects of substituted and unsubstituted alkenylene. For example, alkenylene may refer to a group having 2 to 100 carbon atoms in the main chain. Examples of the alkenylene may include -C=C-, -C-C-C=C-C=C-, or -C-C-C-C=C-, and the like, but are not limited thereto. In the present specification, when used with "C_{x-y} alkenylene," C_{x-y} alkenylene is used to mean a substituted or unsubstituted alkenylene having x to y carbon atoms in the main chain.

The term "heteroalkenylene" used to refer to a molecule by itself or to refer to a part of a molecule means a divalent radical derived from heteroalkenyl. For example, the term "heteroalkenylene" may be used to refer to an alkenylene group comprising one or more heteroatoms in the main chain. For example, heteroalkenylene may refer to a group having 2 to 100 carbon atoms and heteroatoms in the main chain (for example, the sum of the number of carbon atoms and the number of heteroatoms is 2 to 100). The term "heteroalkenylene" may be used with the term "substituted" or "unsubstituted", as needed. In the present specification, when used with "C_{x-y} heteroalkenylene," C_{x-y} heteroalkenylene is used to mean a substituted or unsubstituted heteroalkenylene having the number of x to y carbon atoms and heteroatoms (for example, the sum of the number of carbon atoms and the number of heteroatoms is x to y) in the main chain.

The term "cycloalkene" or "cycloalkenyl" is a cyclic hydrocarbon comprising one or more double bonds on the ring. The "cycloalkenyl" comprises monocyclic and polycyclic groups. Unless otherwise defined, monocyclic cycloalkenyl generally has 3 to about 20, preferably 3 to 10 carbon atoms on the ring. A ring other than the first ring of the polycyclic cycloalkeynyl may be selected from saturated, unsaturated and aromatic rings. The cycloalkeynyl comprises bicyclic molecules in which one, two or three or more atoms are shared between two rings. The term "fused cycloalkenyl" means a polycyclic cycloalkenyl in which each ring shares two adjacent atoms with other rings. A ring other than the first ring of the fused polycyclic cycloalkeynyl may be selected from saturated, unsaturated and aromatic rings. The term "cycloalkenyl" may be used with the term "substituted" or "unsubstituted," and the substituted cycloalkenyl refers to a group provided when one or more hydrogen atoms linked to carbon atom on the ring are substituted with one or more independent substituents. Furthermore, the term "cycloalkenyl" may be used with the term "hetero", wherein heterocycloalkenyl refers to a cycloalkenyl group comprising one or more heteroatoms on the ring.

The term "cycloalkenylene" is used to mean a divalent radical derived from cycloalkenyl. For example, the term cycloalkenylene may be used with the term substituted or unsubstituted. For example, the term cycloalkenylene may be used with the term hetero.

The term "alkyne" or "alkynyl" used to refer to a molecule by itself or to refer to a part of a molecule comprises one or more triple bonds as a straight-chained or branched non-aromatic hydrocarbon. For example, a straight-chained or branched alkynyl group may have 2 to about 60, 2 to 20, or 2 to 10 carbon atoms.

The term "heteroalkynyl" or "heteroalkyne" means an alkynyl comprising one or more heteroatoms. In this case, the heteroatom is each independently selected.

The term "alkynylene" used to refer to a molecule by itself or to refer to a part of a molecule means a divalent radical derived from alkynyl. The term "alkynylene" may be used with the term "substituted" or "unsubstituted," as needed. When the term alkynylene is not used with the term of substituted or unsubstituted, the term alkynylene is intended to encompass the aspects of substituted and unsubstituted alkynylene. For example, alkynylene may refer to a group having 2 to 100 carbon atoms in the main chain. In the present specification, when used with "C_{x-y} alkynylene," C_{x-y} alkynylene is used to mean a substituted or unsubstituted alkynylene having x to y carbon atoms in the main chain.

The term "heteroalkynylene" used to refer to a molecule by itself or to refer to a portion of a molecule means a divalent radical derived from heteroalkynyl. For example, the term "heteroalkynylene " may be used to refer to an alkynylene group comprising one or more heteroatoms in the main chain. For example, heteroalkynylene may refer to a group having 2 to 100 carbon atoms and heteroatoms in the main chain (for example, the sum of the number of carbon atoms and the number of heteroatoms in the main chain is 2 to 100). The term "heteroalkynylene" may be used with the term "substituted" or "unsubstituted," as needed. In the present specification, when used with "C_{x-y} heteroalkynylene," C_{x-y} heteroalkynylene is used to mean a substituted or unsubstituted heteroalkynylene having x to y carbon atoms and heteroatoms (for example, the sum of the number of carbon atoms and the number of heteroatoms is x to y) in the main chain.

The term "cycloalkyne" or "cycloalkynyl" refers to a cyclic hydrocarbon comprising one or more triple bonds on the ring, and is also referred to as a "strained alkyne." The "cycloalkynyl" comprises monocyclic and polycyclic groups. Unless otherwise defined, monocyclic cycloalkynyl generally has 3 to about 10 carbon atoms on the ring. A ring other than the first ring of the polycyclic cycloalkynyl may be selected from saturated, unsaturated and aromatic rings. The cycloalkynyl comprises bicyclic molecules in which one, two or three or more atoms are shared between two rings. The term "fused cycloalkynyl" means a polycyclic cycloalkynyl in which each ring shares two adjacent atoms with other rings. A ring other than the first ring of the fused polycyclic cycloalkynyl may be selected from saturated, unsaturated and aromatic rings. The term "cycloalkynyl" may be used with the term "substituted" or "unsubstituted," and the substituted cycloalkynyl refers to a group provided when one or more hydrogen atoms linked to carbon atom on the ring are substituted with one or more independent substituents. Furthermore, the term "cycloalkynyl" may be used with the term "hetero," wherein heterocycloalkynyl refers to a cycloalkynyl group comprising one or more heteroatoms on the ring.

The term "cycloalkynylene" is used to mean a divalent radical derived from cycloalkynyl. For example, the term cycloalkynylene may be used with the term substituted or unsubstituted. For example, the term cycloalkynylene may be used with the term hetero.

The term "aryl" is used to refer to a group comprising an aromatic ring, and refers to a group derived from arene which is an aromatic compound. The term aryl comprises monocyclic and polycyclic groups. The term "aryl" may be used with the term "hetero," and the heteroaryl is used to refer to an aryl group having one or more heteroatoms on the ring. The term "aryl" may be used with the term "substituted" or "unsubstituted," and the substituted aryl refers to an aryl group in which one or more hydrogen atoms linked to carbon atom on the ring are substituted with one or more substituents. The term "aryl" may be used to encompass both substituted or unsubstituted aryl and substituted or unsubstituted heteroaryl. Examples of aryl include phenyl, pyridyl, naphthyl, biphenyl, and the like, but are not limited thereto.

The term "arylene" is used to mean a divalent radical derived from aryl. For example, the term arylene may be used with the term substituted or unsubstituted. For example, the term arylene may be used with the term hetero. The term arylene may be used to encompass all of the substituted or unsubstituted arylene and the substituted or unsubstituted heteroarylene.

As used herein, the term "substituted" means that, in which the valence of the atom is normal and a substituted compound is stable, one or more hydrogen atoms on an atom are substituted with a substituent including deuterium and hydrogen variants. When the substituent is oxygen (that is, =O), this means that two hydrogen atoms are substituted. When one substituent is a halogen (for example, Cl, F, Br, and I, and the like), this means that one hydrogen atom is substituted with a halogen. When two or more substituents are present in one group, the substituents present in the group may be the same or different. Unless otherwise specified, the type and number of substituents may be arbitrary as long as chemically achievable. Illustratively, the substituent may be selected from -R, =O, =S, -NO₂, -CR₃, -NR₂, =NR, -OR, -SR, -C(=O)R, -C(=O)CR₃, -C(=O)OR, and -C(=O)NR₂, wherein R may be each independently selected from H, halogen, C₁₋₆ alkyl, C₃₋₁₀ cycloalkyl, C₃₋₁₀ heterocycloalkyl, aryl, heteroaryl, -OH, -NH₂, -COOH, =O, =S, and -SH, but are not limited thereto (provided that the substituent is not -H). Representative examples of the substituent include -C₁₋₄ alkyl, -C(=O)H, -C(=O)CH₃, -C(=O)OH, - C(=O)NH₂, -NH₂, =NH, =O, =S, -OH, -NO₂ and -SH, but are not limited thereto. The term substituted or unsubstituted may be used with a term used to refer to a molecule itself or a term used to refer to a part of a molecule. For example, substituted C₁₀₋₂₀ alkylene may mean that one or more hydrogen atoms linked to the main chain are substituted with substituents, wherein each substituent may be independently selected.

In the present specification, when expressing the structure of a compound, a wavy line drawn in a direction roughly perpendicular to the bond (for example, 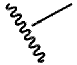 ) is used to indicate a portion where a group and another group are linked. For example, when expressed as a structure 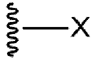 , it indicates that group X in a material, molecule, or compound is linked to another part through a bond. For example, when expressed as a structure 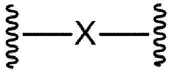 , it indicates that group X in a material, molecule, or compound is linked to other parts through a bond. For example, in a compound having the structure of "A-X," when only the structure of group X is illustrated, it may be expressed as a structure 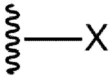 . For example, in a compound having the structure "A-X-B," when only the X structure is illustrated, it may be expressed as a structure 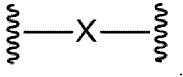 As needed, a wavy line drawn almost perpendicular to the bond may be expressed through an additional notation. For example, in a compound having the structure of A-X-B, when only the structure of group X is illustrated, the structure of group X may be illustrated as 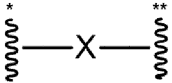 as needed, wherein by mentioning "* is a portion linked to A and ** is a portion linked to B," it is possible to provide information about what part each wavy line indicates linkage to.

Furthermore, a wavy line drawn in a direction roughly perpendicular to the bond indicates that "a structure illustrated with the wavy line" is covalently and directly linked to "a group other than the structure illustrated with the wavy line." The wavy line should not be construed to mean that other additional elements may be included between "a structure illustrate with the wavy line" and "a group other than the structure illustrated with the wavy line." When additional elements may be included, it will be described through separate related descriptions.

The structure " 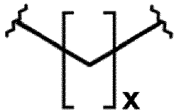 " as used in the structures or formulas disclosed herein is used to mean Cₓ alkylene. For example, the structure 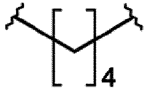 may be used to represent a C₄ alkylene such as -CH₂-CH₂-CH₂-CH₂-. Herein, the case where x is 0 means a bond. In other words, the structure 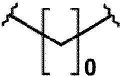 may be represented by the structure

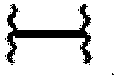

Compounds of the present invention may have a specific geometric or stereoisomeric form. When compounds are disclosed in the present application without being specified, isomers such as cis and trans isomers, (-)- and (+)-enantiomers, (R)- and (S)-enantiomers, diastereoisomers, (D)-isomers, (L)-isomers, and racemates of the compounds are included in the scope of the present application. In other words, when a formula or structure disclosed herein does not have a notation associated with isomers (e.g., * 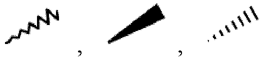 , etc.), it means that the disclosed Formula or structure includes all possible isomers.

As used herein, the term "amino acid" may be used to refer to both amino acids that are not bonded to other amino acids and amino acid residues that are bonded to other amino acids included in proteins or peptides, and may be interpreted appropriately according to the content or context of the paragraph in which the term amino acid is used. As used herein, the term "amino acid" may be used to include both natural and unnatural amino acids. As used herein, natural amino acids refer to 20 types of amino acids that are synthesized in the human body through gene transcription and translation processes. Specifically, the natural amino acids include alanine (Ala, A), arginine (Arg, R), asparagine (Asn, N), aspartic acid (Asp, D), cysteine (Cys, C), glutamic acid (Glu, E), glutamine (Gln, Q), glycine (Gly, G), histidine (His, H), isoleucine (Ile, I), leucine (Leu, L), lysine (Lys K), methionine (Met, M), phenylalanine (Phe, F), proline (Pro, P), serine (Ser, S), threonine (Thr, T), tryptophan (Trp, W), tyrosine (Tyr, Y), and valine (Val, V). As used herein, unnatural amino acids mean amino acids that are not synthesized in the human body through gene transcription and translation processes, are synthesized through other processes that are not transcription and translation processes, or are artificially synthesized, or can be synthesized by other organisms that are not human. The unnatural amino acids may include, for example, ornithine (Orn), diaminopropionic acid (Dap), diaminobutyric acid (Dab), naphthylalanine, and the like. As described above, the term "amino acid" as used herein may be used to refer to both amino acids that are not bonded to other amino acids and amino acid residues that are bonded to other amino acids included in proteins or peptides. For example, alanine may be used to refer to alanine and/or alanine residue. For example, arginine may be used to refer to arginine and/or arginine residue. As used herein, the term "amino acid" may be used to include both L-type and D-type amino acids. In some embodiments, when there is no mention to L-type or D-type, an amino acid may be interpreted as an L-type amino acid.

As used herein, the term "amino acid residue" refers to a structure derived from amino acids included in compounds, peptides, and/or proteins (for example, antibodies, and the like), which are covalently linked to other parts of the compounds, peptides, and/or proteins. For example, when alanine, arginine, and glutamic acid are linked through an amide bond to form a peptide having an ARE sequence, the peptide comprises three amino acid residues, wherein A, R, and E may be referred as an alanine residue, an arginine residue, and a glutamic acid residue, respectively. Furthermore, as described above, in the peptide having an ARE sequence, the peptide may comprise three amino acids, and A, R, and E may also be referred to as alanine, arginine, and glutamic acid, respectively. As another example, when aspartic acid, phenylalanine, and lysine are linked through an amide bond to form a peptide having a DFK sequence, the peptide comprises three amino acid residues, wherein D, F, and K may be referred to as an aspartic acid residue, a phenylalanine residue, and a lysine residue, respectively. Furthermore, as described above, in the peptide having a DFK sequence, the peptide may comprise three amino acids, and D, F, and K may also be referred to as aspartic acid, phenylalanine, and lysine, respectively.

Unless otherwise stated, when describing an amino acid sequence herein, one-letter notation or three-letter notation of an amino acid is used, and it is described in the direction from the N-terminus to the C-terminus. For example, when expressed as RNVP, it refers to a peptide in which arginine, asparagine, valine, and proline are sequentially linked in the direction from the N-terminus to the C-terminus. As another example, when expressed as Thr-Leu-Lys, it refers to a peptide in which threonine, leucine, and lysine are sequentially linked in the direction from the N-terminus to the C-terminus. In the case of amino acids that cannot be represented by the one-letter notation, other letters are used to describe these amino acids, and additional description will be provided. The sequences set forth herein may include sequences having 90%, 91%, 92%, 93%, 94%, 95%, 96%, 97%, 98%, or 99% or more sequence identity with the set forth sequence, provided that the desired function is identical.

As used herein, the term "click-chemistry" refers to a chemical concept introduced by K. Barry Sharpless of the Scripps Research Institute to describe complementary chemical functional groups and chemical reactions designed such that two molecules can rapidly and stably form a covalent bond. The click chemistry of the present specification does not mean a specific reaction but means a concept for a fast and stable reaction. In one embodiment, several conditions should be satisfied in order to form bonds between molecules by click-chemistry. The above conditions include high yield rate, excellent selectivity to reactive sites, operation in a modular manner to combine molecules organically, and proceeding in a thermodynamically stabilized direction to create a product fast and accurately. The click-chemistry of the present specification includes the reaction of mutually reactive pairs in click-chemistry functional groups (for example, including terminal alkyne, azide, strained alkyne, diene (for example, Diels-Alder diene), dienophile (for example, Diels-Alder dienophile), trans-cyclooctene, alkene, thiol, tetrazine, triazine, dibenzocyclooctyne (DBCO) and bicyclononyne (including bicyclo[6.1.0]non-4-yne)). Examples of click chemical reactions include Huisgen 1,3-dipolar cycloaddition (see Tornoe et al. Journal of Organic Chemistry (2002) 67: 3075-3064, and the like); Diels-Alder reaction; inverse electron demand Diels-Alder reaction; nucleophilic addition to small strained rings like epoxide and aziridine; nucleophilic addition to an activated carbonyl group; Staudinger ligation; and an addition reaction to a carbon-carbon double bond or triple bond.

The term "antibody" as used herein is used to refer to an immunoglobulin molecule or a fragment thereof. Immunoglobulins are typically well known and have the ability to specifically bind to one or more antigens. The term "antibody" as used herein is also used to encompass a fragment thereof, so it is irrelevant that the antibody does not have the ability to bind to a specific antigen, as is the case of Fc fragments. Unless used with specific limitations in regard to antibodies, the term "antibody" may be interpreted without particular limitation to include all of monospecific antibodies, bispecific antibodies, trispecific antibodies, monoclonal antibodies, human antibodies, humanized antibodies, recombinant antibodies, chimeric antibodies, and the like. For example, an antibody may include two heavy chains and two light chains. For example, in this case, the antibody may have a structure in which two heavy chains are linked through one or more bridges (for example, a disulfide bond), one heavy chain and one light chain are linked through one or more bridges, and the other heavy chain and light chain are linked through one or more bridges. Antibodies may be divided into an Fc region (or Fc domain) and an Fab region, the Fab region including a site capable of binding to an antigen, and the Fc region including part of a constant region of a heavy chain. The term "antibody" as used herein may be used to include both conjugated and unconjugated antibodies (e.g., free antibodies).

In the present application, when reference is made to the numbering of amino acid residues in the Fc domain (or Fc region) of an antibody, the numbering of amino acid residues follows the EU numbering system, unless otherwise stated. The EU numbering system has been widely used as a sequencing system for the Fc region since the sequence of IgG was studied, as described in Edelman GM, et al., The covalent structure of an entire gammaG immunoglobulin molecule, Proc Natl Acad Sci USA., 1969 May; 63(1):78-85. For example, in lysine 246 in the Fc region, the number 246 is a number assigned according to the EU numbering system. As another example, in lysine 248 in the Fc region, the number 248 is a number assigned according to the EU numbering system.

The term "linked" or "linkage" as used herein means that two or more elements that are present within a conceptualizable structure are linked directly or indirectly (e.g., by other elements such as a linker), and is not intended to mean that other additional elements cannot be present between the two or more elements. For example, a description such as "element B linked to element A" is intended to include both a case where one or more other elements are included between element A and element B (that is, when element A is linked to element B through one or more other elements) and a case where one or more other elements are not present between element A and element B (that is, when element A and element B are directly linked), and is not interpreted in a limited manner.

The term "sequence identity" as used herein is a term used in relation with the degree of similarity between two or more sequences. For example, the term "sequence identity" is used with a term that refers to a reference sequence and a term that represents a proportion (e.g., a percentage). For example, the term "sequence identity" may be used to describe a sequence that is similar or substantially identical to a reference amino acid sequence. When a description such as "a sequence that has 90% or more sequence identity with sequence A" is used, the reference sequence herein is sequence A. For example, the percent sequence identity may be calculated by aligning a reference sequence and a sequence that is a target of measurement of the percentage of sequence identity. The method of calculating and/or determining the percent sequence identity is not particularly limited, and the percent sequence identity may be calculated and/or determined through any reasonable method or algorithm available to a person with ordinary skill in the art.

The term "unit" as used herein is used in some embodiments to distinguish conjugated substances from free substances. The term "unit" used in some embodiments will be described by exemplifying an antibody unit and a free antibody. The free antibody refers to an antibody molecule that is not covalently bound to other molecules or groups. The antibody unit refers to a group derived from a free antibody that is covalently linked to other molecules or groups. For example, when a free antibody and a functional substance are combined through the reaction of a reactive group of a functional group with an amine group of a lysine residue of the free antibody, an antibody-functional group conjugate may be prepared. At this time, a portion derived from the free antibody may be referred to as an antibody unit. In an antibody-functional group conjugate, the antibody unit may be understood to be structurally identical to the free antibody from which the antibody unit originates, except for the portion which is conjugated with a non-antibody portion of the antibody-functional group conjugate. For example, when the amine group of the lysine residue that participates in the reaction in a free antibody is separately illustrated, the structure of the free antibody may be expressed as " Ab-NH₂," In an antibody unit, when a junction between the antibody unit and a portion other than the antibody unit is separately illustrated, the structure of the antibody unit may be expressed as " 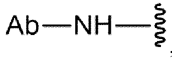 ," Thus, it can be understood that the antibody unit and the free antibody are structurally identical, except for the amine group of the lysine residue used in the reaction. Therefore, in some embodiments, the antibody unit and the free antibody may not be separately distinguished and may be referred to as "antibody," and these terms may be interpreted appropriately according to the context. In some embodiments, when a description such as "the antibody unit is derived from an antibody" is used, it can be understood that the antibody unit and the antibody have the above-described relationship. In some embodiments, when a description such as "the Fc binding unit is derived from a Fc binding substance" is used, it can be understood that the "Fc binding unit and the Fc binding substance" have a relationship similar to the above-described relationship. In other words, it can be understood that the Fc binding unit has the same structure as the Fc binding substance from which it originates, except for a junction where the Fc binding unit is joined to a portion other than the Fc binding unit. As in the case of the antibody unit, in some embodiments, an Fc binding unit may be referred to as an Fc binding substance from which it originates, and these terms may be interpreted appropriately according to the context.

The term "comprise" or "include" as used herein may be used to mean that other elements may additionally be present in addition to what is the object of the term "comprise" or "include" (e.g., an object) or that they are equivalent to what is the object of the term. For example, when a description such as "A includes B" is used, the description of "A includes B" should be construed as not excluding that A further includes additional components other than B. In other words, "A includes B" is intended to encompass cases such as the case where additional elements other than B are present in A (for example, the case where B and C are present in A), the case where A is B, the case where A consists of B, and the case where A is represented by B. Accordingly, when a description such as "A includes B" is used, the description may be modified into "A is B," "A consists of B," or "A is represented by B."

The term "have" as used herein may be used to mean that other elements may additionally be present in addition to what is the object of the term "have" (e.g., an object) or that they are equivalent to what is the object of the term. For example, when a description such as "A has B" is used, the description encompass cases such as the case where additional elements other than B are present in A (for example, the case where B and C are present in A), the case where A is B, the case where A consists of B, and the case where A is represented by B. Accordingly, when a description such as "A has B" is used, the description may be modified into "A is B," "A consists of B," or "A is represented by B."

When compounds (e.g., small compounds, peptides, antibodies, conjugates, and the like) are disclosed herein, it should be understood that their salt forms are also disclosed. Examples of ions that form salts of compounds include ammonium, calcium, sodium, potassium, acetate (CH₃COO⁻), carbonate (CO₃²⁻), chloride (Cl⁻), citrate, cyanide, fluoride (F⁻), nitrate (NO₃⁻), nitrite (NO₂⁻), phosphate (PO₃⁻), sulfate (SO₄²⁻), and the like, but are not particularly limited thereto. Salt-forming ions typically used in the art may be used for the formation of salts of the compounds, as needed. The salt may be, for example, a pharmaceutically acceptable salt, and in this case, the pharmaceutically acceptable salt refers to a salt that has the efficacy of a parent agent and is not biologically undesirable (for example, has little or no toxicity). Suitable salts include, for example, salts which may be formed by mixing a solution of a parent agent with a solution of a pharmaceutically acceptable acid such as hydrochloric acid, phosphoric acid, sulfuric acid, or acetic acid. For example, when a compound includes an acidic moiety, pharmaceutically acceptable salts thereof may include salts formed with suitable organic ligands such as alkali metal ions (sodium or potassium), alkaline earth metal ions (calcium or magnesium), and ammonium ions.

Hereinafter, the structure of an antibody will be specifically explained based on contents generally known in the art for better understanding, and the scope of the present application is not limited by the following description.

The structure of an antibody is divided into a heavy chain region and a light chain region, depending on the type of chain. The structure of an antibody is divided into a fragment antigen-binding region (Fab region) and a fragment crystallizable region (Fc region) according to its antigen-binding function. The structure of an antibody is divided into a variable region and a constant region according to the variability of the amino acid sequence. Other structures of an antibody include a hinge portion and a tail portion. The heavy chain region and the light chain region may be explained as functionally being broadly divided into a fragment antigen-binding region (Fab region) and a fragment crystallizable region (Fc region). The Fab region is a portion that comprises a portion which binds to an antigen (antigen-binding portion). The Fc region is a portion capable of binding to a fc receptor. The heavy chain region may be explained as having both a Fab region and a Fc region, and the light chain region may be explained as having a Fab region.

The Fab region of the heavy chain region comprises a heavy chain variable region (VH) and a heavy chain constant region 1 (CH1). For example, in IgG1, the Fc region is known to comprise a heavy chain constant region 2 (CH2) and a heavy chain constant region 3 (CH3). In this case, the entire heavy chain constant region of the antibody may be referred to as CH. For example, the entire region combining CH1, CH2, and CH3 of IgG1 may be expressed as CH.

The Fab region of the light chain region comprises a light chain variable region (VL) and a light chain constant region (CL). The light chain region may be explained as having no Fc region.

The above-described VH, CH1, CH2, CH3, VL, CL, and the like may each be referred to as an immunoglobulin domain.

The immunoglobulin domains included in the heavy chain region are known to be located in the order of VH, CH1, CH2, and CH3 or in the order of VH, CH1, CH2, CH3, and CH4 in the direction from the N-terminus to the C-terminus. The immunoglobulin domain included in the light chain region is known to be located in the order of VL and CL in the direction from the N-terminus to the C-terminus. In general, it is known that the heavy chain region and the light chain region are linked by a disulfide bond, and the Fab region and Fc region are linked by a hinge portion. Specifically, the C-terminal portion of CH1 and the N-terminal portion of CH2 in the heavy chain region are known to be linked by a hinge portion.

The variable regions (VH and VL) are regions that comprise an antigen-binding portion, and even among the variable regions, there is a portion with the greatest variability (hypervariable region), and the corresponding part is called a complementarity-determining region (CDR). The VH comprises three CDRs, and the three CDRs included in the VH are generally referred to as CDRH1, CDRH2, or CDRH3, respectively. The CDRs in the VH may be understood to be located in the order of CDRH1, CDRH2, and CDRH3 in the direction from the N-terminus to the C-terminus. The VL comprises three CDRs, and the three CDRs included in the VL are generally referred to as CDRL1, CDRL2, or CDRL3, respectively. The CDRs in the VH may be understood to be located in the order of CDRL1, CDRL2, and CDRL3 in the direction from the N-terminus to the C-terminus.

The constant region of an antibody is a region separate from the antigen-binding portion, and it is known that the constant region may interact with cells or molecules of the immune system. For example, the constant region may interact with (may bind to or may be linked to) the cell membrane of immune cells (for example, lymphocytes, neutrophils, dendritic cells, and/or macrophages, and the like). Specifically, the hinge region and/or CH2 portion of the constant region may bind to receptors (FcεRIII, and the like) on the cell membrane of the immune cells. As another embodiment, the constant region may bind to FcRn.

The constant region of the heavy chain region mentioned above (hereinafter referred to as "heavy chain constant region") is roughly divided into five types (classes or isotypes): alpha (α), gamma (γ), delta (δ), epsilon (ε) and mu (µ). In this case, the types of heavy chain constant regions mentioned above are not determined individually for CH1, CH2, CH3, and CH4, but are determined in consideration of all heavy chain constant regions (CH1, CH2, and CH3; or CH1, CH2, CH3, and CH4) included in the antibody.

There are two types of constant regions of the light chain region (hereinafter referred to as "light chain constant regions"), and the two types are lambda (λ) and kappa (κ).

It is known that antibody types may be roughly divided into five types (classes or isotypes). The five types are determined by the type of heavy chain constant region.

The five types of antibodies described above are immunoglobulin M (IgM), immunoglobulin D (IgD), immunoglobulin G (IgG), immunoglobulin A (IgA), immunoglobulin A (IgA), and immunoglobulin E (IgE). When the type of heavy chain constant region of an antibody is classified as alpha, the type of antibody may be recognized as IgA. When the type of heavy chain constant region of an antibody is classified as gamma, the type of antibody may be recognized as IgG. When the type of heavy chain constant region of an antibody is classified as delta, the type of antibody may be recognized as IgD. When the type of heavy chain constant region of an antibody is classified as epsilon, the type of antibody may be recognized as IgE. When the type of heavy chain constant region of an antibody is classified as mu, the type of antibody may be recognized as IgM. For example, each heavy chain of IgG is known to comprise four immunoglobulin domains (VH, CH1, CH2, and CH3).

It is known that among the five antibody types, IgG and IgA may be classified into more detailed subclasses. For example, when the case where the antibody is a human antibody is described, when the type of heavy chain constant region of the antibody is gamma 1 (γ1), the type of antibody is IgG1; when the type of heavy chain constant region of the antibody is gamma 2 (γ2), the type of antibody is IgG2; when the type of heavy chain constant region of the antibody is gamma 3 (γ3), the type of antibody is IgG3; and when the type of heavy chain constant region of the antibody is gamma 4 (γ4), the type of antibody is IgG4. When the heavy chain constant region of the human antibody is alpha 1 (α1), the type of antibody is IgA1; and when the heavy chain constant region of the human antibody is alpha 2 (α2), the type of antibody is IgA2.

### Antibody-Drug Conjugate of the Present Disclosure

### Overview of the antibody-drug conjugate

According to one aspect of the present disclosure, an antibody-drug conjugate is disclosed.

The antibody-drug conjugate means that an antibody and a drug are linked. At this time, the antibody is an anti-claudin18.2 antibody, and the drug is monomethyl auristatin E (MMAE). At this time, the antibody-drug conjugate has a structure in which the drug is linked to one or more of lysine residue 246 (K246) and lysine residue 248 (K248) located in the Fc region of the antibody.

In some embodiments, the antibody-drug conjugate has the structure of Formula 1:

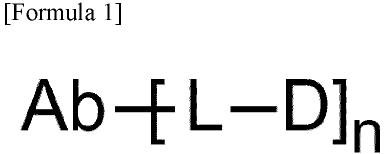

In Formula 1, Ab is an antibody unit.

In Formula 1, L is a linker unit.

In Formula 1, D is a drug unit.

In Formula 1, n is an integer of 1 to 4.

At this time, in Formula 1, the drug unit may be linked to one or more of lysine residue 246 (K246) and lysine residue 248 (K248) of the Fc region of the antibody unit. Specifically, in Formula 1, the linker unit may be covalently linked to an N atom derived from a lysine amino group (ε-amino group) of the antibody.

Hereinafter, each element of the compound of Formula 1 will be described in detail.

### Antibody Unit

The antibody unit is derived from an anti-claudin18.2 antibody (anti-CLDN18.2 antibody) and may be referred to as a conjugated anti-CLDN18.2 antibody. Furthermore, since the structure of the antibody unit is identical to the anti-CLDN18.2 antibody from which it is derived, except for the conjugated parts, and the antibody unit may be referred to as an anti-CLDN18.2 antibody. In this case, the conjugated part may be either lysine residue 246 (K246) or lysine residue 248 (K248) of the heavy chain of the anti-CLDN18.2 antibody.

In some embodiments, the anti-CLDN18.2 antibody may include an Fc region of IgG. In some embodiments, the Fc region of the anti-CLDN18.2 antibody may be an Fc region of IgG.

In some embodiments, the anti-CLDN18.2 antibody may be an IgG antibody. IgG antibodies encompass human IgG antibodies, humanized IgG antibodies, and chimeric IgG antibodies.

IgG may be selected from any one of the subclasses of IgG1, IgG2, IgG3, and IgG4.

In some embodiments, the anti-CLDN18.2 antibody may be an IgG1 antibody. IgG1 antibodies encompass human IgG1 antibodies, humanized IgG1 antibodies, and chimeric IgG1 antibodies.

In some embodiments, the anti-CLDN18.2 antibody may include an Fc region of IgG1. The Fc region of the anti-CLDN18.2 antibody may be an Fc region of IgG1.

In some embodiments, the anti-CLDN18.2 antibody may be an IgG2 antibody. IgG2 antibodies encompass human IgG2 antibodies, humanized IgG2 antibodies, and chimeric IgG2 antibodies.

In some embodiments, the anti-CLDN18.2 antibody may include an Fc region of IgG2. The Fc region of the anti-CLDN18.2 antibody may be an Fc region of IgG2.

In some embodiments, the anti-CLDN18.2 antibody may be an IgG3 antibody. IgG3 antibodies encompass human IgG3 antibodies, humanized IgG3 antibodies, and chimeric IgG3 antibodies.

In some embodiments, the anti-CLDN18.2 antibody may include an Fc region of IgG3. The Fc region of the anti-CLDN18.2 antibody may be an Fc region of IgG3.

In some embodiments, the anti-CLDN18.2 antibody may be an IgG4 antibody. IgG4 antibodies encompass human IgG4 antibodies, humanized IgG4 antibodies, and chimeric IgG4 antibodies.

In some embodiments, the anti-CLDN18.2 antibody may include an Fc region of IgG4. The Fc region of the anti-CLDN18.2 antibody may be an Fc region of IgG4.

In some embodiments, the anti-CLDN18.2 antibody may have an amino acid sequence selected from SEQ ID NO: 1 to SEQ ID NO: 5. Alternatively, the anti-CLDN18.2 antibody may have an amino acid sequence having 80%, 81%, 82%, 83%, 84%, 85%, 86%, 87%, 88%, 89%, 90%, 91%, 92%, 93%, 94%, 95%, 96%, 97%, 98%, or 99% or more sequence identity to the selected amino acid sequence. In a specific embodiment, the anti-CLDN18.2 antibody may have an amino acid sequence of SEQ ID NO: 1 or an amino acid sequence having 80%, 81%, 82%, 83%, 84%, 85%, 86%, 87%, 88%, 89%, 90%, 91%, 92%, 93%, 94%, 95%, 96%, 97%, 98%, or 99% or more sequence identity thereto.

In some embodiments, the anti-CLDN18.2 antibody includes an IgG Fc region, and at this time, the IgG Fc region may have an amino acid sequence selected from SEQ ID NO: 1 to SEQ ID NO: 5. The IgG Fc region may have an amino acid sequence having 80%, 81%, 82%, 83%, 84%, 85%, 86%, 87%, 88%, 89%, 90%, 91%, 92%, 93%, 94%, 95%, 96%, 97%, 98%, or 99% or more sequence identity to the selected amino acid sequence. In a specific embodiment, the anti-CLDN18.2 antibody may include an Fc region of an IgG, and at this time, the Fc region of the IgG may have an amino acid sequence of SEQ ID NO: 1 or an amino acid sequence having 80%, 81%, 82%, 83%, 84%, 85%, 86%, 87%, 88%, 89%, 90%, 91%, 92%, 93%, 94%, 95%, 96%, 97%, 98%, or 99% or more sequence identity thereto.

In some embodiments, the anti-CLDN18.2 antibody or the Fc region thereof may include the amino acid sequence of KPKDTLM (SEQ ID NO: 6) and the amino acid sequence of MHEALHNH (SEQ ID NO: 7).

In some embodiments, the anti-CLDN18.2 antibody or the Fc region thereof may include the amino acid sequence of KPKDTLM (SEQ ID NO: 6) and the amino acid sequence of MHEALHNHY (SEQ ID NO: 8).

In some embodiments, the anti-CLDN18.2 antibody or the Fc region thereof may include the amino acid sequence of GPSVFLFPPKPKDTLM (SEQ ID NO: 9).

In some embodiments, the anti-CLDN18.2 antibody may have an amino acid sequence selected from SEQ ID NOs: 1 to 5 or an amino acid sequence having 80%, 85%, 90%, 95%, 96%, 97%, 98%, or 99% or more sequence identity thereto, and may essentially include the amino acid sequence of KPKDTLM (SEQ ID NO: 6) and the amino acid sequence of MHEALHNH (SEQ ID NO: 7). In some embodiments, the anti-CLDN18.2 antibody may include an IgG Fc region, and at this time, the IgG Fc region may have an amino acid sequence selected from SEQ ID NOs: 1 to 5 or an amino acid sequence having 80%, 85%, 90%, 95%, 96%, 97%, 98%, or 99% or more sequence identity thereto, and may essentially include the amino acid sequence of KPKDTLM (SEQ ID NO: 6) and the amino acid sequence of MHEALHNH (SEQ ID NO: 7).

In some embodiments, the anti-CLDN18.2 antibody may have an amino acid sequence selected from SEQ ID NOs: 1 to 4 or an amino acid sequence having 80%, 85%, 90%, 95%, 96%, 97%, 98%, or 99% or more sequence identity thereto, and may essentially include the amino acid sequence of KPKDTLM (SEQ ID NO: 6) and the amino acid sequence of MHEALHNHY (SEQ ID NO: 8). In some embodiments, the anti-CLDN18.2 antibody may include an IgG Fc region (e.g., the Fc region of the antibody may be an Fc region of IgG), an at this time, the IgG Fc region may have an amino acid sequence selected from SEQ ID NOs: 1 to 4 or an amino acid sequence having 80%, 85%, 90%, 95%, 96%, 97%, 98%, or 99% or more sequence identity thereto, and at this time, the amino acid sequence may essentially include the amino acid sequence of KPKDTLM (SEQ ID NO: 6) and the amino acid sequence of MHEALHNHY (SEQ ID NO: 8).

In some embodiments, the anti-CLDN18.2 antibody may have an amino acid sequence selected from SEQ ID NOs: 1 to 5 or an amino acid sequence having 80%, 85%, 90%, 95%, 96%, 97%, 98%, or 99% or more sequence identity thereto, and may essentially include the amino acid sequence of GPSVFLFPPKPKDTLM (SEQ ID NO: 9). In some embodiments, the anti-CLDN18.2 antibody may include an IgG Fc region, an at this time, the IgG Fc region may have an amino acid sequence selected from SEQ ID NOs: 1 to 5 or an amino acid sequence having 80%, 85%, 90%, 95%, 96%, 97%, 98%, or 99% or more sequence identity thereto, and at this time, the amino acid sequence may essentially include the amino acid sequence of GPSVFLFPPKPKDTLM (SEQ ID NO: 9).

In some embodiments, the antibody having binding affinity to the Fc binding substance or peptide of the present application may be an antibody of the IgG isotype. In some embodiments, the antibody having binding affinity to the Fc binding substance or peptide of the present application may be an antibody of the IgG1 isotype, an antibody of the IgG2 isotype, an antibody of the IgG3 isotype, or an antibody of the IgG4 isotype. In some embodiments, the antibody with binding affinity to the Fc binding substance or peptide of the present application may be an antibody of the IgG1 isotype, an antibody of the IgG2 isotype, or an antibody of the IgG4 isotype.

In some embodiments, the anti-CLDN18.2 antibody may be a known anti-CLDN18.2 antibody (see document [Korean Patent Application No. 10-2021-7023724 (Publication No. 10-2021-0110339)]).

In some embodiments, the heavy chain of the anti-CLDN18.2 antibody may include CDRH1 having the amino acid sequence of SEQ ID NO: 10 (TYGVH) or an amino acid sequence having 90% or more sequence identity thereto, CDRH2 having the amino acid sequence of SEQ ID NO: 11 (VIWAGGSTNYNSALMS) or an amino acid sequence having 90% or more sequence identity thereto, and CDRH3 having the amino acid sequence of SEQ ID NO: 12 (AAYYGNGLDY) or an amino acid sequence having 90% or more sequence identity thereto. In a specific embodiment, the anti-CLDN18.2 antibody may have two heavy chains including CDRH1 having the amino acid sequence of SEQ ID NO: 10, CDRH2 having the amino acid sequence of SEQ ID NO: 11, and CDRH3 having the amino acid sequence of SEQ ID NO: 12.

In some embodiments, the light chain of the anti-CLDN18.2 antibody may include CDRL1 having the amino acid sequence of SEQ ID NO: 13 (KSSQTLLNSGNQKNYLT) or an amino acid sequence having 90% or more sequence identity thereto, CDRL2 having the amino acid sequence of SEQ ID NO: 14 (WASTGES) or an amino acid sequence having 90% or more sequence identity thereto, and CDRL3 having the amino acid sequence of SEQ ID NO: 15 (QNAYFYPFT) or an amino acid sequence having 90% or more sequence identity thereto. In a specific embodiment, the anti-CLDN18.2 antibody may have two light chains including CDRL1 having the amino acid sequence of SEQ ID NO: 13, CDRL2 having the amino acid sequence of SEQ ID NO: 14, and CDRL3 having the amino acid sequence of SEQ ID NO: 15.

In some embodiments, the heavy chain of the anti-CLDN18.2 antibody may include CDRH1 having an amino acid sequence of SEQ ID NO: 10 or an amino acid sequence having 90% or more sequence identity thereto, CDRH2 having an amino acid sequence of SEQ ID NO: 11 or an amino acid sequence having 90% or more sequence identity thereto, and CDRH3 having an amino acid sequence of SEQ ID NO: 12 or an amino acid sequence having 90% or more sequence identity thereto, and the light chain of the anti-CLDN18.2 antibody may include CDRL1 having an amino acid sequence of SEQ ID NO: 13 or an amino acid sequence having 90% or more sequence identity thereto, CDRL2 having an amino acid sequence of SEQ ID NO: 14 or an amino acid sequence having 90% or more sequence identity thereto, and CDRL3 having an amino acid sequence of SEQ ID NO: 15 or an amino acid sequence having 90% or more sequence identity thereto. In a specific embodiment, the anti-CLDN18.2 antibody may have two heavy chains including CDRH1 having the amino acid sequence of SEQ ID NO: 10, CDRH2 having the amino acid sequence of SEQ ID NO: 11, and CDRH3 having the amino acid sequence of SEQ ID NO: 12, and two light chains including CDRL1 having the amino acid sequence of SEQ ID NO: 13, CDRL2 having the amino acid sequence of SEQ ID NO: 14, and CDRL3 having the amino acid sequence of SEQ ID NO: 15.

In some embodiments, the anti-CLDN18.2 antibody may be an antibody including a heavy chain having an amino acid sequence of SEQ ID NO: 16 or an amino acid sequence having 90% or more sequence identity thereto, and a light chain having an amino acid sequence of SEQ ID NO: 17 or an amino acid sequence having 90% or more sequence identity thereto. In a specific embodiment, the anti-CLDN18.2 antibody may have two heavy chains having the amino acid sequence of SEQ ID NO: 16 and two light chains having the amino acid sequence of SEQ ID NO: 17.

### Linker Unit

The linker unit is a structure of a region that connects an anti-claudin18.2 antibody (anti-CLDN18.2 antibody) and a drug.

### Overview of linker unit

L (linker unit) of Formula 1 may have a structure of Formula 2:

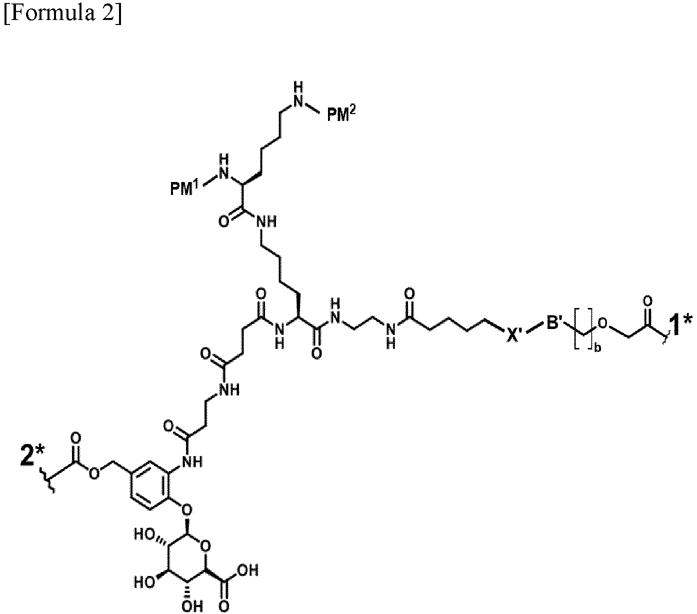

At this time,
in Formula 2, b is an integer of 0 to 6. In some embodiments, b may be 0, 1, 2, 3, 4, 5, or 6, or an integer between two of these numbers. For example, b may be an integer of 1 to 3. In a specific embodiment, b may be 2.

In Formula 2, X' is -NH-, -C(O)-, or -NHC(O)-.

In Formula 2, B' is a group formed by a click-chemistry reaction between click-chemistry functional groups.

In Formula 2, PM¹ and PM² are each independent PEG moieties.

In Formula 2, 1* represents an attachment site with Ab.

In Formula 2, 2* represents an attachment site with D.

### B' of Formula 2

B' of Formula 2 may include any one structure selected from:

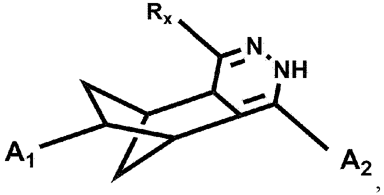

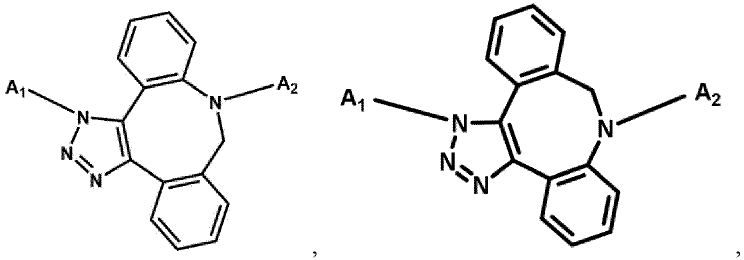

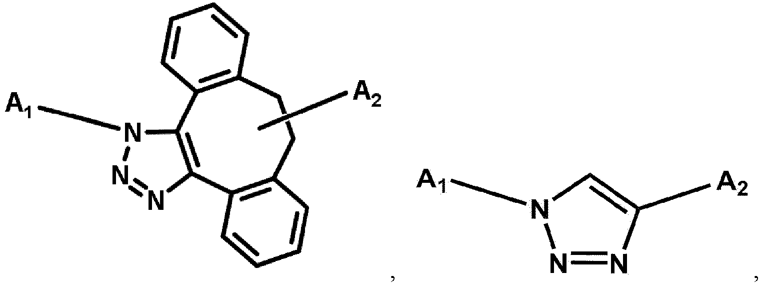

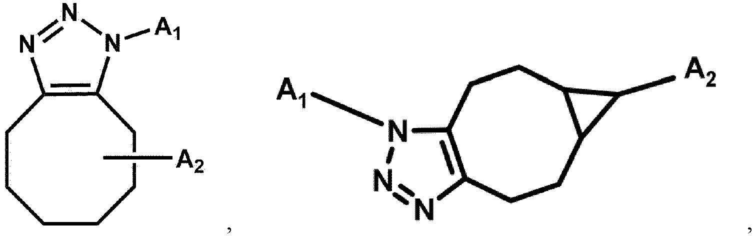

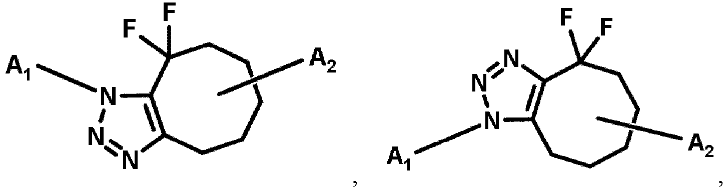

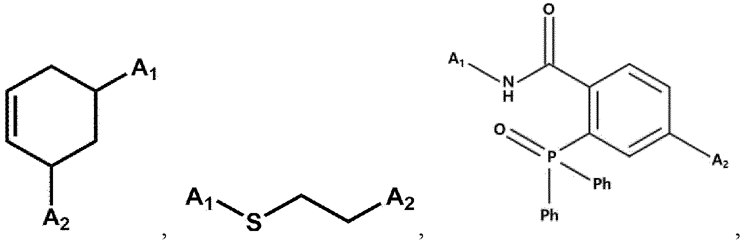

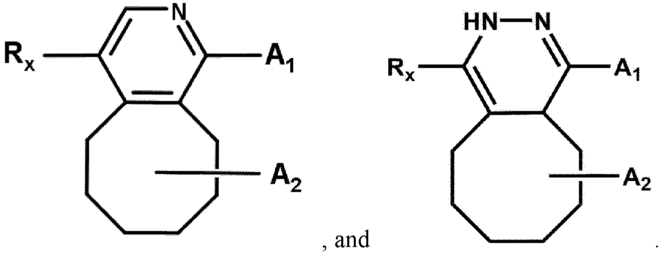

At this time,

Rₓ may be selected from H, a halogen, and a C₁₋₃ alkyl,

A₁ and A₂ each represent an attachment site to the remaining structure of the linker unit. In a specific embodiment, A₁ may represent an attachment site to X'. In another specific embodiment, A₂ may represent an attachment site to X'.

### PM¹ and PM² of Formula 2

In Formula 2, PM¹ and PM² each independently represent a PEG moiety.

At this time, the PEG moiety may include 1 to 30, 1 to 20, or 1 to 10 ethylene glycol units (e.g., -CH₂OCH₂-, -OCH₂CH₂-, or -CH₂CH₂O-).

For example, the PEG moiety may have a structure of Formula 3.

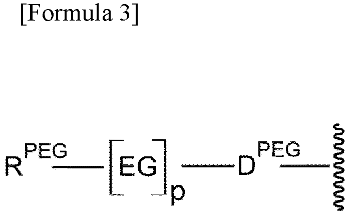

In Formula 3, D^{PEG} is a spacer of the PEG moiety.

For example, D^{PEG} may be a group whose main chain length is 0 to 6 (i.e., a group whose sum of the number of atoms located in the main chain is 0 to 6).

For example, D^{PEG} may be a bond, or a substituted or unsubstituted C₁₋₆ alkylene, a substituted or unsubstituted C₁₋₆ heteroalkylene, a substituted or unsubstituted C₁₋₆ alkenylene, a substituted or unsubstituted C₁₋₆ heteroalkenylene, a substituted or unsubstituted alkynylene, or a substituted or unsubstituted heteroalkynylene, wherein the substituted alkylene, substituted heteroalkylene, substituted alkenylene, or substituted heteroalkynylene may include one or more substituents, and may be selected from -R, =O, =S, -NO₂, -CR₃, -NR₂, -OR, -SR, -C(=O)R, -C(=O)CR₃, -C(=O)OR, and -C(=O)NR₂, wherein R is each independently H, a halogen, a C₁₋₆ alkyl, a C₃₋₁₀ cycloalkyl, a C₃₋₁₀ heterocycloalkyl, an aryl, a heteroaryl, -OH, -NH₂, =O, =S, and -SH. In a specific embodiment, the substituent may be selected from an -C₁₋₄ alkyl, , -C(=O)H, -C(=O)CH₃, -C(=O)OH, -C(=O)NH₂, -NH₂, =O, =S, -OH, -NO₂, and -SH. Here, heteroalkylene, heteroalkenylene, and heteroalkynylene each independently include one or more heteroatoms, wherein each heteroatom may be independently selected from N, O, and S.

In a specific embodiment, D^{PEG} may be a bond, or a substituted or unsubstituted C₁₋₃ alkylene, or a substituted or unsubstituted C₁₋₃ heteroalkylene, wherein the substituted alkylene or substituted heteroalkylene may include one or more substituents, wherein the substituents are =O, and the heteroalkylene may include one or more heteroatoms, wherein each heteroatom may be independently selected from N, O, and S.

In Formula 3, R^{PEG} is a PEG capping group. At this time, the PEG capping group may be absent or may be -CH₃, C₂ alkyl, C₃ alkyl, -NH₂, -CH₂NH₂, - SC(=O)CH₃, -SC(=O)CH₂CH₃, -CH₂SC(=O)CH₃, -CH₂SC(=O)CH₂CH₃, -OH, -CH₂OH, - SH, -CH₂SH, -OCH₃, -CH₂OCH₃, -CH₂OCH₂CH₃, -C(=O)CH₃, -C(=O)CH₂CH₃, - CH₂C(=O)CH₃, -CH₂C(=O)CH₂CH₃, -NHC(=O)CH₃, -NHC(=O)CH₂CH₃, - CH₂NHC(=O)CH₃, -CH₂CH₂NHC(=O)CH₃, -CH₂CH₂NHC(=O)CH₂CH₃, - CH₂NHC(=O)CH₂CH₃, -CH₂CH₂COOH, glucose, or -O-glucose, but is not limited thereto. In a specific embodiment, the PEG capping group may be absent or may be - -CH₃, -OCH₃, -CH₂OCH₃, -C(=O)CH₃, -CH₂C(=O)CH₃, -NHC(=O)CH₃, -CH₂NHC(=O)CH₃, or - CH₂CH₂COOH. In a specific embodiment, the sum of the atomic masses of the atoms belonging to the PEG capping group may be 300 daltons or less, 200 daltons or less, 150 daltons or less, 100 daltons or less, or 50 daltons or less, but is not limited thereto.

In Formula 3, p may be an integer of 1 to 30, preferably 1 to 10.

In Formula 3, [EG] is an ethylene glycol unit. At this time, the ethylene glycol unit is -[CH₂OCH₂]-, -[OCH₂CH₂]-, or -[CH₂CH₂O]-.

In a specific embodiment, in Formula 3, D^{PEG} is -C(=O)-, [EG] is - [OCH₂CH₂]-, p is 8, R^{PEG} is -CH₃.

In a specific embodiment, in Formula 2, each of PM¹ and PM² is:

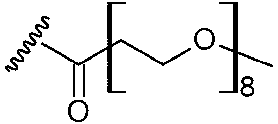

### Specific embodiments of linker unit

In a specific embodiment, the linker unit may have the structure of Formula 4:

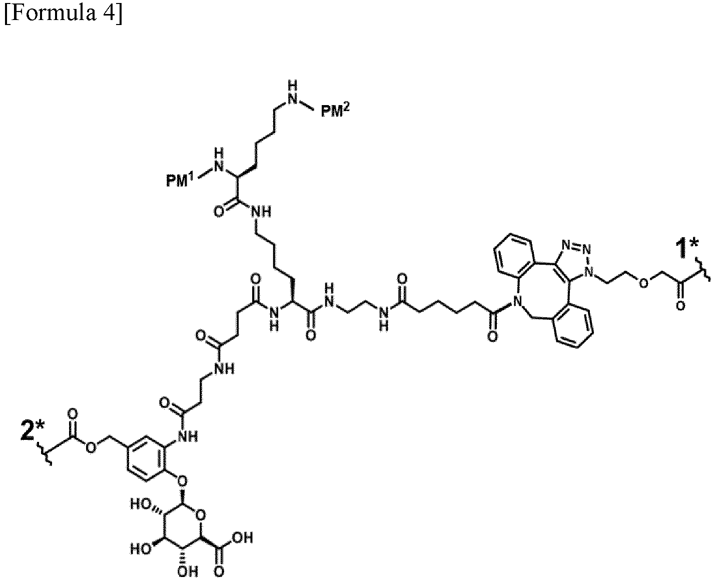

In Formula 4, PM¹ and PM² are each independently a PEG moiety, and the description thereof is as described for PM¹ and PM² in the above-described << PM¹ and PM² of Formula 2>> section.

In Formula 4, 1* represents an attachment site with Ab,

In Formula 4, 2* represents an attachment site with D.

### Drug Unit

The drug unit refers to a drug conjugated to an antibody or a structure derived from the drug. The drug may be monomethyl auristatin E (MMAE).

In a specific embodiment, the drug unit may have a structure of Formula 5:

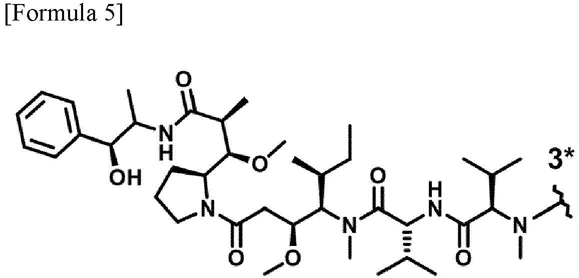

In Formula 5, 3* represents an attachment site to L of Formula 1.

### Position at Which the Drug Unit is Linked

In Formula 1, n is an integer of 1 to 4. In some embodiments, n may be an integer of 1 to 2. In a specific embodiment, n may be 2.

The drug unit may be linked (via L or a linker unit) to any one or more of lysine residue 246 (K246) and lysine residue 248 (K248) of the Fc region of the antibody unit. More specifically, the antibody unit may include two heavy chains (a first heavy chain and a second heavy chain), and at this time, "-L-" may be linked to any one or more of K246 of the first heavy chain, K248 of the first heavy chain, K246 of the second heavy chain, and K248 of the second heavy chain.

For example, when n is 1,
one drug unit may be linked (via L or a linker unit) to K246 of the first heavy chain of the antibody unit. Alternatively, one drug unit may be linked to K248 of the first heavy chain of the antibody unit.

For example, when n is 2,
one drug unit of the two drug units may be linked to K246 of the first heavy chain of the antibody unit, and the other drug unit may be linked to K246 of the second heavy chain. Alternatively, one drug unit of the two drug units may be linked to K248 of the first heavy chain of the antibody unit, and the other drug unit may be linked to K248 of the second heavy chain. Alternatively, one drug unit of the two drug units may be linked to K246 of the first heavy chain of the antibody unit, and the other drug unit may be linked to K248 of the second heavy chain. Alternatively, one drug unit of the two drug units may be linked to K246 of the first heavy chain of the antibody unit, and the other drug unit may be linked to K248 of the first heavy chain.

For example, when n is 3,
the three drug units may be independently linked to K246 of the first heavy chain, K248 of the first heavy chain, and K246 of the second heavy chain, respectively.

Alternatively, the drug units of the generation may be independently linked to K246 of the first heavy chain, K248 of the first heavy chain, and K248 of the second heavy chain, respectively.

More specifically, when n is 3, the antibody-drug conjugate includes three drug units (a first drug unit, a second drug unit, and a third drug unit). At this time, the first drug unit may be linked to K246 of the first heavy chain, the second drug unit may be linked to K248 of the first heavy chain, and the third drug unit may be linked to K246 of the second heavy chain. Alternatively, the first drug unit may be linked to K246 of the first heavy chain, the second drug unit may be linked to K248 of the first heavy chain, and the third drug unit may be linked to K248 of the second heavy chain.

For example, when n is 4,
the four drug units may be independently linked to K246 of the first heavy chain, K248 of the first heavy chain, K246 of the second heavy chain, and K248 of the second heavy chain of the antibody unit, respectively.

More specifically, when n is 4, the antibody-drug conjugate includes four drug units (a first drug unit, a second drug unit, a third drug unit, and a fourth drug unit). At this time, the first drug unit may be linked to K246 of the first heavy chain, the second drug unit may be linked to K248 of the first heavy chain, the third drug unit may be linked to K246 of the second heavy chain, and the fourth drug unit may be linked to K248 of the second heavy chain.

### Specific embodiments of antibody-drug conjugate

In a specific embodiment, Formula 1 may be represented by Formula 6. In other words, the antibody-drug conjugate may have the structure of Formula 6 below:

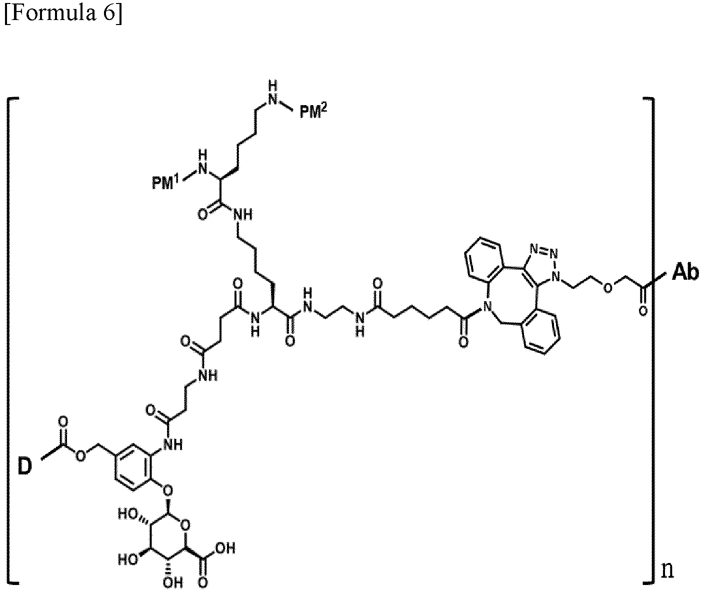

In Formula 6, Ab is an antibody unit, the description of which is as described about the drug unit in the above-described <<Antibody Unit>> section.

In Formula 6, PM¹ and PM² are each independently a PEG moiety, the description of which is as described about PM¹ and PM² in the above-described << PM¹ and PM² of Formula 2>> section.

In Formula 6, D is a drug unit, the description of which is as described for the drug unit in the above-described <<Drug Unit>> section.

In Formula 6, n is an integer of 1 to 4.

In Formula 6, the description of the position at which the drug unit is linked to the antibody unit is as described in the above-described <<Position at Which the Drug Unit is Linked>> section.

In a specific embodiment, Formula 1 may be represented by Formula 7. In other words, the antibody-drug conjugate may have the structure of Formula 7 below:

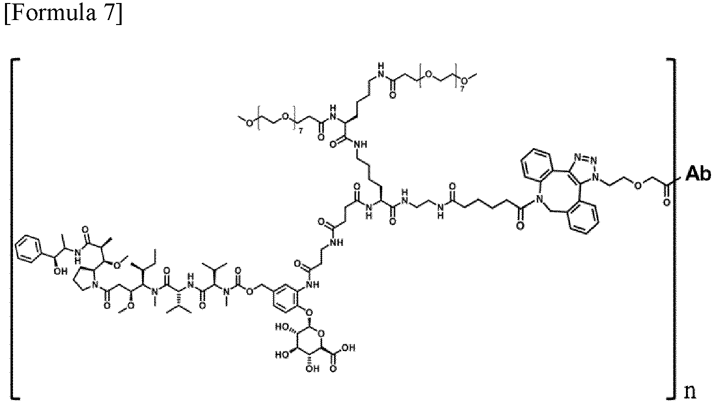

In Formula 7, Ab is an antibody unit, the description of which is as described about the drug unit in the above-described <<Antibody Unit>> section.

In Formula 7, n is an integer of 1 to 4.

In Formula 7, the description of the bonded position of the antibody unit is as described in the above-described <<Position at Which the Drug Unit is Linked>> section.

### Characteristics of the Antibody-Drug Conjugate of the Present Disclosure

### Binding of the antibody-drug conjugate of the present disclosure to an antigen

The antibody-drug conjugate disclosed by the present disclosure may bind to an antigen (claudin18.2 protein). Specifically, the antibody unit included in the antibody-drug conjugate may bind to an antigen (claudin18.2 protein).

In some embodiments, the antibody-drug conjugate may bind to an antigen (claudin18.2 protein) expressed on the surface (or membrane) of a cell. Specifically, the antibody unit included in the antibody-drug conjugate may bind to an antigen (claudin18.2 protein) expressed on the surface (or membrane) of a cell.

In some embodiments, the antibody-drug conjugate may bind to a claudin18.2 positive cell (or a cell expressing claudin18.2). Specifically, the antibody unit included in the antibody-drug conjugate may bind to a claudin18.2 positive cell (or a cell expressing claudin18.2).

In some embodiments, the binding affinity of the antibody-drug conjugate disclosed by the present disclosure to an antigen (claudin18.2 protein) is the same and/or similar to the binding affinity of the antibody (anti-claudin18.2 antibody) prior to conjugation to the antigen (claudin18.2 protein).

### Internalization of the antibody-drug conjugate of the present disclosure into a cell

The antibody-drug conjugate disclosed by the present disclosure may bind to a claudin18.2 protein expressed in a cell and then enter the cell (or be internalized into the cell). At this time, a part corresponding to the beta-glucuronide linker of the antibody-drug conjugate may be cleaved by beta-glucuronidase within the cell. At this time, the beta-glucuronidase may be produced in the lysosome of the cell.

Therefore, the drug unit separated from the antibody-drug conjugate may be released into the cell through the above-described cleavage process.

The structure of the beta-glucuronide linker included in the antibody-drug conjugate is as shown in Formula 8.

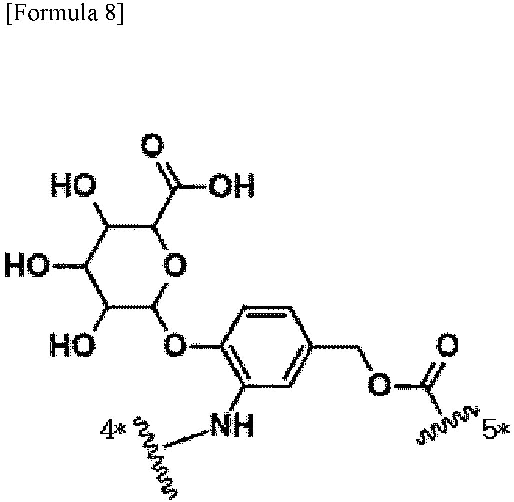

In Formula 8, 4* represents an attachment site to a part of the linker unit.

In Formula 8, 5* represents an attachment site to the drug unit.

### Anticancer effect of antibody-drug conjugate of the present disclosure

The antibody-drug conjugate disclosed by the present disclosure has an anticancer effect. At this time, the anticancer effect refers to inhibiting the occurrence, growth, and/or metastasis of cancer cells. The anticancer effect is exerted through the synergy between the function of the drug released after the antibody-drug conjugate enters the cancer cell and/or the function of the antibody itself.

In some embodiments, the antibody-drug conjugate may attack cancer cells.

In some embodiments, the antibody-drug conjugate may kill cancer cells.

### Half-life of antibody-drug conjugate of the present disclosure

The *in vivo* half-life of the antibody-drug conjugate disclosed by the present disclosure may range from 3 to 7 days. In some embodiments, the *in vivo* half-life of the antibody-drug conjugate ranges from 4 to 6 days. In a specific embodiment, the *in vivo* half-life of the antibody-drug conjugate ranges from 4.5 days to 5.2 days.

In some embodiments, the *in vivo* half-life of the antibody-drug conjugate is the same as and/or similar to the *in vivo* half-life of the antibody (anti-claudin18.2 antibody) prior to conjugation.

### Use of the Antibody-Drug Conjugate of the Present Disclosure

### Overview of the use of the antibody-drug conjugate of the present disclosure

The antibody-drug conjugate of the present disclosure has uses in treating cancer.

The present disclosure discloses a pharmaceutical composition for treating cancer, including the above-described antibody-drug conjugate.

The present disclosure discloses a method of treating cancer using the above-described antibody-drug conjugate.

Various embodiments of the antibody-drug conjugate used in cancer treatment are described below.

### Target Diseases

The target disease for which the antibody-drug conjugate of the present disclosure may be used for therapeutic purposes is a cancer, a tumor, a disease related to the cancer, or a disease related to the tumor.

In some embodiments, the cancer or tumor may be any one selected from bladder cancer, bone cancer, brain cancer, breast cancer, heart cancer, cervical cancer, colon cancer, rectal cancer, esophageal cancer, fibrosarcoma, stomach cancer, gastrointestinal cancer, biliary tract cancer, head and neck cancer, Kaposi's sarcoma, kidney cancer, leukemia, liver cancer, lung cancer, lymphoma, melanoma, myeloma, ovarian cancer, pancreatic cancer, penile cancer, prostate cancer, urogenital cancer, testicular germ cell cancer, thymoma, and thymic cancer. In a specific embodiment, the cancer or tumor may be any one selected from stomach cancer, pancreatic cancer, esophageal cancer, ovarian cancer, lung cancer, liver cancer, bladder cancer, colon cancer, bile duct cancer, and urogenital cancer.

In some embodiments, the cancer or tumor is a claudin18.2 positive cancer or tumor. At this time, the cancer or tumor may be one expressing the claudin18.2 protein, and specifically, the cancer cell or tumor cell may be one expressing the claudin18.2 protein.

In some embodiments, the disease to be treated by the pharmaceutical composition may be gastric cancer. In a specific embodiment, the disease to be treated by the pharmaceutical composition is claudin18.2 positive gastric cancer. At this time, the claudin18.2 positive gastric cancer may refer to gastric cancer caused by cancer cells expressing the claudin18.2 protein.

In some embodiments, the disease to be treated by the pharmaceutical composition may be pancreatic cancer. In a specific embodiment, the disease to be treated by the pharmaceutical composition is claudin18.2 positive pancreatic cancer. At this time, the claudin18.2 positive pancreatic cancer may refer to pancreatic cancer caused by cancer cells expressing the claudin18.2 protein.

In some embodiments, the disease to be treated by the pharmaceutical composition may be esophageal cancer. In a specific embodiment, the disease to be treated by the pharmaceutical composition is claudin18.2 positive esophageal cancer. At this time, the claudin18.2 positive esophageal cancer may refer to esophageal cancer caused by cancer cells expressing the claudin18.2 protein.

In some embodiments, the disease to be treated by the pharmaceutical composition may be ovarian cancer. In a specific embodiment, the disease to be treated by the pharmaceutical composition is claudin18.2 positive ovarian cancer. At this time, the claudin18.2 positive ovarian cancer may refer to ovarian cancer caused by cancer cells expressing the claudin18.2 protein.

In some embodiments, the disease to be treated by the pharmaceutical composition may be lung cancer. In a specific embodiment, the disease to be treated by the pharmaceutical composition is claudin18.2 positive lung cancer. At this time, the claudin18.2 positive lung cancer may refer to lung cancer caused by cancer cells expressing the claudin18.2 protein.

In some embodiments, the disease to be treated by the pharmaceutical composition may be liver cancer. In a specific embodiment, the disease to be treated by the pharmaceutical composition is claudin18.2 positive liver cancer. At this time, the claudin18.2 positive liver cancer may refer to liver cancer caused by cancer cells expressing the claudin18.2 protein.

In some embodiments, the disease to be treated by the pharmaceutical composition may be bladder cancer. In a specific embodiment, the disease to be treated by the pharmaceutical composition is claudin18.2 positive bladder cancer. At this time, the claudin18.2 positive bladder cancer may refer to bladder cancer caused by cancer cells expressing the claudin18.2 protein.

In some embodiments, the disease to be treated by the pharmaceutical composition may be colon cancer. In a specific embodiment, the disease to be treated by the pharmaceutical composition is claudin18.2 positive colon cancer. At this time, the claudin18.2 positive colon cancer may refer to colon cancer caused by cancer cells expressing the claudin18.2 protein.

In some embodiments, the disease to be treated by the pharmaceutical composition may be bile duct cancer. In a specific embodiment, the disease to be treated by the pharmaceutical composition is claudin18.2 positive bile duct cancer. At this time, the claudin18.2 positive bile duct cancer may refer to bile duct cancer caused by cancer cells expressing the claudin18.2 protein.

In some embodiments, the disease to be treated by the pharmaceutical composition may be urogenital cancer. In a specific embodiment, the disease to be treated by the pharmaceutical composition is claudin18.2 positive urogenital cancer. At this time, the claudin18.2 positive urogenital cancer may refer to urogenital cancer caused by cancer cells expressing the claudin18.2 protein.

### Pharmaceutical Composition Including Antibody-Drug Conjugate

### Overview of pharmaceutical composition

According to one aspect of the present disclosure, a pharmaceutical composition including an antibody-drug conjugate for treating cancer, a tumor, a disease related to the cancer, or a disease related to the tumor is disclosed. At this time, the antibody-drug conjugate refers to the antibody-drug conjugate disclosed in the present disclosure. The pharmaceutical composition includes a therapeutically effective amount of the antibody-drug conjugate. The pharmaceutical composition may further include a pharmaceutically acceptable carrier and/or a pharmaceutically acceptable adjuvant. Here, the pharmaceutically acceptable carrier may be used for appropriate formulation of the pharmaceutical composition.

### Target disease of the pharmaceutical composition

The target disease of the pharmaceutical composition, which is cancer, a tumor, a disease related to the cancer, or a disease related to the tumor, is as described in the <<Target Diseases>> sub-section of the <<Use of the Antibody-Drug Conjugate of the Present Disclosure >> section.

### Examples of the formulation of the pharmaceutical composition

In some embodiments, the pharmaceutical composition may be formulated as troches, lozenges, tablets, aqueous suspensions, oily suspensions, prepared powder, granules, emulsions, hard capsules, soft capsules, syrup, or elixirs.

In some embodiments, the pharmaceutical composition may be formulated as injections, suppositories, powder for respiratory inhalation, aerosols for sprays, ointment, powder for application, oils, or creams.

In some embodiments, the pharmaceutical composition may be formulated as injections. Specifically, a therapeutically effective amount of an antibody-drug conjugate may be mixed with a stabilizer or buffer in water to prepare a solution or suspension, which may be formulated for unit dosage in ampoules or vials.

In some embodiments, the pharmaceutical composition may be formulated as an aerosol by mixing a propellant and the like with additives to prepare a water-dispersed concentrate or a wet powder.

In some embodiments, when the pharmaceutical composition is formulated for transdermal use, ointment, creams, powder for application, oils, skin external preparations, and the like may be prepared by adding a therapeutically effective amount of the antibody-drug conjugate to a carrier such as animal fat, vegetable fat, wax, paraffin, starch, tragacanth, cellulose derivatives, polyethylene glycol, silicone, bentonite, silica, talc, zinc oxide, or the like.

### Examples of pharmaceutically acceptable carriers

Pharmaceutically acceptable carriers may be those commonly used in formulations, and may include saline solutions, sterile water, Ringer's solution, buffered saline, cyclodextrin, dextrose solutions, maltodextrin solutions, glycerol, ethanol, liposomes, and the like, but are not limited thereto, and may further include other common additives such as antioxidants and buffers as needed. In addition, diluents, dispersants, surfactants, binders, lubricants, and the like may be additionally added to formulate into injectable formulations such as aqueous solutions, suspensions, and emulsions, pills, capsules, granules, or tablets. Regarding suitable pharmaceutically acceptable carriers and formulations, each ingredient may be preferably formulated using the methods disclosed in Remington's Pharmaceutical Sciences (19th edition, 1995).

In some embodiments, the pharmaceutical composition may include, as a pharmaceutically acceptable carrier, a binder such as lactose, saccharose, sorbitol, mannitol, starch, amylopectin, cellulose, or gelatin; an excipient such as dicalcium phosphate; a disintegrant such as corn starch or sweet potato starch; a lubricant such as magnesium stearate, calcium stearate, sodium stearyl fumarate, or polyethylene glycol wax; a sweetener; a flavoring agent; syrup; a liquid carrier such as a fatty oil; a sterile aqueous solution; propylene glycol; polyethylene glycol; an injectable ester such as ethyl oleate; a suspending agent; an emulsifier; a lyophilized preparation; an external preparation; a stabilizer; a buffering agent; an animal oil; a vegetable oil; wax; a paraffin; starch; tragacanth; a cellulose derivative; polyethylene glycol; silicone; bentonite; silica; talc; zinc oxide, or a suitable combination thereof.

In some embodiment, in order to prepare the pharmaceutical composition of the present disclosure, the antibody-drug conjugate may be mixed with a pharmaceutically acceptable carrier. In other words, the pharmaceutical composition may include a pharmaceutically acceptable carrier. Furthermore, the pharmaceutical composition may further contain one or more other elements suitable for the treatment or prevention of cancer. The term "pharmaceutically acceptable carrier" may be used to include an excipient, a diluent, or an adjuvant. The carrier may be one or more selected from, for example, lactose, dextrose, sucrose, sorbitol, mannitol, xylitol, erythritol, maltitol, starch, gum acacia, alginate, gelatin, calcium phosphate, calcium silicate, cellulose, methylcellulose, polyvinylpyrrolidone, water, physiological saline, a buffer such as phosphate-buffered saline (PBS), methylhydroxy benzoate, propylhydroxy benzoate, talc, magnesium stearate, and mineral oil. The carrier may include a filler, an anti-agglomerating agent, a lubricant, a wetting agent, a flavoring agent, an emulsifier, a preservative, or a combination thereof.

### Treatment Method Using Antibody-Drug Conjugate

### Overview of treatment method

According to one aspect of the present disclosure, a method for treating cancer, a tumor, a disease related to the cancer, or a disease related to the tumor using an antibody-drug conjugate is disclosed. At this time, the antibody-drug conjugate refers to the antibody-drug conjugate disclosed by the present disclosure. The treatment method includes administering an appropriate formulation of the therapeutically effective antibody-drug conjugate, that is, the pharmaceutical composition, to a subject using an appropriate administration method, an appropriate administration regimen, and an appropriate administration dosage.

### Target diseases of treatment method

The target disease of the treatment method, which is cancer, a tumor, a disease related to the cancer, or a disease related to the tumor, is as described in the sub-section <<Target Disease>> of the <<Use of the Antibody-Drug Conjugate of the Present Disclosure>> section.

### Treatment subject

The subject of the treatment method may be a human or a non-human animal.

### Examples of administration method

The treatment method may be to administer to a subject an appropriate formulation of the therapeutically effective antibody-drug conjugate by an appropriate administration method. In one embodiment, the treatment method may be to administer to the subject an appropriate formulation of the therapeutically effective antibody-drug conjugate by one method selected from oral administration, parenteral administration, intravenous administration, intraperitoneal administration, intramuscular administration, transdermal administration, and subcutaneous administration. In a specific embodiment, the treatment method may be to administer to a subject an appropriate formulation of the therapeutically effective antibody-drug conjugate by intravenous administration.

### Examples of dosage

The treatment method may be to administer to a subject an appropriate dosage of an appropriate formulation of the therapeutically effective antibody-drug conjugate. In one embodiment, the dosage may be about 0.01 mg to 1000 mg per kg of the subject's body weight, based on the antibody-drug conjugate. In a specific embodiment, the dosage may be about 0.01 mg/kg to 100 mg/kg of the subject's body weight, based on the antibody-drug conjugate.

### Examples of administration cycle

The treatment method may be to administer to a subject an appropriate formulation of the therapeutically effective antibody-drug conjugate at an appropriate administration cycle. In one embodiment, the pharmaceutical composition including the antibody-drug conjugate of an appropriate dosage may be administered once a day. In another example, the administration cycle may be to administer the pharmaceutical composition including the antibody-drug conjugate of an appropriate dosage twice or more times a day. In another example, the administration cycle may be to administer the pharmaceutical composition including the antibody-drug conjugate of an appropriate dosage at intervals of 1 hour, 2 hours, 6 hours, 12 hours, 24 hours, 2 days, 3 days, 1 week, 2 weeks, 1 month, 2 months, and/or 3 months.

### Therapeutic Uses of the Antibody-Drug Conjugate

According to one aspect of the present disclosure, the use of the antibody-drug conjugate for treating cancer, a tumor, a disease related to the cancer, or a disease related to the tumor is disclosed. At this time, the antibody-drug conjugate refers to the antibody-drug conjugate disclosed by the present disclosure. At this time, the cancer, tumor, disease related to the cancer, or disease related to the tumor is as described in the <<Target Diseases>> sub-section of the <<Use of the Antibody-Drug Conjugate of the Present Disclosure >> section.

**Use of Antibody-Drug Conjugate in the Manufacture of Cancer Therapeutics**

According to one aspect of the present disclosure, the use of the antibody-drug conjugate in the manufacture of a medication for the treating cancer, a tumor, a disease related to the cancer, or a disease related to the tumor is disclosed. At this time, the antibody-drug conjugate refers to the antibody-drug conjugate disclosed by the present disclosure. At this time, the cancer, tumor, disease related to the cancer, or disease related to the tumor is as described in the <<Target Diseases>> sub-section of the <<Use of the Antibody-Drug Conjugate of the Present Disclosure >> section.

### Method of Preparing Antibody-Drug Conjugate

The antibody-drug conjugate disclosed in the present disclosure may be prepared, for example, by the methods disclosed in Example 1 and Example 2.

Hereinafter, the invention provided by the present application is described in more detail through experimental examples and examples. These examples are intended to illustrate the content disclosed by the present application, and the scope of the content disclosed by the present application is not limited by these examples.

### Examples

### Materials and Experimental Methods

### Compounds

Compounds were purchased from commercial suppliers and used without further purification. 5-exo-norbornene carboxylic acid, ammonium sulfate ((NH₄)₂SO₄), *N*-Boc-ethylenediamine, triisopropylsilane (TIS), and 1,2-ethanedithiol (EDT) were purchased from Sigma-Aldrich CO., LTD. (St. Louis, MO, USA).

*N-*methylmaleimide, *N*-ethylmaleimide, *N*-cyclohexylmaleimide, *N-*phenylmaleimide, *N*-benzylmaleimide, *N-tert*-butylmaleimide, and 3-maleimidopropionic acid were purchased from Tokyo Chemical Industry (Tokyo, Japan). *Tert*-butyl thioglycolate was purchased from Angene Chemical PVT., LTD. (Telangana, India). 4-methyltetrazine NHS ester was purchased from Broadpharm Inc. (San Diego, CA, USA). Fmoc-PEG₈-OH was purchased from Quanta Biodesign (Plain City, OH, USA). DM1-SMCC was purchased from eNovation Chemicals LLC. (Bridgewater Township, NJ, USA). DBCO-C6-NHS was purchased from (Lumiprobe, Hong Kong). All amino acids, rink amide resin, and coupling reagents were purchased from AAPPtec LLC. (Louisville, KY, USA), GL Biochem LTD. (Shanghai, China), and Combi-Blocks Inc. (San Diego, CA, USA). Solvents were used without distillation. Trifluoroacetic acid (TFA), N,N-diisopropylethylamine (DIPEA), ammonium hydroxide (ammonia solution), diethylamine, Na₂HPO₄, N,N-dimethylformamide (DMF), dichloromethane (DCM), methanol (MeOH), hexane (Hex), ethyl acetate (EA), and diethyl ether were obtained from Daejung Chemicals & Metals CO., LTD. (Siheung, Korea). High-performance liquid chromatography (HPLC)-grade acetonitrile (ACN), isopropanol, and water were purchased from Thermo Fisher Scientific Inc. (Waltham, MA, USA).

### Antibody and ADC

Two types of anti-claudin18.2 antibody (anti-CLDN18.2 antibody) were prepared (hereinafter referred to as antibody-A and antibody-B).

Antibody-A is an antibody including a heavy chain having the amino acid sequence of SEQ ID NO: 16 and a light chain having the amino acid sequence of SEQ ID NO: 17 (herein, antibody-A has two light chains and two heavy chains, as in the structure of a known general antibody). Information on the antibody is disclosed in the document [Korean Patent Application No. 10-2021-7023724 Application (Publication No. 10-2021-0110339)]. Antibody-A was obtained from GenScript ProBio Inc., and the method for preparing antibody-A is described in detail in "Example 3" of the document [Korean Patent Application No. 10-2021-7023724 (Publication No. 10-2021-0110339)], which is incorporated herein by reference.

Antibody-B is a known antibody (zolbetuximab antibody) and was purchased from Biointron.

Antibody-C, human IgG antibody (Sigma-Aldrich, I4506), was purchased from Sigma-Aldrich CO., LTD. (St. Louis, MO, USA).

ADC-A is antibody-A conjugated with MMAE, and was produced through the process of Example 1 and Example 2.

ADC-B is antibody-B conjugated with MMAE, and Abzena was commissioned for production thereof. At this time, the method for producing ADC-B was performed by referring to the content disclosed in the literature [US 2018/0117174 (application number US 15/565,848)].

ADC-C was produced by conjugating MMAE to antibody-C through the processes of Example 1 and Example 2. At this time, the only difference is that the antibody used in Example 2 is antibody-C, not antibody-A.

### Cells and Plasma

A MIA PaCa-2~CLDN18.2 cell line (C3002) was purchased from Accurus Biosciences Inc. A PATU8988S cell line (ACC 204) was purchased from German Collection of Microorganisms and Cell Cultures GmbH (DSMZ). A MIA PaCa-2(CLDN18.2 -) cell line (CRL-1420) was purchased from American Type Culture Collection (ATCC). An SNU 601 cell line (00601) was purchased from the Korean Cell Line Bank (KCLB). A NUGC-4 cell line (ABC-TC0862) was purchased from AcceGen. An AGS cell line (21739) was purchased from KCLB.

Human plasma was purchased from BIO IVT. Monkey plasma was purchased from GENIA. Rat plasma was purchased from QuBest Bio CO., LTD. Mouse plasma was purchased from QuBest Bio CO., LTD.

### Equipment and Conditions

### Equipment

All peptides and compounds including a payload were characterized using HPLC (Waters, XBridge^{®}, C18, 4.6 × 250 mm, 5 µm).

For HPLC, an HPLC Alliance system (2996 PAD detector and 2695 separations module) manufactured by Waters was used.

As a mass spectrometer used for analysis of all small-molecular weight substances such as peptides and compounds, an LC/MS equipped with Quatro Premier XE equipment manufactured by Waters and an Acquity Waters LC system was used.

All antibodies including antibody conjugates (for example, ADC and the like) were characterized using hydrophobic interaction chromatography (HIC)-HPLC (Thermo Fisher Scientific, MAbPac^{™}, HIC-butyl, 4.6 × 100 mm, 5 µm), size-exclusion chromatography (SEC)-HPLC (Thermo Fisher Scientific, MAbPacTM, SEC-1, 300 Å 4 × 300 mm, 5 µm) and a UV spectrometer (Thermo Fisher Spectrophotometer, MULTISKAN GO / MicroDrop^{™} plate use, N12391).

A Fleta 4 centrifuge (Hanil, Korea) was used for centrifugation in spin desalting.

A UV-Vis spectrometer (Thermo Fisher, 4661030N) was used to measure the OD values in all enzyme-linked immunosorbent assay (ELISA) experiments.

GloMax^{®} Discover (Promega, GM3000) was used to measure cell viability in cytotoxicity experiments.

An S3 Live Cell Analysis Instrument (SARTORIUS, IncuCyte^{®} S3) was used for real-time internalization analysis.

As a centrifuge, Smart R17 Plus (Hanil, SM-R17PL) was used.

### Characterization of peptides and compounds using C18-HPLC

C18-HPLC was performed at a flow rate of 1 mL/min. All compounds were analyzed using the same elution conditions [initial 80% mobile phase A (0.1% TFA in H₂O) for 1 min followed by a 20% to 80% gradient of mobile phase B (0.075% TFA in ACN) in A for 15 minutes]. Chromatograms of peptides and compounds were acquired at 280 nm, and chromatograms of payloads were acquired at 254 nm.

### Characterization of antibodies using HIC-HPLC

HIC-HPLC was performed at a flow rate of 1 mL/min. HIC-HPLC was performed with all antibodies under the same conditions [initial 100% mobile phase A (1.5 M (NH₄)₂SO₄, 50 mM Na₂HPO₄ at pH 7.0, 5% isopropanol) for 1 minute followed by a 0% to 100% gradient of mobile phase B (50 mM sodium phosphate at pH 7.0, 20% isopropanol) in A for 15 minutes]. All chromatograms were acquired at 280 nm.

### Characterization of antibodies and the like using SEC-HPLC

SEC-HPLC was performed at a flow rate of 0.2 mL/min. All antibodies and the like were analyzed under the same conditions [isocratic mobile phase D (1× PBS) for 20 minutes]. All chromatograms were acquired at 280 nm.

### Optical density (OD) value measurement in ELISA experiment using UV-Vis spectrometer

OD value measurement through ELISA was performed by measuring the absorbance at 450 nm using a UV-Vis spectrometer.

### Cell viability measurement for confirming cytotoxicity

To measure cell viability in cytotoxicity experiments, the measurement of luminescence using GloMax^{®} Discover was used, and the measurement method was according to the equipment manual.

### Real-time internalization analysis

Real-time internalization measurement was performed according to the manuals of the reagents and equipment.

### Process of pretreating plasma sample and PK sample using centrifuge

Centrifugation in the pretreatment process was performed under the conditions of 13,000 rpm and 4°C for 10 minutes.

### Example 1. Preparation of Fc binding substance, compound, and the like

### Example 1.1 Preparation of Fc binding substance

The present inventors referred to the content disclosed in the document [WO2020/184944 (Application No. PCT/KR2020/003282)] to prepare an Fc binding substance represented by FcBP(Orn) below. FcBP(Orn) may be represented by Ac-PEG8-DCAWHOrnGELVWCT-NH₂, and the structure thereof is as follows:

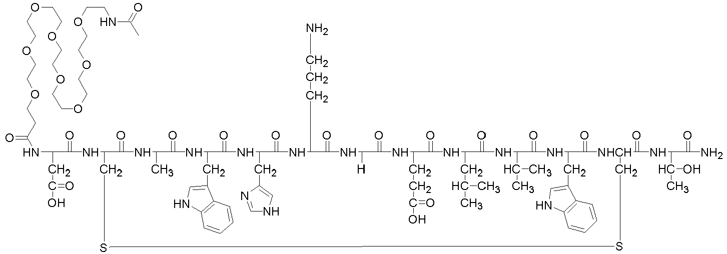

The present inventors prepared FcBP(Orn) using solid-phase peptide synthesis (SPPS).

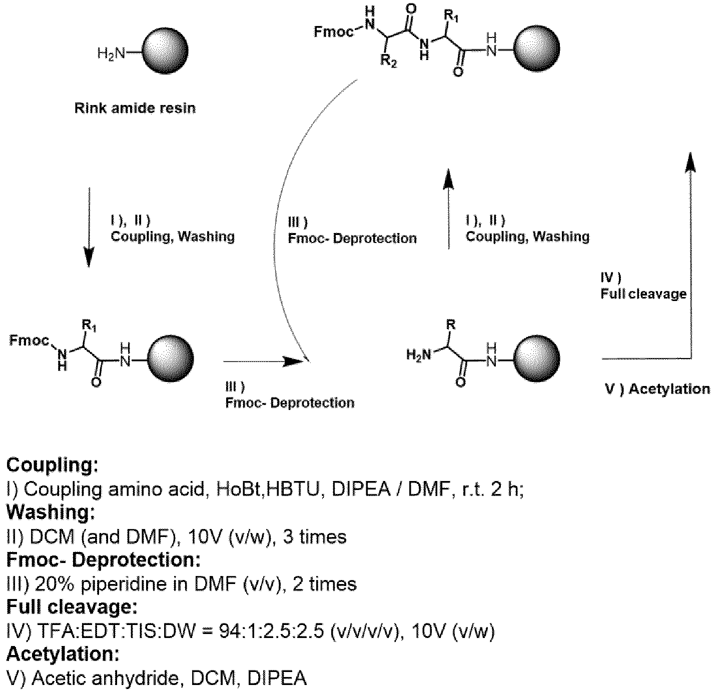

### Example 1.1.1 List of Fmoc amino acids used and order of introduction

Fmoc-L-Thr(tBu)-OH, Fmoc-Cys(Trt)-OH, Fmoc-L-Trp(Boc)-OH, Fmoc-L-Val-OH, Fmoc-L-Leu-OH, Fmoc-L-Glu(OtBu)-OH, Fmoc-Gly-OH, Fmoc-Orn(Boc)-OH, Fmoc-L-His(Trt)-OH, Fmoc-L-Trp(Boc)-OH Fmoc-Ala-OH, Fmoc-Cys(Trt)-OH, and Fmoc-Asp(tBu)-OH.

The specific preparation method is described in detail in the document [WO 2020/184944 (Application No. PCT/KR2020/003282)] which is incorporated herein by reference.

### Example 1.1.2 Introduction of amino acids

The amount of reagent used in the following process was based on 0.25 mmol. 0.5 g of a rink amide resin (0.48 mmol/g, Peptides International, USA) was put into a synthesis reactor, and 1 mmol of each Fmoc-amino acid block was weighed and prepared in the order of the peptide amino acid sequence from the C-terminal to the N-terminal.

The reactions of activating the Fmoc-amino acid and attaching the activated residue to the clear amide resin were performed sequentially from the C-terminal amino acid.

Fmoc removal was performed in 20% piperidine-containing DMF, and for the activation and introduction of residues, amino acids prepared according to the sequence were mixed with 2 mL of a 0.5 M hydroxybenzotriazole (HOBt)-containing DMF solution, 2 mL of a 0.5 M hexafluorophosphate azabenzotriazole tetramethyl uronium (HBTU)-containing DMF solution, and 174 µL of DIPEA for five minutes, and then the resulting mixture was poured into a reactor containing a resin and mixed for two hours.

The introduction reaction was confirmed by the Kaiser test, and when it was determined that unreacted amines remained, the introduction reaction was repeated once more, or capping was performed with a 20% Ac2O-containing DMF solution. The resin was sufficiently washed with DMF and DCM before moving on to the next step in each introduction reaction and Fmoc removal process. Such a process was performed repeatedly until a target peptide sequence was completed.

### Example 1.1.3 Introduction of H-PEG8-OH

To introduce H-PEG8-OH at the N-terminal after all amino acids had been introduced, 1 mL of 0.5 M Fmoc-N-amido-dPEG8-acid in a DMF solution, 1 mL of a 0.5 M HBTU-containing DMF solution, 1 mL of a 0.5 M HOBt-containing DMF solution, and 87 µL of DIPEA were mixed for five minutes, and then the resulting mixture was poured into a reactor containing a resin and mixed for two hours.

The progress of the reaction was confirmed by the Kaiser test and when it was determined that unreacted amines remained, the reaction time was extended for another one to three hours, or the reaction solution was emptied and the above-described reaction process was repeated again. After removing the N-terminal Fmoc protecting group using 20% piperidine-containing DMF, the resin was sufficiently washed with a solution of DMF and DCM, and then capping was performed with a 20% Ac2O-containing DMF solution. After capping, the resin to which the peptide was attached was dried with a solution of DCM and diethyl ether, and the weight was measured.

The peptide was cleaved from the resin by stirring 250 mg of the resin to which the peptide prepared during the amino acid introduction process was attached with 2 mL of a mixed solution of TFA, TIS, water, and EDT (94:1.0:2.5:2.5) at room temperature for 120 minutes. The cleavage mixture was filtered, the resulting filtrate was concentrated to about half with nitrogen gas, and then diethyl ether was poured to precipitate the peptide. The precipitated peptide was further washed three times with diethyl ether and dried with nitrogen gas. After the dried precipitate was dissolved in 0.1% TFA-30% ACN-containing water, the resulting solution was stirred for six hours, and then concentrated.

After the concentrate was dissolved in a 0.01 M ammonium acetate buffer (pH 6.5) solution containing 5%-DMSO-20%-ACN at a concentration of 0.1 mg/mL, the resulting solution was stirred for three days while being exposed to air. The progress of a disulfide bond formation reaction was observed by HPLC, and when it was determined that the reaction was no longer progressing, the reaction solution was lyophilized to obtain a peptide precipitate.

### Example 1.1.4 Purification

The peptide precipitate obtained by lyophilization in the above-described H-PEG8-OH introduction process was purified by prep-LC and then lyophilized. The obtained peptide was confirmed to have a purity of 90% or more by analytical HPLC, and the molecular weight of the synthesized peptide was confirmed by LC/MS.
- LC/MS analysis results: [M/3+H]=666.26; [M/2+H]=999.38
- HPLC analysis results: 9.004 min, purity: 99.9%

### Example 1.2 Preparation of Compound 1

The present inventors prepared Compound 1 below, using the obtained peptide.

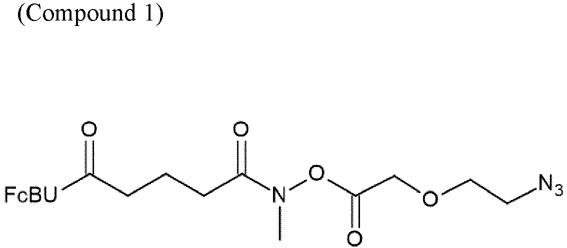

At this time, FcBU is PEG8-DCAWHOrn'GELVWCT-NH₂ derived from FcBP(Orn), and Orn' is conjugated ornithine.

Specifically, the structure of Compound 1 is as follows:

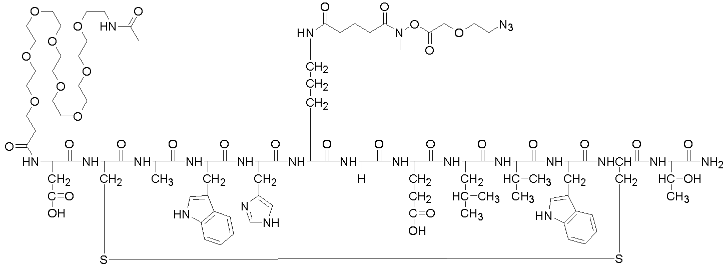

### (Exact mass: 2265.0082)

The preparation process of Compound 1 is described with reference to the following reaction scheme:

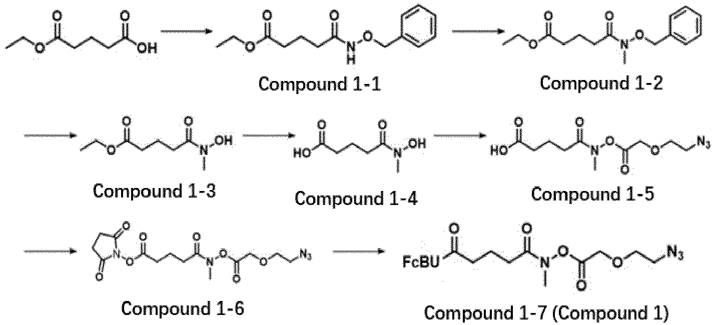

After 5-ethoxy-5-oxopentanoic acid (10 g, 68.4 mmol) was dissolved in 240 mL of dichloromethane and 9.6 mL of N,N-dimethylformamide, O-benzylhydroxylamine (6.5 g, 52.7 mmol), hexafluorophosphate azabenzotriazole tetramethyl uronium (HATU) (23.7 g, 62.3 mmol), and DIPEA (17.6 mL, 100.6 mmol) were added, and the resulting mixture was stirred at room temperature for 18 hours. Ethyl acetate (EtOAc) and distilled water were added, and the organic layer was extracted. The collected organic layer was dried over anhydrous magnesium sulfate. After filtrating, concentrating, and purifying the organic layer by column chromatography, Compound 1-1 (11.4 g, 95%) was obtained.

After Compound 1-1 (5.4 g, 21.5 mmol) was dissolved in 180 mL of N,N-dimethylformamide, potassium carbonate (K₂CO₃, 6.5 g, 47.3 mmol) and iodomethane (5.4 mL, 86.0 mmol) were added, and the resulting mixture was stirred at room temperature for three hours. Ethyl acetate and distilled water were added to extract the organic layer. The collected organic layer was dried over anhydrous magnesium sulfate (MgSO4). After filtrating, concentrating, and purifying the organic layer by column chromatography, Compound 1-2 (5.0 g, 87.7%) was obtained.

After Compound 1-2 (1 g, 3.7 mmol) was dissolved in 24 mL of tetrahydrofuran, lithium hydroxide (LiOH, 0.23 g in 8 mL of water) was added, and the resulting mixture was allowed to react at room temperature for three hours. Water and ethyl acetate were added, and water was extracted. The aqueous layer was acidified to pH 3 using 4 N HCl. Ethyl acetate was added to extract the organic layer. The collected organic layer was dried over magnesium sulfate (MgSO₄). After filtrating, concentrating, and purifying the organic layer by column chromatography, Compound 1-3 (0.7 g, 74.7%) was obtained.

After Compound 1-3 (0.85 g, 3.4 mmol) was dissolved in 13 mL of methanol, Pd/C (0.05 g) was added, and the resulting mixture was stirred under hydrogen atmosphere. The reaction solution was filtered through Celite and concentrated under vacuum to obtain Compound 1-4 (0.46 g, quantitative yield) without purification.

After Compound 1-4 (1 g, 6.208 mmol) was dissolved in 4 mL of dichloromethane, triethylamine (1.77 mL, 13.0368 mmol) and 2-(2-azidoethoxy)acetyl chloride* (1.13 g, 6.828 mmol) were added, and the resulting mixture was stirred under a nitrogen gas atmosphere at room temperature for three hours. After concentration, ethyl acetate and 10% citric acid solution were added to extract the organic layer. After concentration, the organic layer was purified by column chromatography to obtain Compound 1-5 (1.65 mg, 92.2%).

After Compound 1-5 (200 mg, 0.694 mmol) was dissolved in 6.8 mL of dichloromethane, triethylamine (0.1015 mL, 0.728 mmol) and N,N,N',N'-tetramethyl-O-(N-succinimidyl)uronium tetrafluoroborate (TSTU, 229.8 mg, 0.8634 mmol) were added, and the mixture was allowed to react at room temperature for 3 hours under an argon atmosphere. After concentration, the mixture was purified by column chromatography to obtain Compound 1-6. (221.8 mg, 83%)

After FcBP(Orn) (200 mg, 0.1002 mmol) was dissolved in 0.5 mL of N,N-dimethylformamide, N,N-diisopropylethylamine (0.07 mL) and Compound 1-6 (40.51 mg) were added, and the resulting mixture was stirred at room temperature for two hours under an argon atmosphere. After concentration, the mixture was purified by reverse-phase column chromatography to obtain Compound 1-7 (Compound 1) (226.9 mg, 64.8%).

Thereafter, the obtained Compound 1 was analyzed by mass spectrometry and HPLC, and the results are as follows:
- LC/MS analysis results: [M/2+H]=1134.35; [M+H]=2268.37
- HPLC analysis result: 13.19 min, purity 99.9%

### Example 1.3 Preparation of Compound 2 (Payload 1)

### Example 1.3.1 Preparation of Compound 2-1

40 mL of dichloromethane was added to 2-chlorotrityl chloride resin (1.4 mmol/g, 1 g) (1 equiv), and the resulting mixture was stirred for more than 30 minutes. Thereafter, the solution was removed, and ethylenediamine (5.6 mmol, 4 equiv) and N,N'-diisopropylethylamine (5.6 mmol, 4 equiv) were mixed with dichloromethane and then mixed with the resin. After stirring at room temperature for more than two hours, the reaction solution was removed. Compound 2-1 was prepared by washing the resin three or more times with a sufficient amount of each of dichloromethane and dimethylformamide to remove the residual reaction solution.

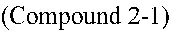

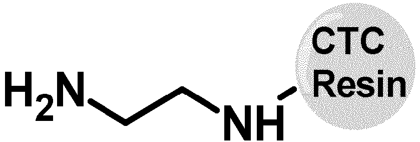

### Example 1.3.2 Preparation of Compound 2-2

N-alpha-fluorenylmethoxycarbonyl-N-epsilon-allyl-oxycarbonyl-L-lysine (Fmoc-Lys(alloc)-OH) (2.8 mmol, 2 equiv), N,N'- diisopropylcarbodiimide (2.8 mmol, 2 equiv), hydroxybenzotriazole (5.6 mmol, 4 equiv), and dimethylformamide were sufficiently mixed, and the resulting mixture was added to the resin. After stirring at room temperature for more than two hours, the reaction solution was removed. To remove the residual reaction solution, the resin was with sufficient amounts of dichloromethane and dimethylformamide at least three times each, thereby preparing Compound 2-2. The preparation of Compound 2-2 was confirmed by the Kaiser test.

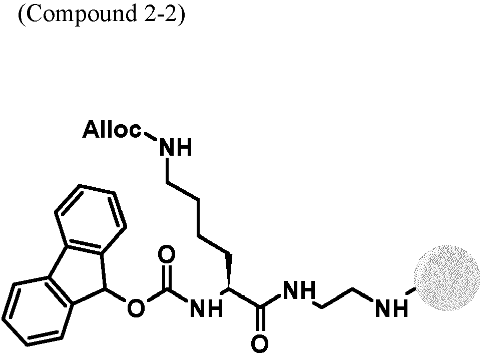

### Example 1.3.3 Preparation of Compound 2-3

A 20% piperidine solution dissolved in dimethylformamide was prepared, stirred at room temperature for 10 minutes, and the reaction solution was removed. The above-described process was performed a total of two times. To remove the residual reaction solution, the resin was with sufficient amounts of dichloromethane and dimethylformamide at least three times each, thereby preparing Compound 2-3.

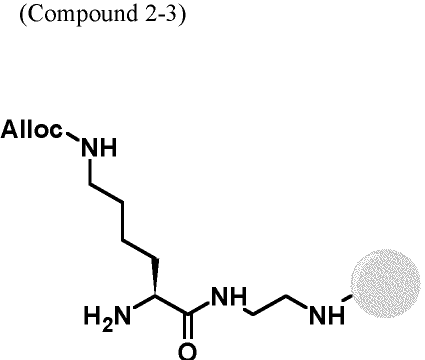

### Example 1.3.4 Preparation of Compound 2-4

Mono-tert-butyl succinate (2.8 mmol, 2 equiv), N,N'-diisopropylcarbodiimide (2.8 mmol, 2 equiv), hydroxybenzotriazole (5.6 mmol, 4 equiv), and dimethylformamide were sufficiently mixed, and the resulting mixture was added to the resin. After stirring at room temperature for more than two hours, the reaction solution was removed. To remove the residual reaction solution, the resin was with sufficient amounts of dichloromethane and dimethylformamide at least three times each, thereby preparing Compound 2-4.

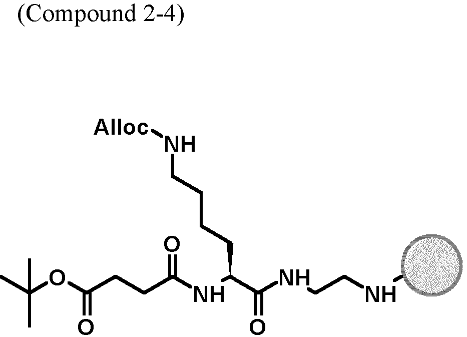

### Example 1.3.5 Preparation of Compound 2-5

The remaining resin was washed three or more times with a sufficient amount of dichloromethane. Tetrakis(triphenylphosphine palladium) (0.7 mmol, 0.5 equiv), 1,3-dimethylbarbituric acid (14 mmol, 10 equiv), and dichloromethane were sufficiently mixed, and the resulting mixture was added to the resin. After stirring at room temperature for about one hour, the reaction solution was removed. To remove the remaining reaction solution, the resin was with sufficient amounts of dichloromethane and dimethylformamide at least three times each, thereby preparing Compound 2-5.

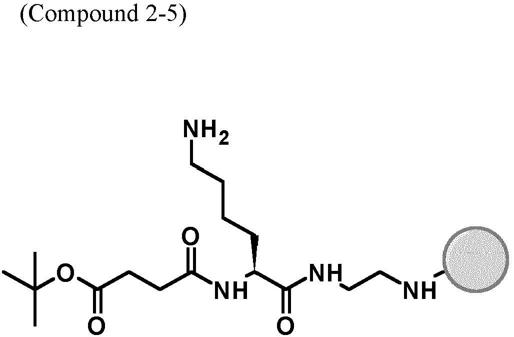

### Example 1.3.6 Preparation of Compound 2-6

N-alpha-N-epsilon-bis(9-fluorenylmethyloxycarbonyl)-L-lysine (Fmoc-Lys(fmoc)-OH) (2.8 mmol, 2 equiv), N,N'-diisopropylcarbodiimide (2.8 mmol, 2 equiv),

### hydroxybenzotriazole (5.6 mmol, 4 equiv), and

dimethylformamide were sufficiently mixed, and the resulting mixture was added to the resin. After stirring at room temperature for more than two hours, the reaction solution was removed. To remove the residual reaction solution, the resin was with sufficient amounts of dichloromethane and dimethylformamide at least three times each, thereby preparing Compound 2-6. The preparation of Compound 2-6 was confirmed by the Kaiser test.

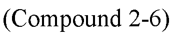

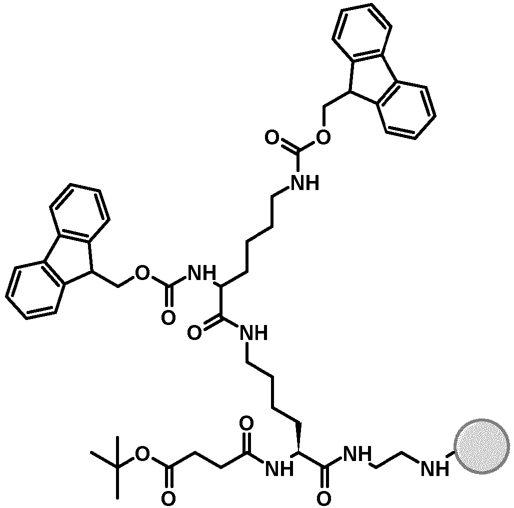

### Example 1.3.7 Preparation of Compound 2-7

A 20% piperidine solution dissolved in dimethylformamide was prepared, stirred at room temperature for 10 minutes, and the reaction solution was removed. The above-described process was performed twice in total. To remove the remaining reaction solution, the resin was with sufficient amounts of dichloromethane and dimethylformamide at least three times each, thereby preparing Compound 2-7.

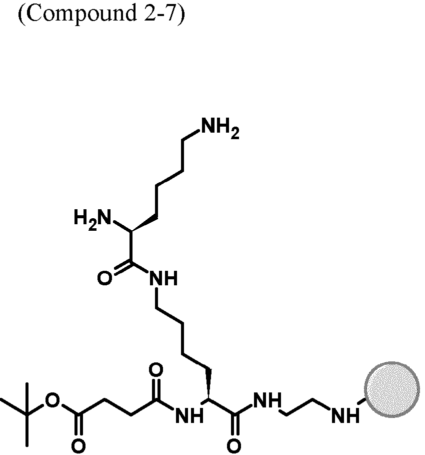

### Example 1.3.8 Preparation of Compound 2-8

2,5,8,11,14,17,20,23-octaoxahacosacosan-26-oic-acid (m-PEG8-acid) (2.8 mmol, equiv), N,N'-diisopropylcarbodiimide (2.8 mmol, 2 equiv), hydroxybenzotriazole (5.6 mmol, 4 equiv), and dimethylformamide were sufficiently mixed, and the resulting mixture was added to the resin. After stirring at room temperature for more than two hours, the reaction solution was removed. To remove the residual reaction solution, the resin was with sufficient amounts of dichloromethane and dimethylformamide at least three times each, thereby preparing Compound 2-8.

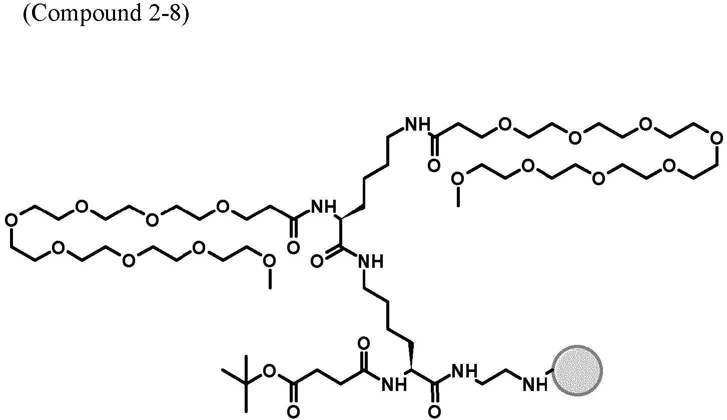

### Example 1.3.9 Preparation of Compound 2-9

A mixture of trifluoroacetic acid and distilled water in a ratio of 95:5 was prepared, a sufficient amount thereof was added to the resin, and, the resulting mixture was stirred at room temperature for two hours. After stirring, the solution and the resin were separated. A sufficient amount of 0 °C diethyl ether was added to the separated solution, mixed, and the resulting mixture was allowed stand at 0 °C for more than two hours to ensure sufficient precipitation. Compound 2-9 was prepared through centrifugation and drying. The preparation of Compound 2-9 was confirmed through mass and HPLC analyses ([M/2+H]=603.38; [M+H]=1206.21 (Exact mass: 1204.72)).

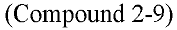

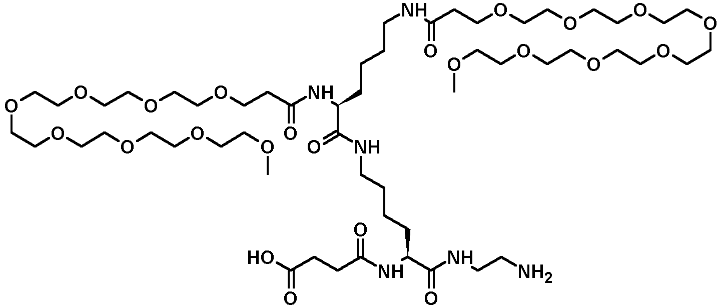

### Example 1.3.10 Preparation of Compound 2-10

1 g (0.83 mmol) of the product obtained from the previous process was dissolved in dimethylformamide, and then DBCO-C6-NHS (0.995 mmol, 1.2 equiv) and N,N'-diisopropylethylamine (1.659 mmol, 2 equiv) were added, and the resulting mixture was stirred at room temperature for more than one hour. After completion of the reaction, the reaction solution was purified by Prep-HPLC (C18) and lyophilized to obtain the product (Compound 2-10) (0.394 mmol, 47.5%). The preparation of Compound 2-10 was confirmed through mass and HPLC analyses ([M/2+H]=761.09 (Exact mass: 1519.84)).

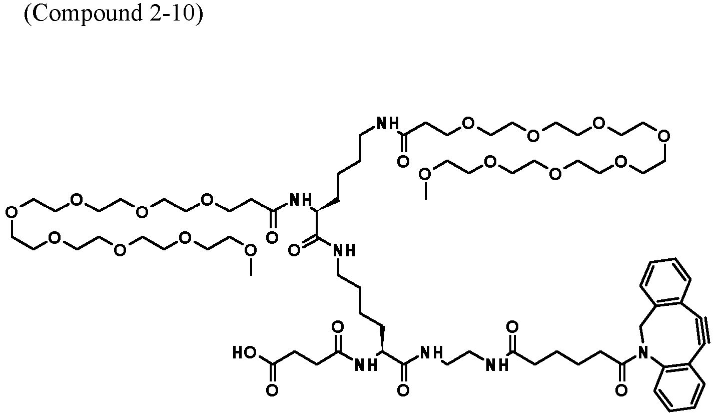

### Example 1.3.11 Preparation of Compound 2-11

The product (0.394 mmol) obtained from the previous process was dissolved in dimethylformamide, and then tetramethyl-O-(N-succinimidyl)uronium tetrafluoroborate (0.788 mmol, 2 equiv) and N,N'-Diisopropylethylamine (0.788 mmol, 2 equiv) were added, and the resulting mixture was stirred at room temperature for more than one hour. After completion of the reaction, the reaction solution was purified by Prep-HPLC (C18) and lyophilized to obtain the product (Compound 2-11) (0.138 mmol, 35%). The preparation of Compound 2-11 was confirmed through mass and HPLC analyses ([M/2+H]=809.67 (Exact mass: 1616.86)).

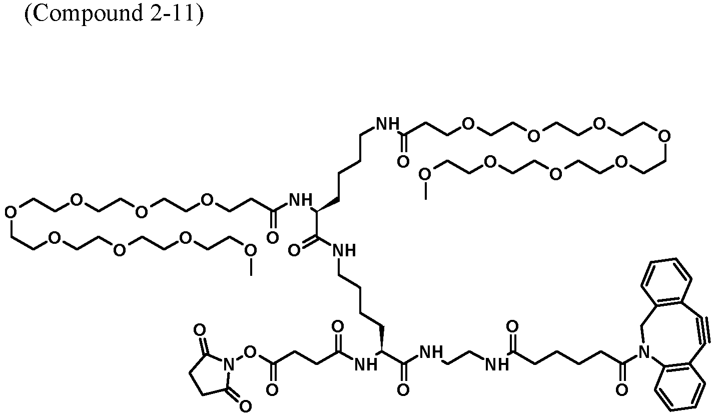

### Example 1.3.12 Preparation of Compound 2-12 (Compound 2, Payload 1)

The product (0.138 mmol) obtained from the previous process was dissolved in dimethylformamide, and then H₂N-BG-MMAE (0.152 mmol, 1.1 equiv) and N,N'-diisopropylethylamine (0.276 mmol, 2 equiv), which were prepared in advance, were added, and the resulting mixture was stirred at room temperature for more than one hour. After completion of the reaction, the reaction solution was purified by Prep-HPLC (C18) and lyophilized to obtain the product (Compound 2, Payload 1) (0.065 mmol, 47.5%).

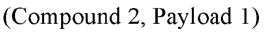

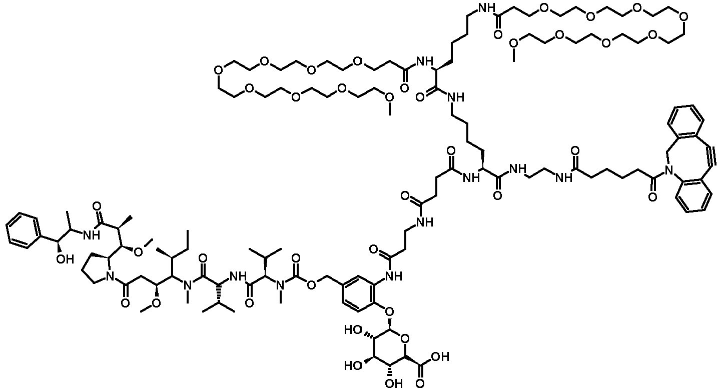

Thereafter, the obtained Compound 2 was analyzed by mass spectrometry and HPLC, and the results are as follows:
- LC/MS analysis result: [M/2+H] = 1318.06; [M+H] = 2635.38 (Exact mass: 2631.45)
- HPLC analysis result: 16.721 min, purity 100%

### Example 2. Preparation of ADC-A

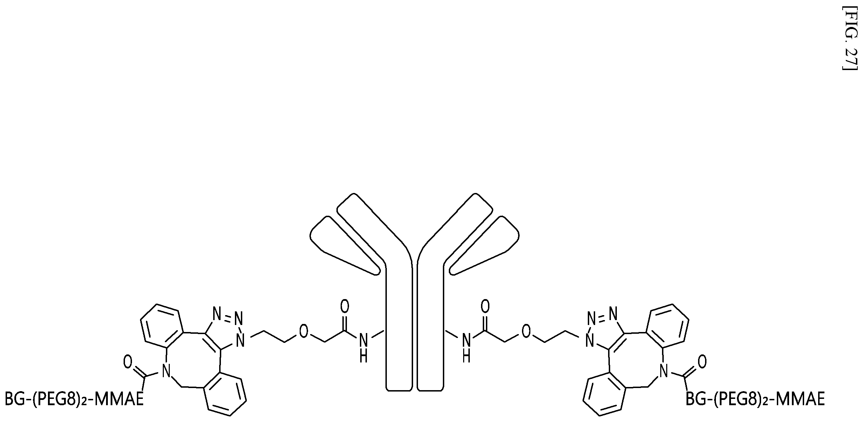

The present inventors prepared ADC-A by conjugating a drug (MMAE) to an anti-claudin18.2 antibody (antibody-A). The structure of ADC-A is shown in FIG. 27.

At this time, ADC-A includes an antibody including two heavy chains, and a payload is linked to lysine residue 246 or 248 of one of the heavy chains, and a payload is linked to lysine residue 246 or 248 of the other heavy chain. At this time, the structure of the payload linked to each heavy chain is the same.

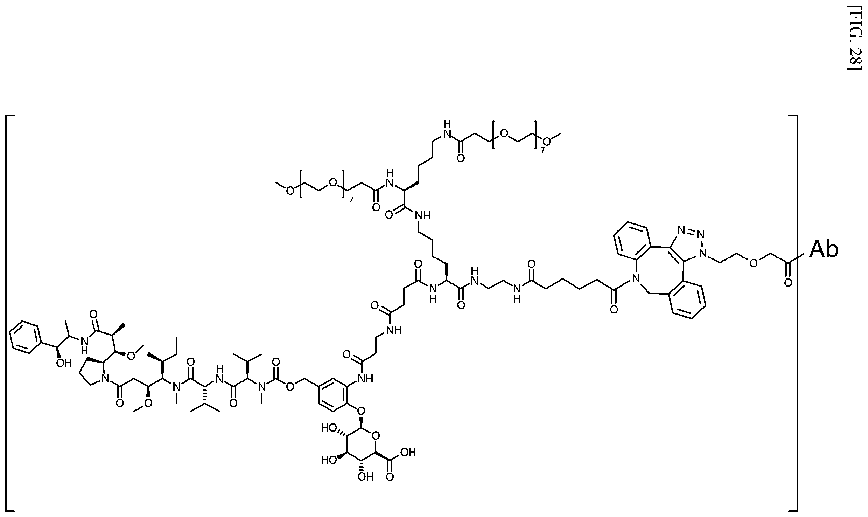

Specifically, the structure in which the payload is linked to lysine residue 246 or 248 of one of the heavy chains of the antibody in ADC-A is shown in FIG. 28.

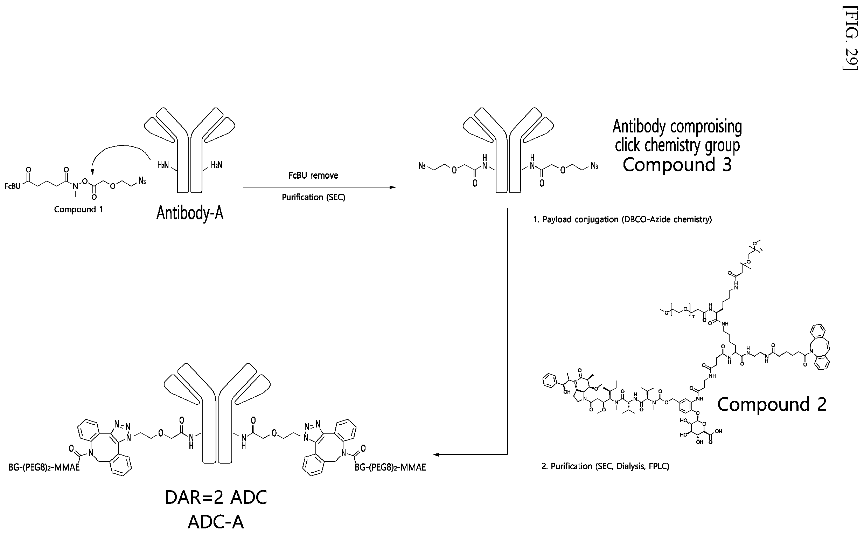

The preparation process of ADC-A is described with reference to the reaction process of FIG. 29.

### Example 2.1 Preparation of Compound 3

The present inventors prepared a conjugate (Compound 3) in which an azide is linked to antibody-A.

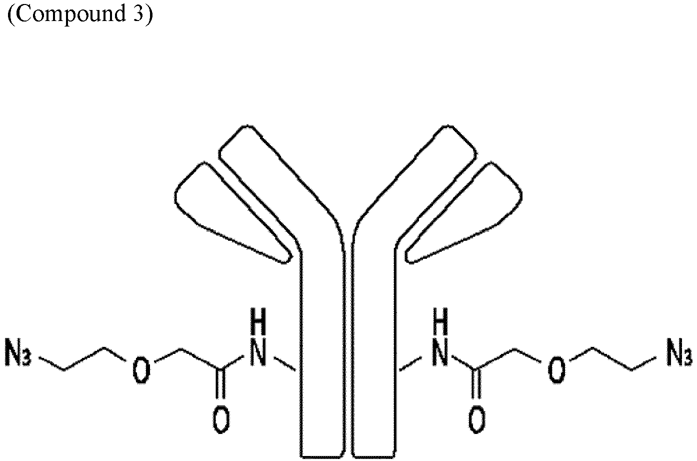

### The specific method is as follows.

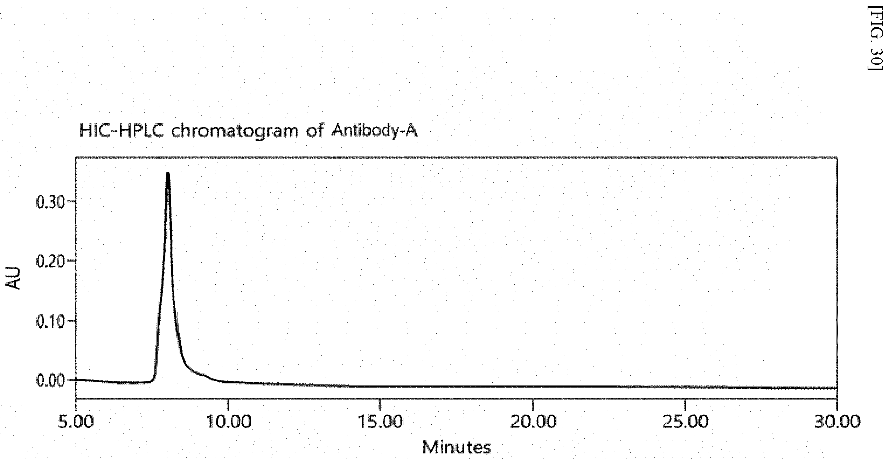

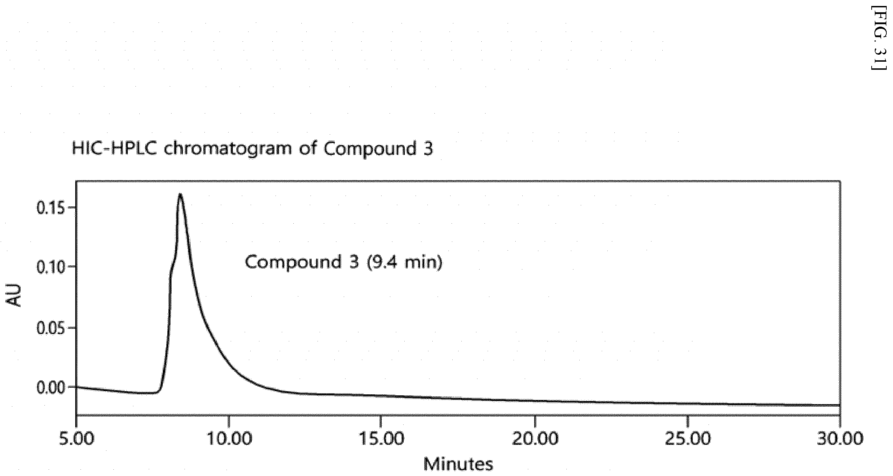

Compound 1 was prepared at a concentration of 10 mM in DMSO solvent. The prepared compound 1 (277.8 µL, 2,778 nmol, 4.0 equiv) was mixed with 101.4 mg of antibody-A (146 kDa, 5.2 mg/mL, 19.5 mL, 694.5 nmol). The resulting mixture was stirred under 1× PBS (pH 7.4), and the reaction was performed for three hours. The final DMSO product was fixed at 10%. The reaction (cross-linking of Compound 1 with antibody-A) was monitored through HIC-HPLC analysis, and the monitoring results are shown in FIGS. 30 and 31.

After completion of the reaction, Compound 1 that did not react with antibody-A was removed using spin desalting (Zeba^{™} spin desalting columns, 40 K molecular weight cut-off, 10 mL, 1000 g-force) and SEC. As a result, Compound 3 was obtained.

### Example 2.2 Preparation of ADC-A

The present inventors used the obtained Compounds 2 and 3 to prepare a conjugate (ADC-A) in which a payload of Compound 2 is linked to antibody-A. The specific method is as follows.

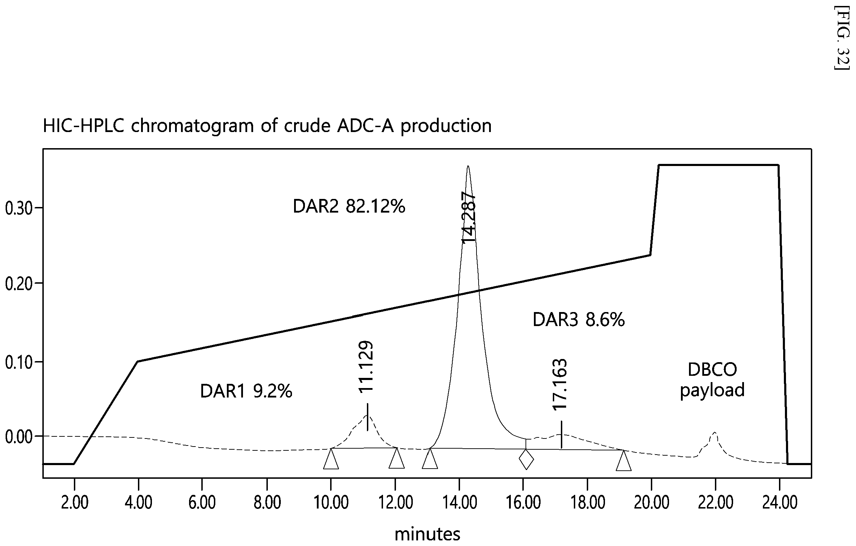

Compound 2 was prepared at a concentration of 10 mM in DMSO solvent. The prepared Compound 2 (416.7 µL, 4,167 nmol, 6 equiv) was mixed with 101.4 mg of Compound 3 (146 kDa, 5.2 mg/mL, 19.5 mL, 694.5 nmol). The resulting mixture was mixed under 1× PBS (pH 7.4) and incubated at 25 °C for 24 h. The reaction was monitored through HIC-HPLC, and the monitoring results are shown in FIG. 32.

After completion of the reaction, Compound 2 that did not react with Compound 3 was removed using spin desalting (Zeba^{™} spin desalting columns, 40 K molecular weight cut-off, 10 mL, 1000 g-force) and dialysis (pH 7.4, 1 × PBS, 4 h, 4 h, 12 h, 3 times). As a result, crude ADC-A (DAR 2) was obtained.

### Example 3. Verification of efficacy of ADC-A

### Example 3.1 Target specificity verification 1 - Confirmation of binding to antigen-expressing cells

The present inventors conducted an experiment according to Example 3.1.1 in order to confirm whether antibody-A and ADC-A are capable of specifically binding to the claudin18.2 protein (CLDN18.2), and the results are as described in Example 3.1.2.

### Example 3.1.1 Experimental method

A CHO-K1 cell line (hereinafter, referred to as "Claudin18.1 CHO-K1") into which a gene (SEQ ID NO: 18) encoding the claudin18.1 protein (CLDN18.1) was transiently transfected, a CHO-K1 cell line (hereinafter, referred to as "Claudin18.2 CHO-K1") into which a gene (SEQ ID NO: 19) encoding the claudin18.2 protein (CLDN18.2) was transiently transfected, and a CHO-K1 cell line (hereinafter, referred to as "MOCK CHO-K1") into which only a NOCK vector (empty vector) was transiently transfected were prepared. At this time, a pcDNA3.1 vector was used for Claudin18.1 CHO-K1, and a pcDNA3.1(+) vector was used for Claudin18.2 CHO-K1.

The CHO-K1 cell lines were prepared by the following processes.

The CHO-K1 cell lines were cultured in RPMI1640 containing 10% fetal bovine serum (FBS) and 1% penicillin/streptomycin in an incubator under the conditions of 37 °C and 5% CO₂.

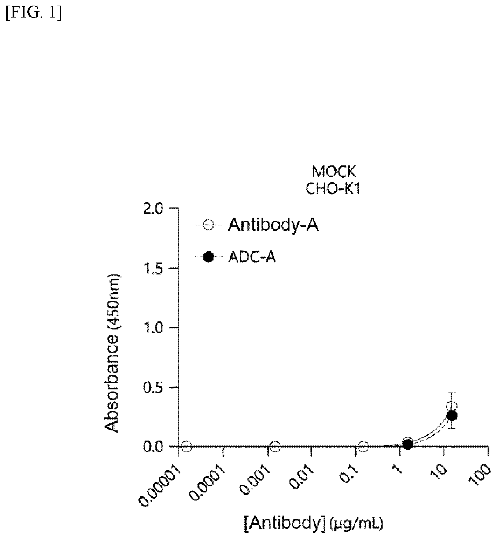

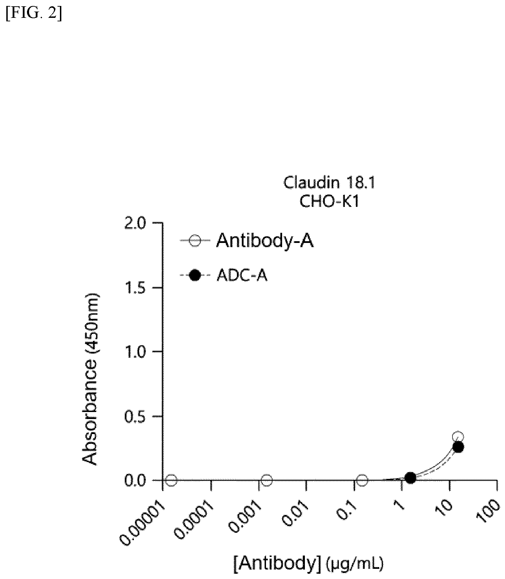

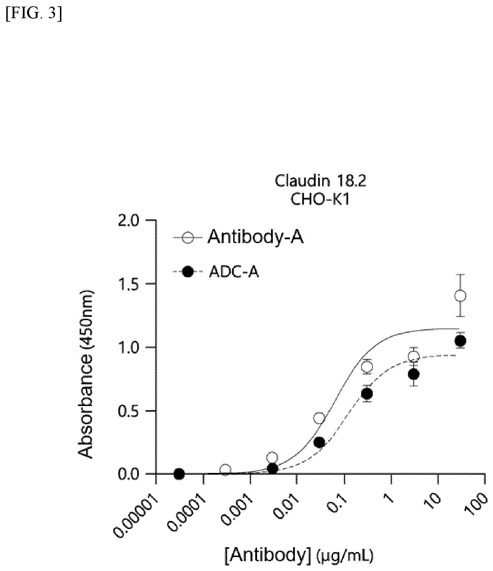

Transient transfection was performed according to the TransIT2020 manual, the transfected cells were plated on a 96-well plate for culture, and then treated with each of antibody-A and ADC-A. Thereafter, cell-based enzyme-linked immunosorbent assay (ELISA) was performed, and optical density (OD) values were measured. At this time, the measured values are shown in FIGS. 1 to 3.

The measured results were used for a regression analysis using GraphPad PRISM^{®} 9.0 software to calculate EC50 and Bmax. The cell binding affinity of antibody-A and ADC-A was evaluated based on the calculated values. At this time, the calculated values are listed in Table 1.

### Example 3.1.2 Experimental results

Through FIGS. 1 to 3, it was confirmed that antibody-A and ADC-A did not bind to the negative control MOCK CHO-K1 cell line and Claudin18.1 CHO-K1 cell line, and specifically bound to only the Claudin18.2 CHO-K1 cell line expressing CLDN 18.2.

Through Table 1, it was confirmed that the EC50 and binding maximum (Bmax) of ADC-A, which represents the maximum binding affinity, were almost the same as those of antibody-A. In other words, it can be seen that although the linker and payload were conjugated to antibody-A, the binding affinity of the antibody to the antigen was not significantly affected.

**[Table 1]**

| **Sample** | **MOCK-CHO-K1** | | **CLDN18.1~CHO-K1** | | **CLDN18.2-CHO-K1** | |
|---|---|---|---|---|---|---|
| | Antibody-A | ADC-A | Antibody-A | ADC-A | Antibody-A | ADC-A |
| **Bmax** | - | - | - | - | 1.149 | 0.9386 |
| **Kd** | - | - | - | - | 0.06363 µg/ml | 0.1230 µg/ml |
| | | | | | 0.4242 nM | 0.82 nM |

### Example 3.2. Target specificity verification 2 - Confirmation of binding to antigen protein

The present inventors conducted an experiment according to Example 3.2.1 in order to confirm whether antibody-A and ADC-A are capable of binding to the claudin18.2 protein (CLDN18.2), and the results are as described in Example 3.2.2.

### Example 3.2.1 Experimental method

To confirm the binding strength to an antigen, a CLDN18.2~VLP (virus-like particle) expressing the claudin18.2 protein (CLDN18.2) was prepared. At this time, CLDN18.2~VLP is CSB-MP005498HU (A5) from CUSABIO TECHNOLOGY LLC.

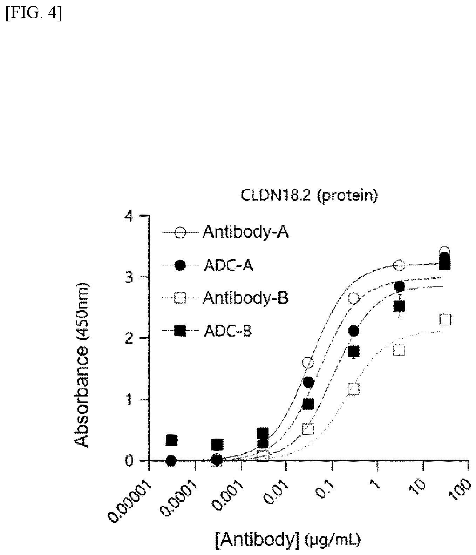

After a 96-well plate was coated with the CLDN18.2 VLP for ELISA and treated with each of antibody-A and ADC-A, indirect ELISA was performed to measure OD values. At this time, the measured values are shown in FIG. 4.

The measured results were used for a regression analysis in GraphPad PRISM^{®} 9.0 software to calculate EC50 and Bmax. The antigen binding affinity of antibody-A, ADC-A, antibody-B, and ADC-B were evaluated based on the calculated values. At this time, the calculated values are listed in Table 2.

### Example 3.2.2 Experimental results

Through FIG. 4 and Table 2, it can be confirmed that the measured binding affinity of each test substance to CLDN18.2 VLP was higher in the order of antibody-B, ADC-B, ADC-A, and antibody-A. These results mean that antibody-A and ADC-A have better binding affinity to an antigen than ADC-B and antibody-B.

**[Table 2]**

| **Sample** | **CLDN18.2 (antigen protein)** | | | |
|---|---|---|---|---|
| | Antibody-A | ADC-A | Antibody-B | ADC-B |
| **Bmax** | 3.229 | 2.995 | 2.134 | 2.869 |
| **Kd** | 0.03186 µg/ml | 0.05272 µg/ml | 0.02154 µg/ml | 0.1138 µg/ml |
| | 0.2124 nM | 0.3515 nM | 1.436 nM | 0.7587 nM |

### Example 3.3. Target specificity verification 3 - Confirmation of binding to antigen-expressing cancer cells

The present inventors conducted an experiment according to Example 3.3.1 in order to confirm whether antibody-A and ADC-A are capable of binding to cancer cells expressing the claudin18.2 protein (CLDN18.2 positive cancer cells), and the results are as described in Example 3.3.2.

### Example 3.3.1 Experimental method

Four types of cell lines (MIA PaCa-2~CLDN18.2, PATU8988S, parental MIA PaCa-2(CLDN18.2-), and SNU601) were prepared. At this time, MIA PaCa-2~CLDN18.2 and PATU8988S are CLDN18.2-positive pancreatic cancer cell lines. MIA PaCa-2(CLDN18.2-) is a CLDN18.2-negative pancreatic cancer cell line. SNU601 is a CLDN18.2-positive gastric cancer cell line.

The prepared cell lines were cultured in DMEM containing 10% FBS and 1% penicillin/streptomycin (MIA PaCa-2~CLDN18.2, MIA PaCa-2(CLDN18.2-)), RPMI1640 containing 10% FBS and 1% penicillin/streptomycin (SNU601), and DMEM containing 5% FBS, 5% serum, 1% penicillin/streptomycin, and 2 mM L-glutamine (PATU8988S) in an incubator under the conditions of 37 °C and 5% CO₂.

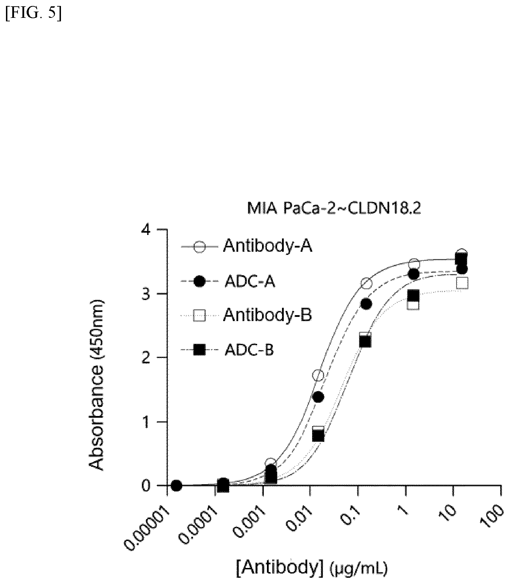

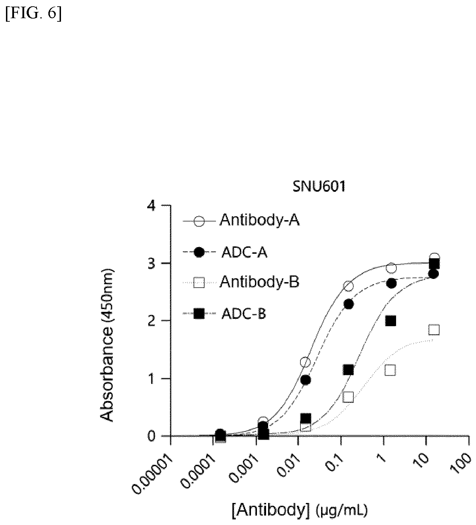

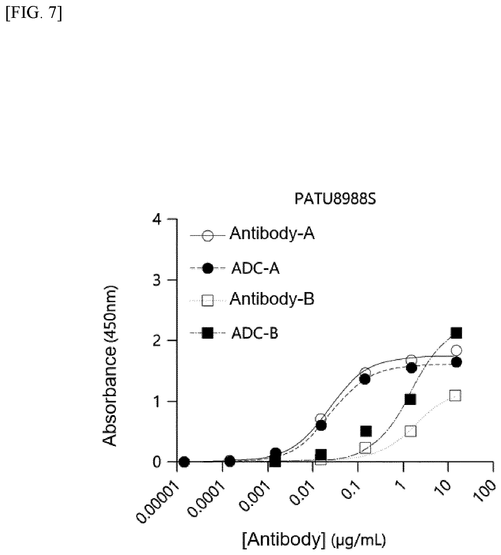

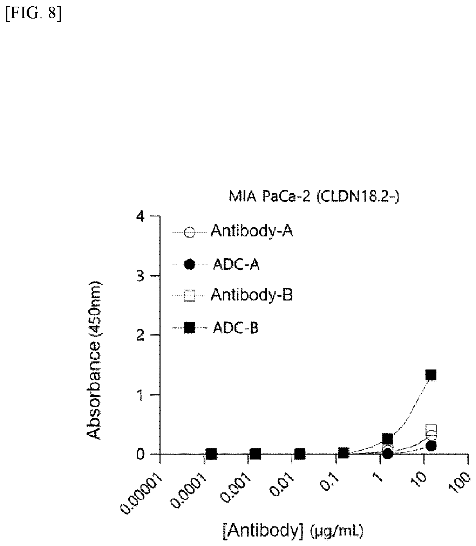

The prepared cell lines were plated on 96-well plates for culture, and then treated with each of antibody-A, ADC-A, antibody-B, and ADC-B. Thereafter, cell-based ELISA was performed, and OD values were measured. At this time, the measured values are shown in FIGS. 5 to 8.

The measured results were used for a regression analysis in GraphPad PRISM^{®} 9.0 software to calculate EC50 and Bmax. The cell binding affinity of antibody-A, ADC-A, antibody-B, and ADC-B to each cell line was evaluated based on the calculated values. At this time, the calculated values are listed in Table 3.

### Example 3.3.2 Experimental results

Through FIGS. 5 to 8 and Table 3, it can be confirmed that the measured binding affinity to the pancreatic cancer and gastric cancer cell lines was higher in the order of antibody-B, ADC-B, ADC-A, and antibody-A.

In addition, it was confirmed that the binding affinity was higher in the order of PATU8988S, SNU601, and MIA PaCa-2~CLDN18.2 for all test substances (antibody-A, ADC-A, ADC-B, and antibody-B), and it was confirmed that none of the test substances bound to parental MIA PaCa-2, which is a CLDN18.2 negative cell line. These experimental results mean that the expression level of CLDN18.2 was higher in the order of PATU8988S, SNU601, and MIA PaCa-2~CLDN18.2.

**[Table 3]**

| **Sample** | **Antibody-A** | | **ADC-A** | | **Antibody-B** | | **ADC-B** | |
|---|---|---|---|---|---|---|---|---|
| | **Kd** | **Bmax** | **Kd** | **Bmax** | **Kd** | **Bmax** | **Kd Bmax** | |
| **MIA PaCa-2 (CLDN18.2+)** | 0.016 µg/ml 0.107 nM | 3.534 | 0.022 µg/ml 0.146nM | 3.354 | 0.045 µg/ml 0.3 nM | 3.053 | 0.065 µg/ml 0.433 nM | 3.326 |
| **SNU601 (CLDN18.2+)** | 0.020 µg /ml | 3.002 | 0.028 µg /ml | 2.752 | 0.032 µg /ml | 1.701 | 0.027 µg /ml | 2.800 |
| | 0.133 nM | | 0.187 nM | | 0.213 nM | | 0.18 nM | |
| **PATU8988S (CLDN18.2+)** | 0.23 µg /ml | 1.743 | 0.026 µg /ml | 1.607 | 1.87 µg /ml | 1.214 | 1.57 µg /ml | 2.326 |
| | 0.153 nM | | | | 12.467 nM | | 10.467 nM | |
| | | | 0.173 nM | | | | | |
| **MIA PaCa-2 (CLDN18.2-)** | - | - | - | - | - | - | - | - |

### Example 3.4 Cellular internalization verification

The present inventors conducted an experiment according to Example 3.4.1 in order to confirm the cellular internalization effect of ADC-A, and the results are as described in Example 3.4.2.

### Example 3.4.1 Experimental method

In order to confirm the intracellular uptake (internalization) of antibody-A and ADC-A according to the presence or absence of CLDN18.2 expression, a MIA PaCa (CLDN18.2-) cell line, which did not express CLDN18.2, and MIA PaCa-2~CLDN18.2 and SNU601 cell lines, which expressed CLDN18.2, were prepared.

The prepared cell lines were cultured in DMEM containing 10% FBS and 1% penicillin/streptomycin (MIA PaCa-2~CLDN18.2, MIA PaCa-2 (CLDN18.2-)) and RPMI1640 containing 10% FBS and 1% penicillin/streptomycin (SNU601) in an incubator under the conditions of 37 °C and 5% CO₂. The MIA PaCa (CLDN18.2-), MIA PaCa-2~CLDN18.2, and SNU601 cell lines were plated on 96-well plates at 3,000 to 5,000 cells/well.

Samples for internalization verification, antibody-A and ADC-A, were prepared according to the IncuCyte^{®} Fabfluor-pH reagent manual.

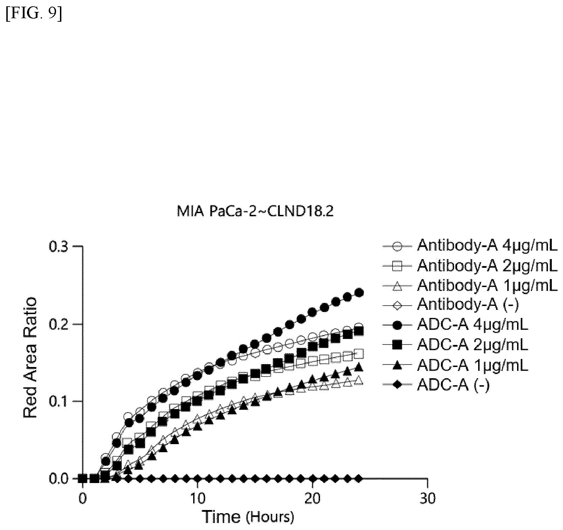

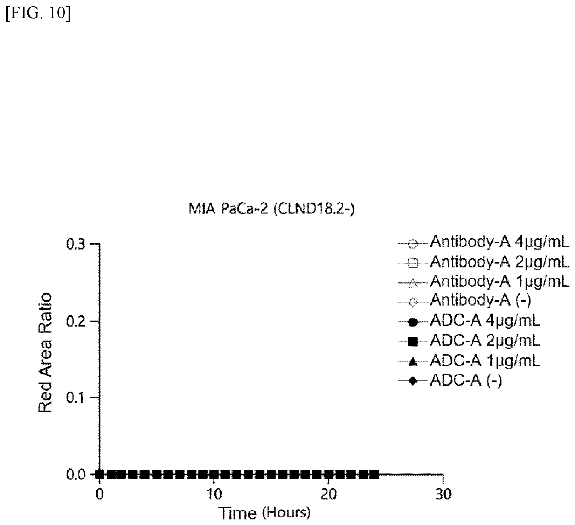

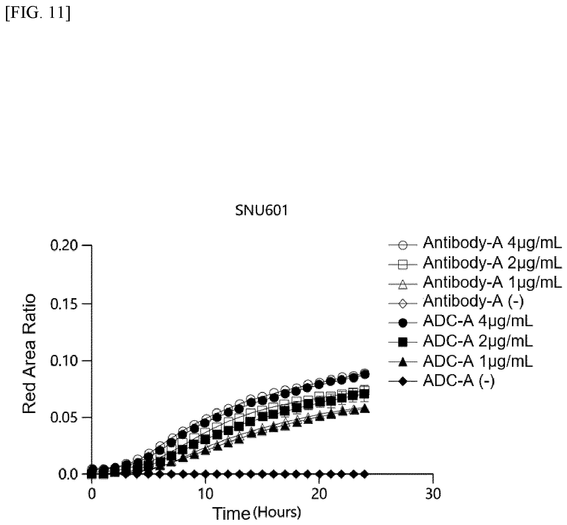

Finally, antibody-A and ADC-A were diluted to each of 8, 4, and 2 µg/mL, and designated wells were treated with 50 µL of each. The internalization levels of the plates mixed with the sample were measured every hour using the IncuCyte^{®} Live-Cell Analysis System. The measured values are shown in FIGS. 9 to 11.

### Example 3.4.2 Experimental results

Through FIGS. 9 to 11, it was confirmed that antibody-A and ADC-A were rapidly internalized within 20 hours in MIA PaCa-2~CLDN18.2 and SNU601 cell lines expressing CLDN18.2. On the other hand, it was confirmed that antibody-A and ADC-A were not internalized in MIA PaCa-2 (CLDN18.2-), which did not express CLDN18.2. In addition, antibody-A and ADC-A exhibited similar internalization efficiency at almost all concentrations. These results confirmed that the linker and the drug (payload) constituting ADC-A did not affect cellular internalization compared to antibody-A, and that internalization was determined by the expression of the antigen CLDN18.2 in the cell lines.

### Example 3.5 Cytotoxicity evaluation

The present inventors conducted an experiment according to Example 3.5.1 in order to evaluate the cytotoxicity of ADC-A, and the results are as described in Example 3.5.2.

### Example 3.5.1 Experimental method

Four types of cell lines (MIA PaCa-2~CLDN18.2, PATU8988S, parental MIA PaCa-2 (CLDN18.2-), SNU601, NUGC-4, and AGS) were prepared. At this time, MIA PaCa-2~CLDN18.2 and PATU8988S are CLDN18.2-positive pancreatic cancer cell lines. MIA PaCa-2 (CLDN18.2-) is a CLDN18.2-negative pancreatic cancer cell line. SNU601 and NUGC-4 are CLDN18.2-positive gastric cancer cell lines. AGS is a CLDN18.2-negative gastric cancer cell line.

The prepared cell lines were cultured in DMEM containing 10% FBS and 1% penicillin/streptomycin (MIA PaCa-2~CLDN18.2, MIA PaCa-2 (CLDN18.2-)), RPMI1640 containing 10% FBS and 1% penicillin/streptomycin (SNU601, NUGC-4, and AGS), and DMEM containing 5% FBS, 5% horse serum, 1% penicillin/streptomycin, and 2 mL L-glutamine (PATU8988S) in an incubator under the conditions of 37 °C and 5% CO₂.

The prepared cell lines were plated on 96-well plates for culture at 500 to 5,000 cells/well, and then treated with each test substance (antibody-A, ADC-C, ADC-A, antibody-B, ADC-C, and a combination of antibody-A and MMAE) serially diluted 10-fold (10000, 1000, 100, 10, 1, 0.1, 0.01, 0.001, and 0 ng/mL). At this time, the combination of antibody-A and MMAE means that cells were treated with antibody-A and MMAE in combination, and the concentration of MMAE is the same molar concentration as the concentration of MMAE included in other ADCs (e.g., ADC-A).

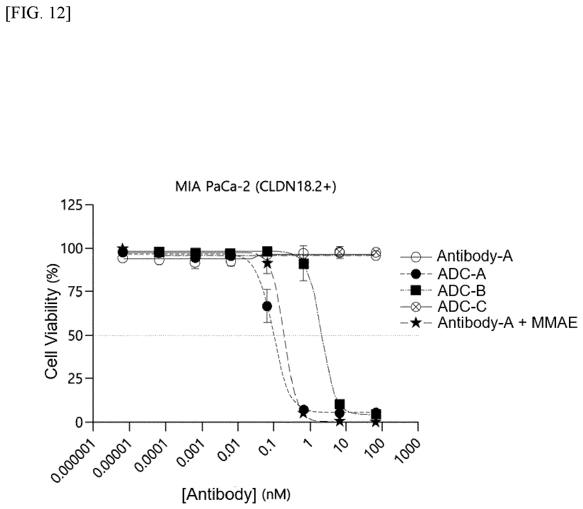

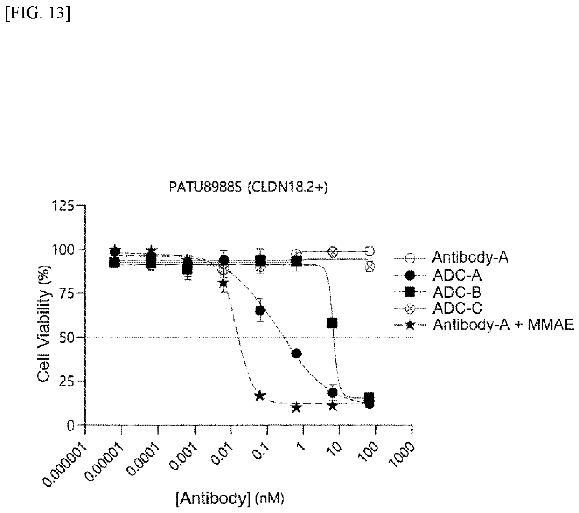

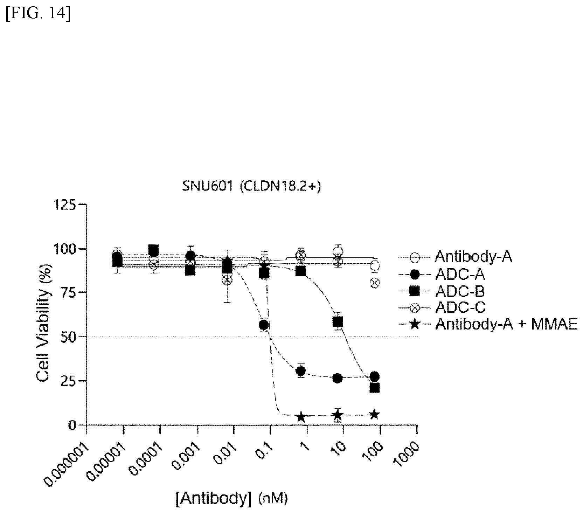

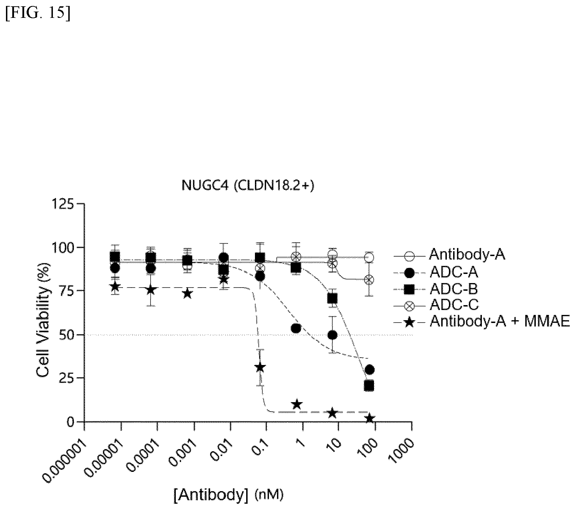

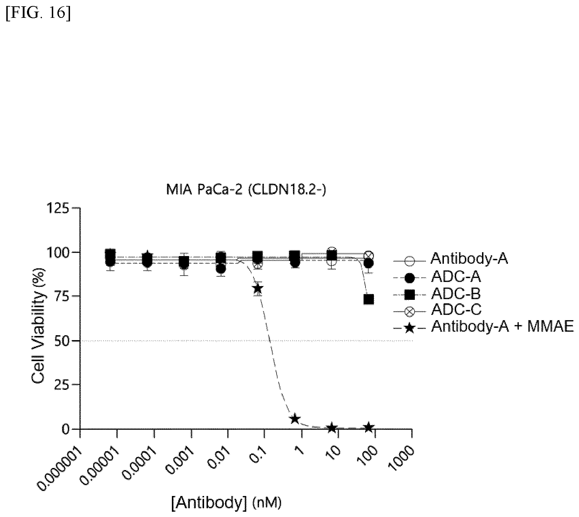

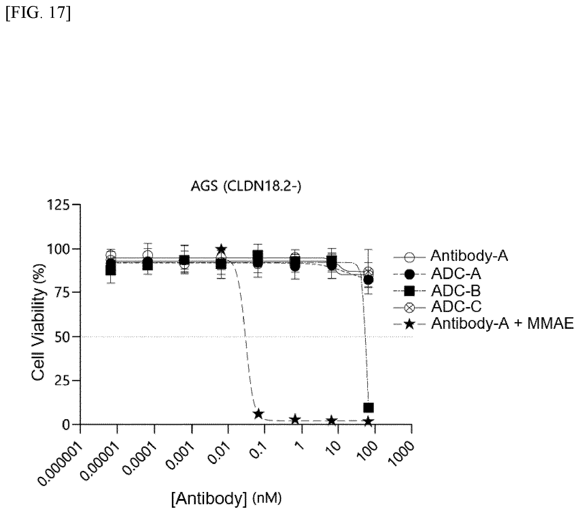

Changes in cell viability according to treatment of each test substance were measured using the same method as the CellTiter-Glo manual. At this time, the measured values are shown in FIGS. 12 to 17.

The measured results were used for a regression analysis in GraphPad PRISM^{®} 9.0 software to calculate IC50. The cytotoxicity of each test substance was evaluated based on the calculated values. At this time, the calculated values are listed in Table 4.

### Example 3.5.2 Experimental results

Through FIGS. 12 to 17 and Table 4, it was confirmed that ADC-A exhibited anticancer activity in CLDN18.2-positive cancer cell lines regardless of the type of cancer. In addition, it was confirmed that the IC50 value of ADC-A was 10 to 35-fold lower than that of ADC-B, which was a control. In particular, in the MIA PaCa-2~CLDN18.2 cell line, ADC-A exhibited a lower IC50 value than that of the antibody-A and MMAE co-administration group, and also exhibited an IC50 value of 1 nM or less in PATU8988S and SNU601. In addition, in the CLDN18.2-negative cell lines, it was confirmed that ADC-A had almost no toxicity up to the highest treatment concentration, but ADC-B, which was a control, exhibited toxicity.

**[Table 4]**

| **Cell line** | **MIA PaCa-2 (CLDN18.2+)** | **PATU8988S (CLDN18.2+)** | **SNU601 (CLDN18.2+)** | **NUGC4 (CLDN18.2+)** | **MIA PaCa-2 (CLDN18.2-)** | **AGS (CLDN18.2-)** |
|---|---|---|---|---|---|---|
| **Disease** | Pancreatic cancer | Pancreatic cancer | Gastric cancer | Gastric cancer | Pancreatic cancer | Gastric cancer |
| **Analyzed value** | IC₅₀ (nM) | | | | | |
| **Antibody-A** | -* | - | - | - | - | - |
| **ADC-C** | - | - | - | - | - | - |
| **ADC-A** | 0.1009 | 0.2785 | 0.0950 | 1.595 | - | - |
| **ADC-B** | 2.121 | 7.194 | 10.42 | 20.01 | - | 54.31 |
| **Antibody-A + MMAE**** | 0.1955 | 0.0167 | 0.0971 | 0.0580 | 0.1416 | 0.0302 |

| | | | | | | |
|---|---|---|---|---|---|---|
| *: IC₅₀ value could not be derived. | | | | | | |

### Example 3.6 Plasma stability evaluation

The present inventors conducted an experiment according to Example 3.6.1 in order to evaluate the plasma stability of ADC-A, and the results are as described in Example 3.6.2.

### Example 3.6.1 Experimental method

Human, monkey, rat, and mouse plasma were prepared. After the prepared plasma was treated with each of the test substances (antibody-A, ADC-A, and ADC-B), the plasma was incubated at a temperature of 37 °C for 5 minutes, 1 day, 5 days, 8 days, 12 days, and 15 days (a total of 108 samples were prepared).

Only the supernatant was taken from the prepared samples using a centrifuge at each time point, and this was used for analysis. The plasma stability of the test substances over time was confirmed by an ELISA analysis. ELISA plates were coated with CLDN18.2 VLP and treated with the plasma that was diluted 5000-fold, and then treated with each of antibody-A, ADC-A, and ADC-B. An anti-human IgG antibody (Promega, W4031) was used to analyze the amount of total antibodies (total Ab), and an anti-MMAE antibody (an antibody produced in-house through ABFRONTIER) was used to analyze the amount of total ADC. At this time, total Ab included not only an antibody to which a linker was not conjugated, such as antibody-A, but also a substance to which a linker was conjugated, such as ADC-A. Total ADC included only a substance to which a linker was conjugated, such as ADC-A or ADC-B, and did not include an antibody to which a linker was not conjugated. For example, when the linker was cleaved in ADC-A, it was not included in total ADC.

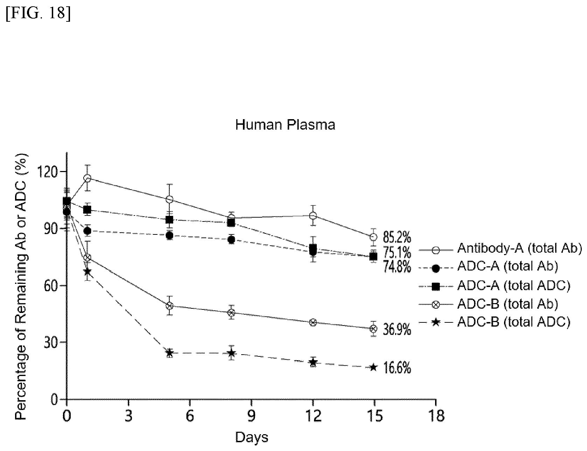

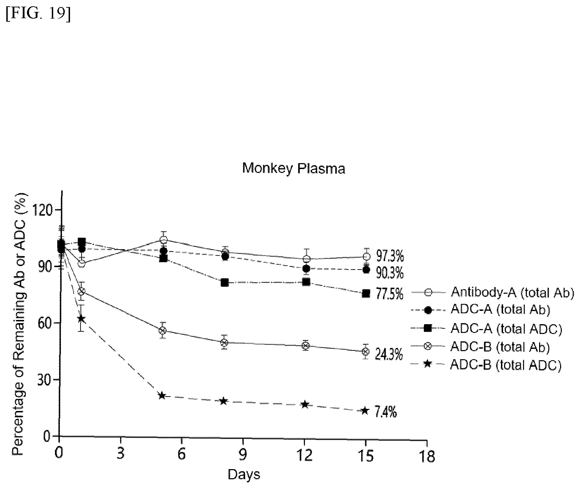

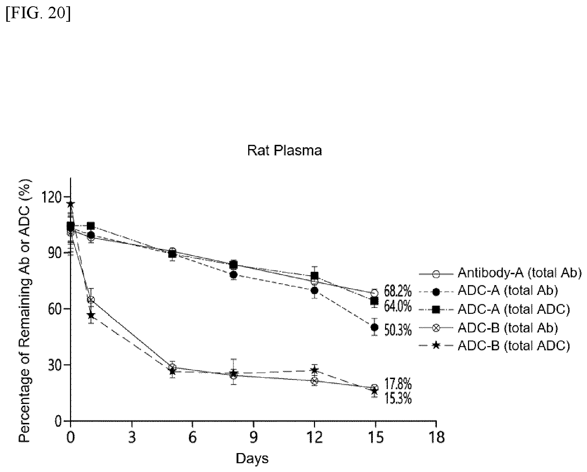

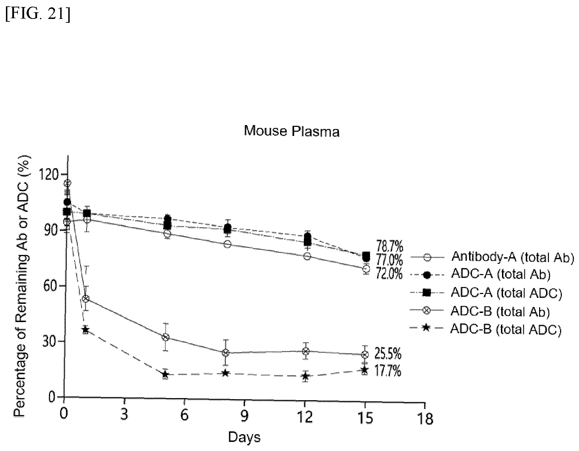

The amount of total antibodies or total ADC over time was converted into a relative percentage based on the remaining amount of the amount on day 0 (5 minutes). The converted results are shown in FIGS. 18 to 21.

### Example 3.6.2 Experimental results

### Through FIGS. 18 to 21, the following results can be seen.

In the case of 15 days in human plasma, the total antibody for antibody-A was maintained at 85.2%, the total antibody for ADC-A was maintained at 74.8%, the total ADC for ADC-A was maintained at 75.1%, the total antibody for ADC-B was maintained at 36.9%, and the total ADC for ADC-B was maintained at 16.6%.

In the case of 15 days in monkey plasma, the total antibody for antibody-A was maintained at 97.3%, the total antibody for ADC-A was maintained at 90.3%, the total ADC for ADC-A was maintained at 77.5%, the total antibody for ADC-B was maintained at 24.3%, and the total ADC for ADC-B was maintained at 7.4%.

In the case of 15 days in rat plasma, the total antibody for antibody-A was maintained at 68.2%, the total antibody for ADC-A was maintained at 64.0%, the total ADC for ADC-A was maintained at 50.3%, the total antibody for ADC-B was maintained at 17.8%, and the total ADC for ADC-B was maintained at 15.3%.

In the case of 15 days in mouse plasma, the total antibody for antibody-A was maintained at 72.0%, the total antibody for ADC-A was maintained at 78.7%, the total ADC for ADC-A was maintained at 77.0%, the total antibody for ADC-B was maintained at 25.5%, and the total ADC for ADC-B was maintained at 17.7%.

The stability of antibody-A and ADC-A in plasma exhibited similar levels and trends in all types of plasma for 15 days. In contrast, the total antibody and the total ADC for ADC-B exhibited a pattern of a rapid decrease over time. Since ADC-A exhibited the same values as antibody-A in plasma over time, it may be interpreted that aggregation or degradation do not occur in ADC-A due to the linker and drug conjugated to the antibody. Therefore, it can be seen that ADC-A exhibited stability equivalent to that of the antibody in *in vitro* plasma, and that the linker and drug conjugated to the antibody in ADC-A did not affect the stability of ADC.

### Example 3.7 Evaluation of in vivo drug efficacy

The present inventors conducted experiments according to Examples 3.7.1 to 3.7.4 in order to evaluate the *in vivo* efficacy of ADC-A, and the results are as described in Example 3.7.5.

### Example 3.7.1 Cell culture

The SNU601 cell line, which is a CLDN18.2-positive gastric cancer cell line, was cultured in an RPMI culture solution containing 10% FBS and 1% penicillin/streptomycin in an incubator under the conditions of 37 °C and 5% CO₂. The SNU601 cell line was regularly sub-cultured once a week through trypsin-ethylenediaminetetraacetic acid (EDTA) treatment.

### Example 3.7.2 Tumor inoculation and drug administration

For tumor development, the SNU601 cell line (1 x 10⁷) was mixed with serum-free medium and Matrigel, and the resulting mixture was subcutaneously inoculated into the left flank of each BALB/c nude mouse (purchased from Charles River Laboratories Japan Inc.). The mice were randomly assigned to eight groups (G1 to G8) on the basis that the average tumor volume in each group reached about 155 mm³. Thereafter, drugs were administered to each group as shown in Table 5.

**[Table 5]**

| **Group** | **Treatment** | **Drug dose (mg/kg)** | **Number of times of administration and administration period** | **Administration route** | **Number of animals (n)** |
|---|---|---|---|---|---|
| **G1** | Saline | - | Once/day 1 | Intravenous injection | 8 |
| **G2** | Antibody-A | 2 | Once/day 1 | Intravenous injection | 8 |
| **G3** | ADC-C | 1 | Once/day 1 | Intravenous | 8 |
| | | | | injection | |
| **G4** | ADC-A | 1 | Once/day 1 | Intravenous injection | 8 |
| **G5** | ADC-A | 1 | Twice/ day 1 and day 7 | Intravenous injection | 8 |
| **G6** | ADC-A | 1.5 | Once/day 1 | Intravenous injection | 8 |
| **G7** | ADC-A | 1.75 | Once/day 1 | Intravenous injection | 8 |
| **G8** | ADC-A | 2 | Once/day 1 | Intravenous injection | 8 |

### Example 3. 7.3 Observation and measurement of tumors

All animals were observed twice a day for mortality, abnormal symptoms, pain, and signs of stress, and once a day for clinical signs. The changes in body weight and the tumor volume were measured three times a week, and the tumor weight was determined after autopsy.

The tumor volume, tumor difference, and tumor growth inhibition were calculated as follows:

### Tumor volume (mm³) = (Length of major axis x length of minor axis²)/2

Tumor difference (%) = ((Tumor weight_{test group} - Tumor weight_{control (G1)}) / Tumor weight_{control (G1)}) x 100

Tumor growth inhibition (%) = 100 - ((T_{f} / Tᵢ)_{test group}) / (((T_{f} / Tᵢ))_{control}) x 100.

T_{f} is the last tumor volume measured before autopsy, and Tᵢ is the first tumor volume measured.

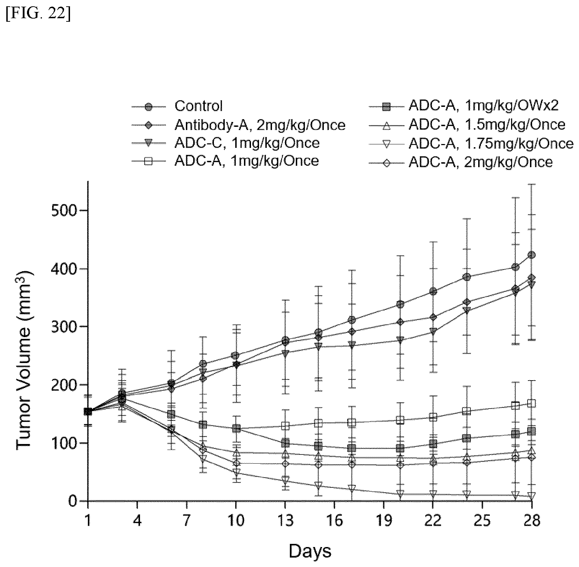

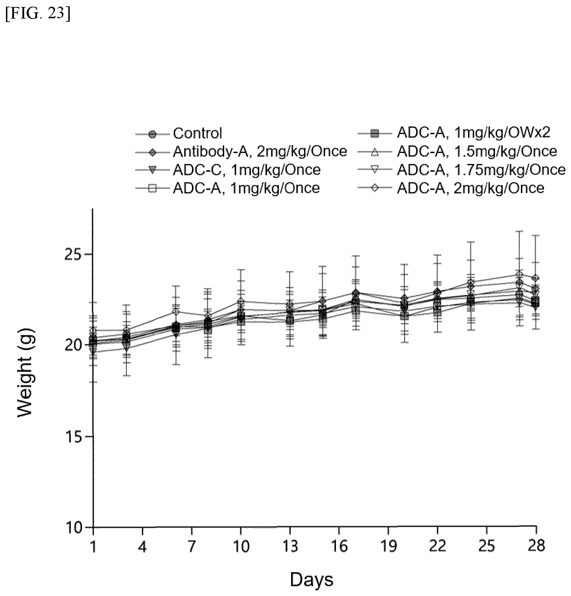

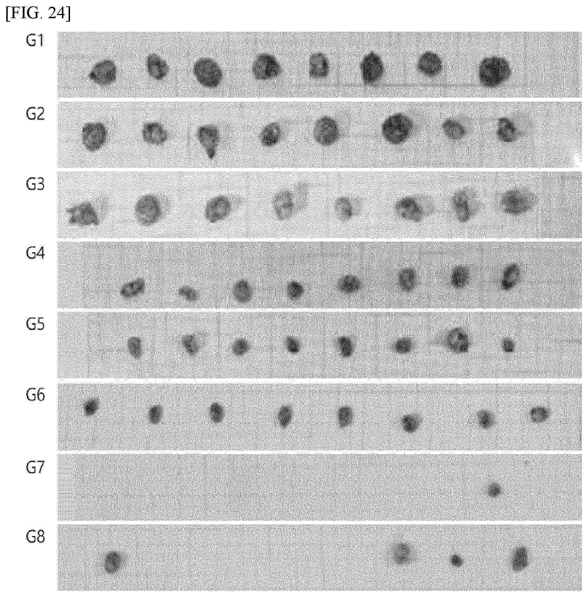

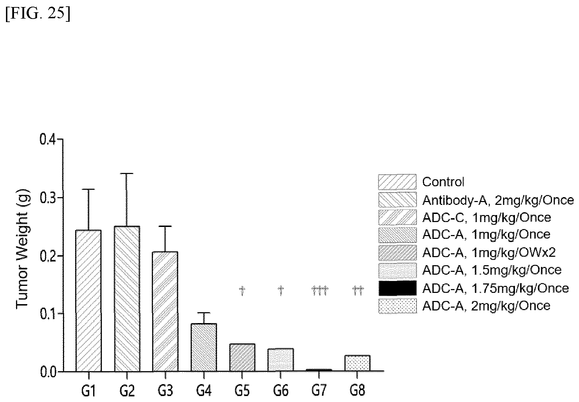

At this time, the measured tumor volume is shown in FIG. 22. The measured body weight is shown in FIG. 23. The shape of the tumor after autopsy was as shown in FIG. 24. The tumor weight determined after autopsy is shown in FIG. 25.

### Example 3. 7.4 Statistical analysis

Numerical data regarding the study was calculated as mean and standard deviation. Group variances were compared using the Bartlett's test at a 0.05 significance level for each parameter. When the difference between group variances was not significant, a parametric one-way analysis of variance (ANOVA) was performed. When a significant difference between means was indicated by ANOVA (p≤0.05), group mean comparison was performed between the control and each treatment group using the Dunnett's test. All considered groups were compared using the non-parametric Kruskal-Wallis test whenever the Bartlett's test showed a heterogeneous group variance (p≤0.05). When significance was found by the Kruskal-Wallis test (p≤0.05), the significance of the difference between the control and each treatment group was assessed using the Dunn's test. Significance was reported at the levels of 0.05, 0.01 and 0.001 for the comparison of each group. All statistical analyses were performed using GraphPad PRISM^{®} Version 5.0.

### Example 3. 7.5 Experimental results

### No unscheduled deaths were observed during the study period.

There were no effects of the test substances (antibody-A, ADC-A, and ADC-C) on the changes in body weight during the study period. The mean change in the body weight of each group is shown in Table 6 and FIG. 23.

**[Table 6]**

| **Group** | **Body weight (g) (mean ± standard deviation)** | |
|---|---|---|
| | **Day 1** | **Day 28** |
| **G1** | 20.4 ± 1.2 | 22.4 ± 1.1 |
| **G2** | 20.2 ± 1.3 | 23.0 ± 1.5 |
| **G3** | 19.6 ± 1.7 | 22.0 ± 1.2 |
| **G4** | 20.2 ± 0.8 | 22.2 ± 0.9 |
| **G5** | 20.0 ± 1.0 | 22.3 ± 0.7 |
| **G6** | 20.1 ± 0.6 | 22.8 ± 1.0 |
| **G7** | 20.4 ± 1.2 | 22.6 ± 1.0 |
| **G8** | 20.8 ± 1.5 | 23.6 ± 2.3 |

On day 28, it was confirmed that the mean tumor volumes in groups G7 and G8 were significantly lower than that in the control. The mean tumor volume and tumor growth inhibition of each group during the study period are shown in Table 7 and FIG. 22. The mean tumor weight after autopsy in each group is shown in Table 8 and FIG. 25.

**[Table 7]**

| **Group** | **Tumor volume (mm³) (mean ± standard deviation)** | | | | | **TGI* (%)** |
|---|---|---|---|---|---|---|
| | **Day 1** | **Day 8** | **Day 15** | **Day 22** | **Day 28** | **Day 28** |
| **G1** | 155.6 ± 26.6 | 234.0 ± 50.2 | 290.3 ± 79.0 | 359.8 ± 85.9 | 423.0 ± 122.0 | - |
| **G2** | 155.8 ± 26.3 | 211.3 ± 41.7 | 280.4 ± 73.3 | 316.8 ± 82.7 | 384.3 ± 108.2 | 9.3 |
| **G3** | 155.8 ± 26.1 | 220.7 ± 61.6 | 264.3 ± 74.8 | 289.8 ± 68.3 | 372.1 ± 94.8 | 12.2 |
| **G4** | 155.9 ± 25.5 | 131.7 ± 21.4 | 132.8 ± 28.1 | 145.0 ± 36.7 | 169.2 ± 39.4 | 60.1 |
| **G5** | 155.7 ± 25.2 | 132.1 ± 19.9 | 95.3 ± 25.7 | 97.7 ± 47.8 | 119.3 ± 60.6 | 71.8 |
| **G6** | 155.2 ± 24.2 | 95.6 ± 20.3 † | 78.8 ± 29.7 | 73.8 ± 33.4 | 87.7 ± 42.0 | 79.2 |
| **G7** | 155.2 ± 24.5 | 72.8 ± 15.4 ††† | 26.5 ± 6.9 ††† | 11.1 ± 18.5 ††† | 7.3 ± 20.5 ††† | 98.3 |
| **G8** | 155.9 ± 24.5 | 88.0 ± 38.9 †† | 62.2 ± 53.2 †† | 66.2 ± 76.5 | 75.2 ± 100.9 † | 82.2 |

| | | | | | | |
|---|---|---|---|---|---|---|
| * TGI: Tumor growth inhibition †: P<0.05; ††: P<0.01; †††: P<0.001. vs G1. | | | | | | |

Significance was calculated by the Kruskal-Wallis test and the Dunn's multiple comparison test.

**[Table 8]**

| **Group** | **Tumor weight (g) (mean ± standard deviation)** |
|---|---|
| | **Day 28 after first administration** |
| **G1** | 0.243 ± 0.070 |
| **G2** | 0.250 ± 0.092 |
| **G3** | 0.205 ± 0.044 |
| **G4** | 0.082 ± 0.020 |
| **G5** | 0.047 ± 0.033 † |
| **G6** | 0.038 ± 0.012 † |
| **G7** | 0.002 ± 0.006 ††† |
| **G8** | 0.027 ± 0.035 †† |

| | |
|---|---|
| **[00664]** †: P<0.05; ††: P<0.01; †††: P<0.001. vs G1. | |

Significance was calculated by the Kruskal-Wallis test and the Dunn's multiple comparison test.

In this example, an SNU601 gastric cancer tumor model was created using BALB/c nude mice, and the anticancer efficacy of antibody-A, ADC-A, and ADC-C was evaluated. In all ADC-A treatment groups, the measured tumor volume was lower than that of the control, and the measured tumor growth inhibition (TGI) was 60.1% to 98.3%. In particular, when ADC-A was administered at 1.75 mg/kg (G7), the TGI was 98.3%, indicating a very strong anticancer effect.

No unscheduled deaths occurred in any of the animals, and no significant change in body weight was observed. No histological gastric lesions associated with the antibody-A and ADC-A administration groups were observed even in the highest concentration administration group G8. In conclusion, ADC-A exhibited high anticancer efficacy in a gastric cancer model without weight loss or histological gastric lesions.

### Example 3.8 Pharmacokinetics evaluation

The present inventors conducted an experiment according to Example 3.8.1 in order to evaluate the pharmacokinetics of ADC-A, and the results are as described in Example 3.8.2.

### Example 3.8.1 Experimental method

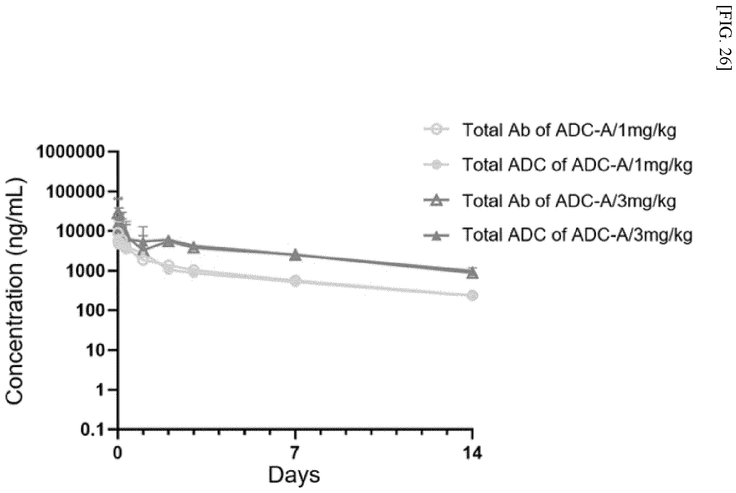

After a single administration of ADC-A at doses of 1 and 3 mg/kg to rats (purchased from SAMTAKO Bio Korea CO., LTD.) through the tail vein, blood was collected for a certain period of time and analyzed to compare and evaluate the pharmacokinetic profile. The supernatant was obtained from the plasma using a centrifuge, and the concentration of ADC-A in the plasma was analyzed using the ELISA method. Parameters were calculated by performing a pharmacokinetic analysis in a noncompartmental manner using Phoenix^{®} WinNonlin^{®} software. The calculated values are shown in FIG. 26.

### Example 3.8.2 Experimental results

As a result of a single intravenous administration of the test substance to rats, it was confirmed that all animals were exposed to the test substance, and the pharmacokinetic profile according to the administered dose was evaluated. After a single intravenous administration of ADC-A to rats at doses of 1 and 3 mg/kg, as a result of measuring the concentration of total antibodies (total Ab) in blood, the half-life (t1/2) was determined to be 4.5 and 4.7 days, and the total clearance was determined to be 2.9 and 2.8 mL/hr/kg, respectively. As a result of measuring the concentration of total ADC, the half-life (t1/2) was determined to be 5.2 and 4.9 days, and the total clearance was determined to be 3.0 and 2.5 mL/hr/kg, respectively. Through the analysis, it was confirmed that the blood concentrations of total antibody and total ADC for ADC-A over time were similar.

## Claims

1. An antibody-drug conjugate having a structure of following formula 1: 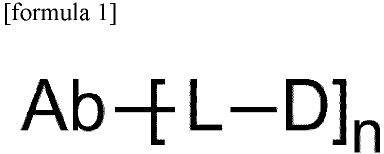
wherein, Ab is an antibody unit,
L is a linker unit,
D is a drug unit,
n is an integer of 1 to 4,
the antibody unit is a conjugated anti-claudin18.2 antibody,
the drug unit is linked to one or more of K246 and K248 of a Fc region of the antibody unit,
the linker unit has a structure of following formula 2: 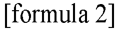 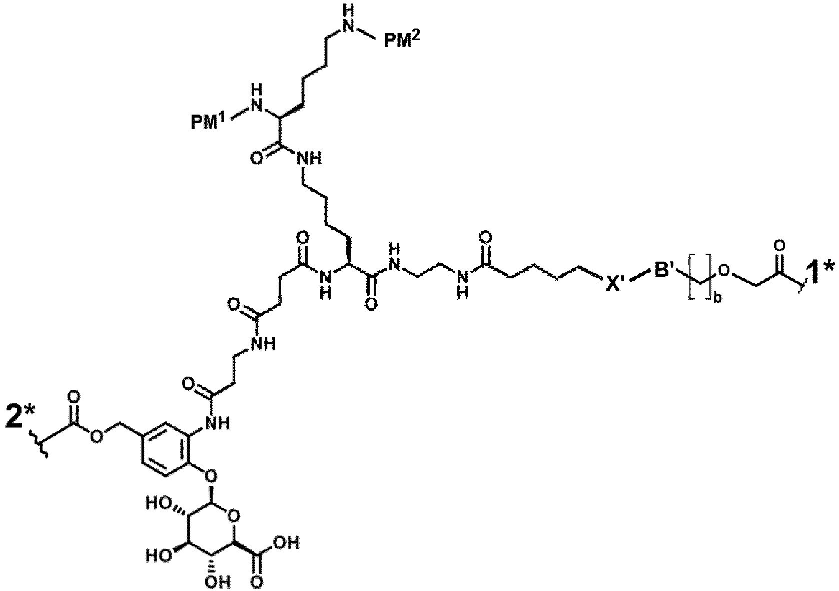
wherein, b is an integer of 0 to 6,
X' is NH-, -C(O)-, or -NHC(O)-,
B' is a group formed by a click-chemistry reaction between click-chemistry functional groups,
each of PM¹ and PM² is independently a PEG moiety, wherein the PEG moiety comprises 1 to 10 ethylene glycol units, wherein the ethylene glycol unit is CH₂OCH₂-, -OCH₂CH₂- or -CH₂CH₂O-,
1* represents an attachment site with Ab,
2* represents an attachment site with D,
the drug unit is a Monomethyl auristatin E (MMAE).

2. The antibody-drug conjugate of claim 1,
B comprises one structure selected from 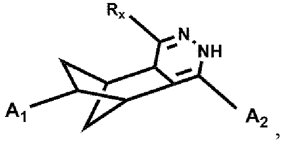 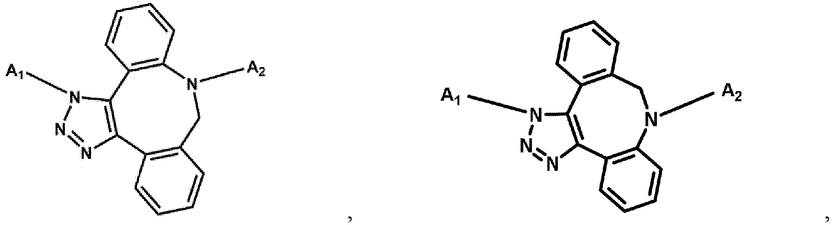 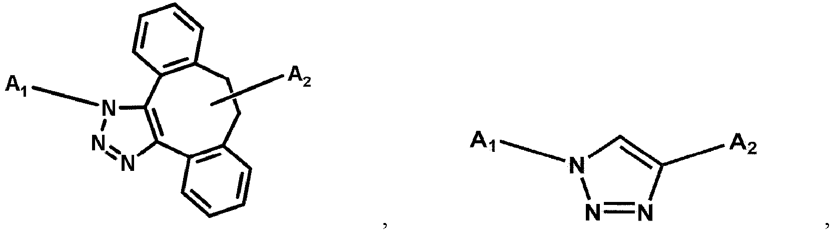 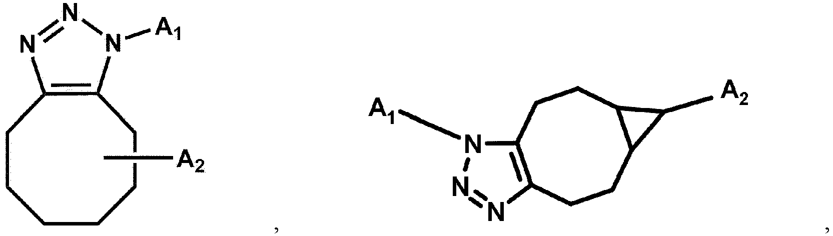 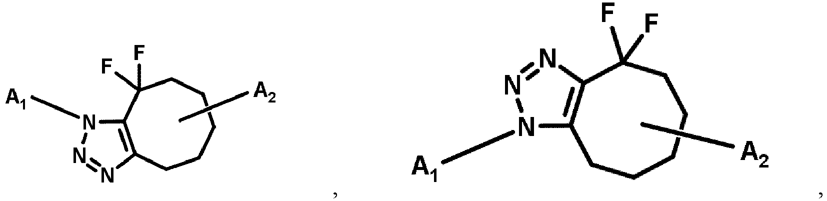 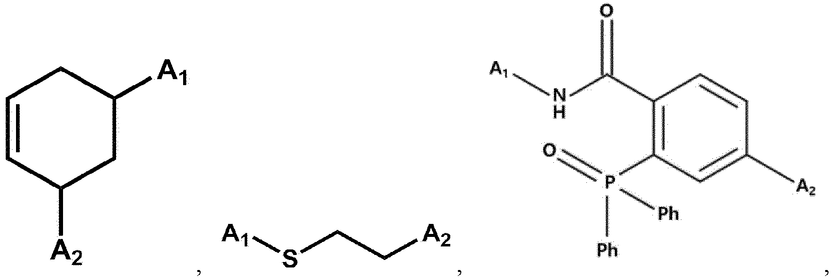 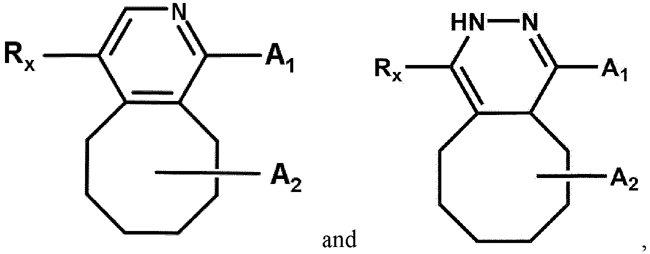
wherein, Rₓ is selected from H, halogen and C₁₋₃ alkyl,
A₁ and A₂ represent an attachment site with the remaining structure of the linker unit, respectively.

3. The antibody-drug conjugate of claim 1,
B' is 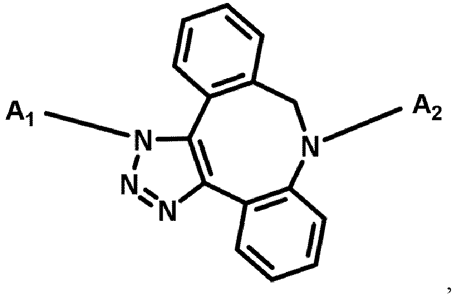
wherein, A2 represents an attachment site with X'.

4. The antibody-drug conjugate of claim 1,
b is 2.

5. The antibody-drug conjugate of claim 1,
X' is -C(O)-.

6. The antibody-drug conjugate of claim 1,
PM¹ and PM² represent the PEG moiety, respectively,
the PEG moiety comprises 1 to 10 ethylene glycol units,
the ethylene glycol unit is -[CH₂OCH₂]-, -[OCH₂CH₂]- or -[CH₂CH₂O]-.

7. The antibody-drug conjugate of claim 1,
PM¹ and PM² have the following structure, respectively,

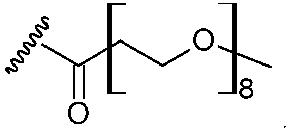

8. The antibody-drug conjugate of claim 1,
the drug unit has the following structure of formula 5: 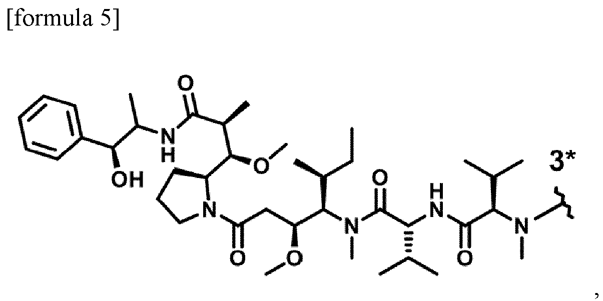 wherein, 3* represents an attachment site with L.

9. The antibody-drug conjugate of claim 1,
wherein the structure of the conjugated anti-claudin18.2 antibody is the same as that of the anti-claudin18.2 antibody, except for the conjugated portion with the linker unit.

10. The antibody-drug conjugate of claim 9,
the conjugated portion is K246 or K248 of the heavy chain of the anti-claudin18.2 antibody.

11. The antibody-drug conjugate of claim 9,
the anti-claudin18.2 antibody is an IgG antibody,
the IgG antibody is selected from the subclasses of IgG1, IgG2, IgG3, and IgG4.

12. The antibody-drug conjugate of claim 9,
wherein the heavy chain of the anti-claudin18.2 antibody comprises CDRH1 represented by an amino acid sequence of SEQ ID NO: 10, CDRH2 represented by an amino acid sequence of SEQ ID NO: 11, and CDRH3 represented by an amino acid sequence of SEQ ID NO: 12, andwherein the light chain of the anti-claudin18.2 antibody comprises CDRL1 represented by an amino acid sequence of SEQ ID NO: 13, CDRL2 represented by an amino acid sequence of SEQ ID NO: 14, and CDRL3 represented by an amino acid sequence of SEQ ID NO: 15.

13. The antibody-drug conjugate of claim 9,
the anti-claudin18.2 antibody comprises the heavy chain represented by an amino acid sequence of SEQ ID NO: 16, and the light chain represented by an amino acid sequence of SEQ ID NO: 17.

14. The antibody-drug conjugate of claim 1,
n is 2,
the antibody unit comprises two heavy chains (a first heavy chain and a second heavy chain),
the antibody-drug conjugated comprises two drug units (a first drug unit and a second drug unit),
wherein, the first drug unit is linked to one of K246 and K248 of the first heavy chain,
the second drug unit is linked to one of K246 and K248 of the second heavy chain.

15. The antibody-drug conjugate of claim 14,
the first drug unit is linked to K246 of the first heavy chain,
the second drug unit is linked to K248 of the second heavy chain.

16. The antibody-drug conjugate of claim 1,
the antibody-drug conjugate has a structure of the following formula 7:

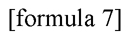

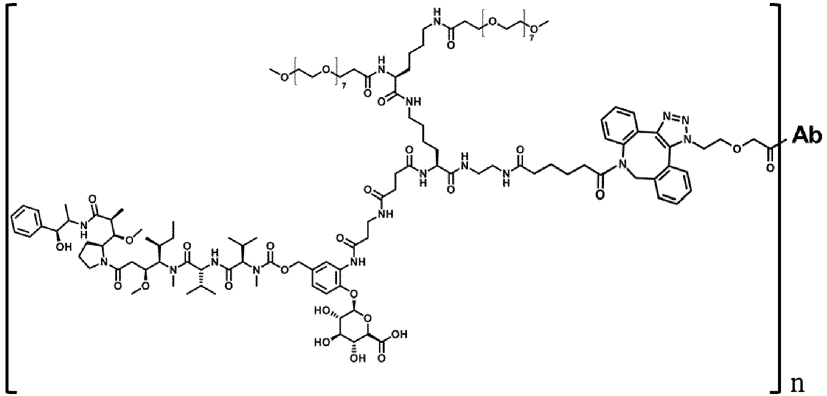

17. A pharmaceutical composition for treating a cancer, comprising a therapeutically effective amount of an antibody-drug conjugate,
the antibody-drug conjugate is an antibody-drug conjugate according to any one of claims 1 to 16.

18. The pharmaceutical composition for treating cancer of claim 17,
the pharmaceutical composition for treating cancer further comprises a pharmaceutically acceptable carrier and/or a pharmaceutically acceptable adjuvant.

19. A method for treating a cancer, comprising:
administering a pharmaceutical composition comprising a therapeutically effective amount of an antibody-drug conjugate,
wherein, the antibody-drug conjugate is an antibody-drug conjugate according to any one of claims 1 to 16.

20. Use of an antibody-drug conjugate according to any one of claims 1 to 16 for treating a cancer.

21. Use of an antibody-drug conjugate according to any one of claims 1 to 16 for a manufacture of a medication for treating a cancer.
